# EUROPEAN PATENT SPECIFICATION

(11) **EP 3 305 892 B1**
(45) Date of publication and mention of the grant of the patent: **09.09.2020**
(21) Application number: 16799402.9
(22) Date of filing: 24.05.2016
(51) Int. Cl.: C12N 7/00, A01N 63/00, C09K 17/32

(54) **METHOD FOR THE PREVENTION AND/OR THE BIOLOGICAL CONTROL OF WILT CAUSED BY RALSTONIA SOLANACEARUM**
VERFAHREN ZUR PRÄVENTION UND/ODER BIOLOGISCHEN KONTROLLE DER DURCH RALSTONIA SOLANACEARUM VERURSACHTEN VERWELKUNG
PROCÉDÉ DE PRÉVENTION ET/OU DE LUTTE BIOLOGIQUE CONTRE LA FLÉTRISSURE CAUSÉE PAR RALSTONIA SOLANACEARUM

(30) Priority: 26.05.2015 ES 201530730
(43) Date of publication of application: 11.04.2018
(73) Proprietor: Universitat De València, Estudi General, 46010 València (ES); Instituto Valenciano De Investigaciones Agrarias, 46113 Moncada Valencia (ES)
(72) Inventor: GONZÁLEZ BIOSCA, Elena, 46010 Valencia (ES); LÓPEZ GONZÁLEZ, María Milagros, 46113 Moncada Valencia (ES); ÁLVAREZ ORTEGA, María Belén, 46113 Moncada Valencia (ES)
(74) Representative: Elzaburu S.L.P.
(86) International application number: PCT/ES2016/070392
(87) International publication number: WO 2016/189180

(56) References cited:
- JP-A- 2005 278 513
- JP-A- 2007 252 351
- KR-A- 20120 055 801
- US-A1- 2014 044 679
- A. BHUNCHOTH ET AL: "Isolation of Ralstonia solanacearum -infecting bacteriophages from tomato fields in Chiang Mai, Thailand, and their experimental use as biocontrol agents", JOURNAL OF APPLIED MICROBIOLOGY., vol. 118, no. 4, 13 February 2015 (2015-02-13), pages 1023-1033, XP055397458, GB ISSN: 1364-5072, DOI: 10.1111/jam.12763
- A. FUJIWARA ET AL: "Biocontrol of Ralstonia solanacearum by Treatment with Lytic Bacteriophages", APPLIED AND ENVIRONMENTAL MICROBIOLOGY, vol. 77, no. 12, 15 June 2011 (2011-06-15) , pages 4155-4162, XP055397455, US ISSN: 0099-2240, DOI: 10.1128/AEM.02847-10
- B. BALOGH ET AL: "Phage Therapy for Plant Disease Control", CURRENT PHARMACEUTICAL BIOTECHNOLOGY, vol. 11, no. 1, 1 January 2010 (2010-01-01), pages 48-57, XP055273957, NL ISSN: 1389-2010, DOI: 10.2174/138920110790725302
- BAE JU YOUNG ET AL.: 'Biocontrol Potential of a Lytic Bacteriophage PE 204 against Bacterial Wilt of Tomato.' JOURNAL OF MICROBIOLOGY AND BIOTECHNOLOGY. vol. 22, no. 12, 2012, pages 1613 - 1620, XP055331998

## Description

### Technical Field

The invention relates to biological control of pathogenic organisms to crop plants. More specifically, the invention relates to a method for the prevention and/or biological control of bacterial wilt caused by *Ralstonia solanacearum,* to bacteriophages useful therefor and to the use of the aforementioned bacteriophages and of compositions containing them for biological control of this bacterium.

### Background of the Invention

*R. solanacearum* produces bacterial wilt worldwide in more than 200 plant species belonging to more than 50 botanical families, and many of these species susceptible to this pathogen are of agricultural interest. There are also many other crops that are colonized by the bacteria, but do not develop symptoms, which are considered tolerant crops. This bacterium especially attacks staple crops such as potatoes in more than eighty countries, with losses exceeding 950 million dollars. For this reason, it is considered as a potential agent of bioterrorism and in the European Union (EU) as a quarantine organism (Anonymous 2000: Council Directive 2000/29/EC), which is subject to strict prevention and control measures regulated by two European directives (Anonymous 1998, 2006: Council Directive 98/57/EC and Commission Directive 2006/63/EC).

*R. solanacearum* presents great intraspecific diversity, and has therefore long been considered a complex of species consisting of four phylogenetic groups or phylotypes. In 2014, after a taxonomic revision, a reclassification of this complex was proposed (Safni et al., 2014). The phylotypes I and III of *R. solanacearum* they have been classified as the new species *R. pseudosolanacearum* and phylotype IV in the new subspecies *R. syzygii* subsp. *indonesiensis.* Phylotype II retains the name of the species *R. solanacearum.*

In the text of this document the term "the species formerly known as *R. solanacearum"* is used to refer to *R. solanacearum* in studies, data, patents, publications, literature, etc., prior to the taxonomic revision of Safni et al (2014), regardless of whether the name corresponds or not with the current classification. Furthermore, the term *"R. solanacearum"* is used to refer to the species *R. solanacearum* as described after the aforementioned taxonomic revision, i.e. the species is constituted solely by strains of phylotype II.

In Spain, as in most EU countries, there have been outbreaks of the disease caused by *R. solanacearum* in several areas, usually in potatoes and in some cases tomatoes. In all cases of outbreaks the eradication measures set out in the relevant legal regulations were implemented. However, this bacterium can survive in the environment, water, soil or other reservoirs (Alvarez et al., 2007, 2008, 2010). Thus, subsequent outbreaks have been associated with irrigation with surface water contaminated with this bacteria (Caruso et al., 2005), as these are one of the main routes of introduction and spread of the pathogen into new areas, which comes through irrigation water. In fact, it described the presence of *R. solanacearum* in various waterways both in Spain and virtually all EU countries. Therefore, the EU Directives prohibit irrigation with water contaminated with this bacteria.

This represents a practical problem for the farmer, as the most affected crops are irrigated crops (tomato and potato) and irrigation water is a scarce commodity in Spain and other countries of the Mediterranean basin, where the bacteria are also present. Also in the area where outbreaks of the disease have been detected, host plants cannot be grown over a period of at least 4 years from the detection of an outbreak.

The large capacity of survival of *R. solanacearum* in the environment (water, soil or other reservoirs) impedes control . In the past and up to the present moment, control through farming practices and chemical control are being used. Thus, in areas of the world where this pathogen is present in the soil, usually in developing countries, growing host plants is problematic and the most widely used methods of control are farming techniques with varying results. Resistant cultivars have also been obtained, however, resistance may be unstable (Hartman and Elphinstone, 1994).

With respect to control via chemical or physical treatments, generally they are not effective. The application of copper compounds, antibiotics and soil fumigants have been used without much success (Lopez and Biosca, 2005), and are also expensive and have a great impact on the environment. Other chemical treatments such as chlorination or physical treatments, such as ultraviolet radiation of water contaminated with bacterial pathogens, have limited effectiveness where particles are present in the water, even if very small (Marco-Noales et al., 2008). Furthermore, it has been shown that both of aforementioned disinfectant treatments can induce various bacteria to enter into the viable but non-culturable state (VBNC) (Oliver et al., 2005 Santander et al, 2012.) Or cause reversible cell damage (McFeters and LeChevallier, 2000). These bacterial cells in the VBNC state or having reversible damage can recover cultivability and pathogenicity after coming into contact with susceptible host plants (Santander et al., 2012). These circumstances demonstrate the need for alternative methods that preferably destroy the bacterial cells.

An alternative would be biological control using specific bacteriophages of *R. solanacearum.* This biocontrol strategy has been successful in the treatment of other diseases caused by phytopathogenic bacteria (Jones et al., 2007). Bacteriophages in general, their isolation and characterization, as well as their use for plant disease control can be considered well known in the prior art (Balogh et al., 2010).

In that vein, Japanese patent JP4532959-B2 (publication number JP2005278513) describes three types of bacteriophages with bacteriolytic activity on Japanese strains of the species formerly known as *R. solanacearum* and from 2014 belonging to the new species *R. pseudosolanacearum.* Type 1, with double-stranded DNA genome (dsDNA) of approximately 250 kbp, and types 2 and 3, with a genome of single-stranded DNA (ssDNA) of 4.5 and 6 kbp respectively. Bacteriophages are characterized and distinguished by the size of their genomes and their activity against six strains of bacteria (C319, M4S, Ps29, Ps65, Ps72 and Ps74), all of which are sensitive to type 1 bacteriophages, while the type 2 only lyses one strain (the C319 strain) and type 3 lyses four of the six strains (M4s, Ps29, PS65 and Ps74) and a fifth in one trial (the C319 strain). Trials with restriction endonucleases have only been performed with type 1 bacteriophages and show that its genome (dsDNA) targets *PstI* and *KpnI,* since in the resulting restriction profiles several distinct bands are observed. Finally, the use of two of the three types of bacteriophages (types 1 and 2) to control the disease caused by species formerly known as *Ralstonia solanacearum* (currently *R. pseudosolanacearum)* by addition to soil cultivation where the plant to be protected is growing.

Japanese Patent JP4862154-B2 arises from a limitation of the above because, as is indicated therein, "it is not enough to control the effect." In this second patent is included a new type of bacteriophage lytic activity against all strains of the species formerly known as *R. solanacearum* which were tested (only a total of 15 strains) and perform characterization, but do not demonstrate their capacity for biocontrol, since no biocontrol tests were performed on plants with this new type of bacteriophage. In short, the second patent only provides a new type of lytic bacteriophage with a range of hosts that is seemingly larger than previous types.

Yamada et al (2007) describe the isolation of four types of bacteriophages that infect specific strains of the species formerly known as *R. solanacearum,* from 2014 belonging to the new species *R. pseudosolanacearum,* from soil samples taken in different areas of Japan. These authors perform structural characterization including a morphological characterization by electron microscopy virions and molecular characterization by restriction analysis of the four types of bacteriophages, and some lytic activity tests with cultured bacteria in Petri dishes. Two types of bacteriophages described are mioviruses, the genus to which all the bacteriophages infecting the species formerly known as *R. solanacearum* (now *R. pseudosolanacearum)* belong described before publication of the article by Yamada et al, and the other two types are filamentous bacteriophages of the inovirus type. Among other applications, the usefulness of the bacteriophages' lytic activity as biocontrol agents for the eradication of the species formerly known as *R. solanacearum* (now *R. pseudosolanacearum)* in contaminated soils and preventing wilting caused by the bacteria in vegetable crops is suggested, showing preference for bacteriophages capable of infecting a wide range of pathogenic strains, although only testing 15 strains of this host. The only tests with plants included in the aforementioned article referred to observing a plant injected with a strain previously infected with one of the isolated lysogenic bacteriophages, concluding that filamentous phages were not satisfactory for a disease control effect. This same test method has been used in other studies of the same group also with filamentous bacteriophages of the *Inoviridae* family [see Addy et al., 2012 and International Patent Application WO2012/147928], where it is shown that aforementioned method of inoculation, in the case of lysogenic bacteriophage type ΦRSM, leads to the emergence of avirulent strains of *R. solanacearum* that help control the disease caused by the virulent forms of aforementioned bacteria. However, besides the fact that the inoculation tests have only been performed in 5 plants per strain and without repetition [see material and methods Addy et al., 2012], the practical application of this test method in nursery or in the field is highly questionable because the work required to inject each individual plant.

In another article by the same research group (Fujiwara et al., 2011) test results demonstrating utility as biocontrol agents on the species formerly known as *R. solanacearum* (currently *R. pseudosolanacearum)* are provided for three lytic phages: two types of bacteriophages of the *Myoviridae* family described by Yamada and collaborators (2007, 2010), ΦRSA1 and ΦRSL1, as well as the effect of other additional bacteriophage ΦRSB1, with was previously isolated (Kawasaki et al., 2009). The latter belongs to the *Podoviridae* family and, like ΦRSA1, is capable of causing lysis in a higher number of strains, up to 13 of 15 strains of the species formerly known as *R. solanacearum* (of which at least 13 are currently classified as *R. pseudosolanacearum)* (Yamada et al., 2007; Kawasaki et al, 2009). It is shown that treatment of the bacteria with ΦRSA1, ΦRSB1 and ΦRSL1 either individually or in possible combinations , except treatment with ΦRSL1 alone, results in a rapid decrease in cell density of the host bacteria, which is only an initial decrease because is followed by the appearance of resistance visible by OD (optical density) in less than 2 days. To avoid such resistance, Fujiwara and colleagues (2011) selected the use of miovirus ΦRSL1 individually with respect to other combinations with ΦRSA1 and ΦRSB1, although it is noteworthy that this is the lowest bacteriophage lytic potential of the three.

Tests performed on plants described in the article by Fujiwara et al (2011), all done with ΦRSL1, entailed two treatments with a suspension of aforementioned bacteriophage at high concentrations conducted with a spacing of one month, first to the seed and then to the resulting plants; after two days these plants were individually inoculated with the bacterial suspension by direct contact to the cut tips of the roots (for 30 seconds) and then transplanted. This method of inoculation, like the previous used by these authors in other publications and patents, it is very difficult if not impossible to implement in nurseries or in cultivated fields. Furthermore, in aforementioned publication the reproducibility of the results is not indicated.

Fujiwara et al (2011) also describe stability tests on ΦRSL1 on two plants pre-treated with aforementioned bacteriophage and soil in contact therewith, detecting bacteriophages in the roots and in the rhizosphere soil 4 months after inoculation, although it was not verified whether the recovered bacteriophages are of the same type as those inoculated. Also, the effect of temperature on the stability of ΦRSA1, ΦRSB1 and ΦRSL1 was tested in presence and absence of soil (in SM buffer, Tris-HCl, NaCl, MgS0₄ and gelatine). The stability was monitored for only 15 days. At the same temperature, major differences in the stability of the three bacteriophages in the presence of soil were observed. Both in the absence and presence of soil, the differences were greater with increasing temperature but, in the absence of soil, marked differences start from 28°C. After 15 days incubation in the absence of soil at 50°C, 10% of ΦRSL1 bacteriophages survived, ΦRSB1 bacteriophages were not detected after 9 days of incubation, and after 3 days of incubation in the case of ΦRSA1.

Thus, the work of the Japanese group which includes Yamada, Addy and Fujiwara, show that in some cases and with some bacteriophages that infect the species formerly known as *R. solanacearum* (now *R. pseudosolanacearum),* can be used as agents for prevention of disease caused by the bacteria. Except in one case, the podovirus ΦRSB1, ruled out by these authors for biocontrol (Fujiwara et al., 2011), bacteriophages described and used in aforementioned tests belong to families *Myoviridae* or *Inoviridae.* In aforementioned tests, the bacteriophages are applied directly to the soil, seedlings or in roots or stems of plants. No evidence of the potential effectiveness of other means of administration of bacteriophages are given, such as perhaps through irrigation water. This is not surprising, because in Japan *R. pseudosolanacearum* has not been detected in natural watercourses, unlike the case in many European countries and in some areas of the US where this type of water is one of the reservoirs and routes of dissemination of *R. solanacearum.*

Bhunchoth et al. (Bhunchoth et al., 2015) describes the isolation of bacteriophages infecting the species formerly known as *R. solanacearum* (now *R. pseudosolanacearum)* from tomato fields in Thailand and their experimental use as biocontrol agents. 12 bacteriophages isolated from tomato fields are disclosed, from which 9 phages are podoviruses. Podovirus J2 in combination with another podovirus, ΦRSB2, very efficiently lysed host cells in contaminated soil.

An article of a Korean group, the group of Ju Young Bae (Bae et al., 2012), also relates to bacterial wilt caused by the species formerly known as *R. solanacearum* (now *R. pseudosolanacearum*) and the biocontrol potential of a lytic bacteriophage, PE204, which is characterized by a dsDNA genome similar to that of the members of the *Podoviridae* family.

The ability to control *R. solanacearum* in water by using bacteriophages has been seen in other studies in some areas of eastern and western Europe. Thus, the summary of the Georgian patent application GEU20041089 suggests neutralization of aforementioned bacteria in plants, soil and water using a mixture of polyvalent bacteriophages in equal proportion.

The present group of inventors, however, has previously suggested the use of specific bacteriophages of the species formerly known as *R. solanacearum* in irrigation water to control bacterial wilt caused by aforementioned pathogen (Alvarez et al., 2006a; Alvarez et al, 2006b). The influence of the conditions of watercourses in the survival of the bacteria have been verified, finding that the presence of native microbiota and temperatures of 24°C favoured the disappearance with respect to the temperature of 14°C and sterile water used as a control (Alvarez et al., 2006a). None of these disclosures provides information about the phylotype of the tested strains, so it cannot be aforementioned that these phages could act on the current *R. solanacearum,* consisting of strains phylotype II.

The group of the present inventors have also reported the isolation of specific lytic bacteriophages of the species formerly known as *R. solanacearum* from rivers in Spain (Alvarez et al., 2006a, Alvarez et al., 2006b), but without specifying the method of isolation and the specific place of isolation of each. The authors have reported initial data on the characterization of one of them, saying that it seems to show lytic activity between 14°C and 31°C, but not at lower temperatures (9°C) or higher temperatures (32-39°C) even in natural irrigation water pH ranges from 6.5 to 8.2. The initially characterized bacteriophage appears to be specific to the species formerly known as *R. solanacearum,* it shows lytic activity on 30 strains of different origins, of which the phylotype has not been disclosed. The bacteriophage showed lytic activity against other bacterial isolates of river water. Aforementioned bacteriophage also results in the reduction of bacterial wilt in tomato plants irrigated with water containing mixtures of bacteriophage and the species formerly known as *R. solanacearum.*

The data obtained by Alvarez and his colleagues support the possibility of using bacteriophages to prevent and/or control bacterial wilt, in particular by addition to irrigation water. However, data released by the group so far do not identify the specific watercourses from which the lytic bacteriophages obtained by them can be isolated. Nor was specific data given on any of aforementioned bacteriophages in order to facilitate the identification via the structural characteristics . Data on the range of temperatures and pH in which aforementioned bacteriophages are active has only been reported for one, which was also claimed to be able to lyse 30 different strains of the species formerly known as *R. solanacearum,* without specifying the particular strains in which it has proven activity.

Therefore, there is still a lack of specific lytic bacteriophages which have been established to, individually, be able to reduce bacterial wilt when added to irrigation water. For the single bacteriophage on which this type of tests have been carried out, reflected in the academic presentation made by the present group of inventors (Alvarez et al, 2006a; Alvarez et al, 2006b), data have not been disclosed to allow identification other than by the lytic activity at different pHs and temperatures and the claim that decreases bacterial wilt in tomato plants, not revealing the genus or family or any concrete data on watercourses in which it is present (and from which it could be isolated) or the specific method of the isolation or the phylotype of the host strains that are affected.

Thus, neither for bacteriophages isolated by Alvarez et al. or for the bacteriophages whose use is suggested in the Georgian patent application GEU20041089 in the form of polyvalent mixtures, are there available data on survival under natural conditions, in particular on their survival in the conditions of the waters in which they would be used. A central factor in determining the suitability of a bacteriophage as biocontrol agent is precisely the survival under natural conditions. When bacteriophages of phytopathogenic are applied to soil or plants to eliminate aforementioned pathogenic bacteria, the time they can stay active until they find their target cell is the limiting factor in the process, which causes repeated applications of these bacteriophages to be required for effective control. And in the case of the species formerly known as *R. solanacearum,* its control in watercourses, especially in irrigation water and containers (tanks, storage vessels, reservoirs...) it is important, especially in Europe, because:
i) the main crops affected by *R. solanacearum* are irrigated (especially potato and tomato),
ii) there is currently a shortage of water in Spain and other countries of the Mediterranean basin where the pathogen is present,
iii) there are official prohibitions throughout the EU to use contaminated water for irrigation of host plants (Anonymous, 1998, 2006 water: Council Directive 98/57/EC and Commission Directive 2006/63/EC),
iv) no control methods available for use in water,
v) a priority objective of EU policy is the conservation of the environment (Montesinos et al, 2008; Horizon 2020 Program).

Therefore, it would be desirable to control the populations of bacteria in river water and/or irrigation in a biological treatment that is effective and respectful of the natural environment. And, as was aforementioned previously, it is preferable for the method to result in the death of the bacteria.

In the case of plant pathogen *R. solanacearum,* the habitats of which are the host plants and soil, a biological agent that is supplied by water must have biological characteristics that allow it to survive in that environment, which is not the usual environment of the bacteria or its specific bacteriophages. If such survival is be prolonged and bacteriophages maintain their lytic activity on the host after long periods in water, this further favours applicability in the field as they can be transferred directly by natural and simple means such as water, without needing to encapsulated them or to provide other physical and/or biological means to protect their viability until contact with the target cell. This high survival rate would also facilitate the preparation of a commercial form, which could be in an aqueous medium without requiring refrigeration (or even lower temperatures) to maintain effectiveness.

However, it should be noted that bacteriophages are obligate intracellular parasites, and as such, require the host cell for perpetuation. Since they reach the cell in different ways, depending on the types of bacteriophages and types of host cells, survival time in the environment is expected in order to allow them to come into contact with the host cell. It is known that this time can vary between different bacteriophages, which requires study in each particular case. For example, significant variations are observed in the survival of bacteriophages the same serotype/genotype (Brion et al., 2002) or even between bacteriophages of aquatic pathogenic fish bacteria, the natural habitat of which is water (Pereira et al., 2011). In the latter case, three months has come to be regarding as good survival time of bacteriophages in water, accepting that bacteriophages displaying increased survival in water are good candidates for the control of bacterial fish diseases in aquiculture (Pereira et al., 2011).

It should be noted that *R. solanacearum* is a phytopathogenic bacterium whose natural environment is frequently the xylem of plants and soil, but not water. Since it is not an indigenous bacteria to aquatic environments, specific bacteriophages are not expected to have a high water survival rate. In fact, none of the previously mentioned publications and patents describes the viability and specific lytic activity of lytic bacteriophages of the species formerly known as *Ralstonia solanacearum* in environmental water in the absence of host cells.

And yet, it would be beneficial to have specific lytic bacteriophages against *R. solanacearum,* particularly that show a broad spectrum of strains of that species on which they are active and that also have a high survival rate in water, preferably of at least a month or more, more preferably several months. This could make possible biological treatment of irrigation water contaminated with *R. solanacearum,* using specific bacteriophages of the pathogen, which could prevent or reduce bacterial wilt in polluted areas that may impact susceptible plants. Treatment with these bacteriophages present the usual advantages over chemical treatments, as well as those associated with other treatments of biocontrol, especially with bacteriophages:
- High specificity for the host cell,
- Natural replication only in the host cell,
- Harmless to other living beings, including microbiota beneficial to the plants to be protected,
- Using biological treatment via irrigation water is easier and cheaper than chemical control, and does not require protective measures for staff during application, since it is harmless to humans,
- Lower environmental impact, especially compared to copper compounds and antibiotics used in agriculture, for which many pathogens have developed resistance, reducing the effectiveness of such chemical treatments and also increasing the chemical contamination of soil, plants and fruit,
- Less legal use restrictions, can be applied where chemical control is prohibited,
- Since they are inert in their extracellular state, bacteriophages can be combined with other control strategies and/or biocontrol to increase disease control,
- Easy, low-cost production because they increase their number in the presence of target bacteria,
- Easy adjustment of the dose of bacteriophages to be used depending on the concentration of pathogen to be treated.

The present invention provides a solution to the problem of the absence of bacteriophages that display a broad spectrum of strains of the bacterium on which they are active and that also have a high survival rate in water, preferably at least one month, or more preferably, of at least several months.

### Summary of the Invention

The present invention is based on isolation, from river water from various regions of Spain, several bacteriophages capable of lysing the bacteria *R. solanacearum,* and the results of structural, functional and molecular characterization, as well as the genomics, from which the following have been established:
- They all belong to the same viral species, the a new species belonging to the *Podoviridae* family,
- They are specific to aforementioned bacteria,
- They are active on a wide range of strains of *R. solanacearum,*
- They are active at different temperatures, pHs, salinity and aeration and in the presence of light,
- They have a high survival rate in water, more than three years, in natural waters of different chemical and pH compositions and at different temperatures, in the absence of the host, maintaining their lytic capacity after such long periods,
- They are able to decrease wilt caused by *Ralstonia solanacearum* if plants are irrigated with water containing at least one of aforementioned bacteriophages.

Tests were able to verify that these features are also shared by the bacteriophage for which a partial functional characterization (lytic activity in liquid medium at different temperatures and pHs, initial host range and ability to control wilting in plants) was already described in previous work of the present group of inventors (Alvarez et al., 2006a, Alvarez et al., 2006b), without the specific origin, method of isolation, survivability having been described and without a molecular and genomic characterization having been made that would allow taxonomic classification, for which until now the family to which it belongs is unknown.

Their high specificity, the wide range of strains they are active on and, most particularly, their high survival rate in water, makes all these bacteriophages very suitable agents for biocontrol of *R. solanacearum* in water in natural watercourses and/or irrigation water and reservoirs , and for the prevention and/or treatment of wilt produced by aforementioned bacteria in plants. This is because maintaining lytic activity after long periods favours the use in field crops or in greenhouses or nurseries or in other situations, to which they can be transferred directly via water, a natural and simple way, without encapsulating them or any other physical, chemical and/or biological means to protect the viability until contact with the target cell. Thus, implementation costs are reduced since the number of applications required over time is reduced, the long-term efficiency of the product is increased in agricultural systems in which it is intended to be used, and outbreaks of disease caused by *R. solanacearum* can be prevented and combated more effectively. Thus, control agents of *R. solanacearum* based on these bacteriophages have a greater "added value" than other products for farmers and nursery keepers, the major potential consumers of aforementioned product.

In addition, as it was previously stated, such very high survival rate in water of the bacteriophages of the invention, while maintaining the lytic capacity after long periods in aforementioned medium in the absence of a host was not expected for a specific bacteriophage of a bacterium whose natural environment is not water as it is the case of *R. solanacearum,* and this ability has not been observed in other specific lytic bacteriophages of aforementioned bacteria, isolated from natural watercourses of Spain by the present inventors.

Therefore, in a first aspect, the invention relates to a bacteriophage able to lyse cells of *Ralstonia solanacearum* selected from the group of:
a) vRsoP-WF2 (DSM 32039), vRsoP-WM2 (DSM 32040), vRsoP-WR2 (DSM 32041), or
b) a podovirus whose genome has the sequence of SEQ ID NO:1 (corresponding to vRsoP-WF2), SEQ ID NO:2 (corresponding to vRsoP-WM2) or SEQ ID NO:3 (corresponding to vRsoP-WR2).

Hereinafter, the term "bacteriophage of the invention" is used to refer to any one of these bacteriophages.

In another aspect, the invention relates to a composition comprising at least one of the bacteriophages of the invention, or combinations of them. This composition will be considered a composition of the present invention.

In a further aspect, the invention relates to the use of at least one of the bacteriophages of the invention, or combinations , to control *R. solanacearum* in natural watercourses, channelled water streams, natural reservoirs of water, irrigation water and irrigation water reservoirs, by adding one or more of these bacteriophages to irrigation water or reservoirs .

In another aspect, related to the previous aspect, the invention relates to the use of at least one of the bacteriophages of the invention, or combinations , or compositions of the invention to control *R. solanacearum* in soil by addition of one or more of the aforementioned bacteriophages or a composition of the invention to aforementioned soil through irrigation water with which the soil is watered, or pre-treated with the aforementioned bacteriophages or aforementioned composition.

In an additional aspect, the invention relates to a method for preventing or controlling bacterial wilt caused by *Ralstonia solanacearum* in plants, comprising the steps of:
a) adding a composition to the water to be used for watering plants, comprising bacteriophages belonging to at least one of the bacteriophages of the invention, or combinations;
b) watering plants with aforementioned water.

As indicated previously, the term *"Ralstonia solanacearum,"* without reference to the previous meaning of this term is used in the invention to refer to the species *R. solanacearum* as described after the last taxonomic review, i.e. the species constituted by phylotype II strains. Conversely, when the term "species formerly known as *R. solanacearum"* is used, it refers to the bacteria that were considered to be within the term *R. solanacearum* in studies, data, patents, publications, literature, etc., prior to the taxonomic revision of Safni et al. (2014), regardless of whether the name corresponds or not to the current classification.

Accordingly, the present invention, i.e. the "method for the prevention and/or biological control of wilt caused by *Ralstonia solanacearum,* by means of the use of bacteriophages useful therefor and compositions ", refers to *R. solanacearum* as described after taxonomic revision of Safni et al. (2014). The three inventions of other authors mentioned in this document refer to the "species previously known as *R. solanacearum"* that, in cases where information is available (patent documents, publications, etc.), it mostly refers to strains reclassified as the new species *R. pseudosolanacearum.*

### Brief Description of the Figures

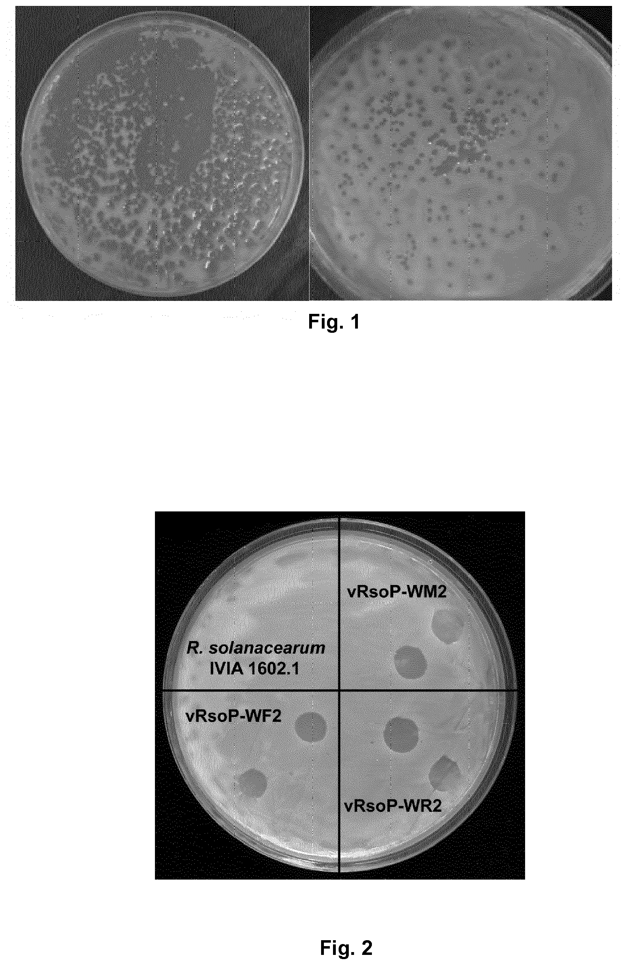

Fig. 1 shows photographs of the culture medium dishes from the lytic activity tests on the bacteriophages isolated from river water against *Ralstonia solanacearum.* Darker areas correspond to areas of lysis and/or isolated plaques, which are bacteriophage breeding areas in the bacterial lawn, which allow the lysis of the bacteria to be observed in the culture medium, the massive growth of the aforementioned bacterium being seen in whitish and opaque areas.
Fig. 2 shows a photograph of a culture medium dish with bacterial lawn from strain IVIA 1602.1 of *R. solanacearum* on which lysis tests were performed. In each quadrant, the bacteriophage contained in the suspension added to the bacterial lawn is indicated; the location of the control quadrant without bacteriophages (upper left quadrant, marked with the name of the bacterial strain) is also indicated.

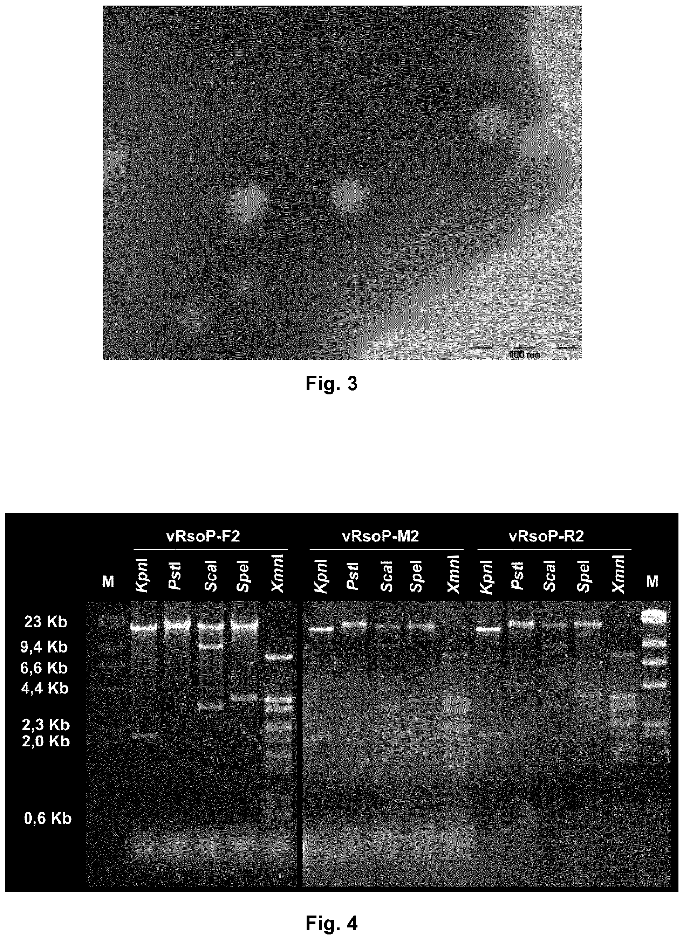

Fig. 3 shows a photograph of the bacteriophages of the present invention obtained by transmission electron microscopy after negative staining. It is observed that they present a non-enveloped, polygonal head (40 to 60 nm in diameter depending on the bacteriophage) and a short tail.
Fig. 4 is a photograph obtained after subjecting to electrophoresis the samples in which digestion of the DNA of bacteriophages vRsoP-WF2, vRsoP- WM2 or vRsoP-WR2 (as indicated at the top of the photograph) had been carried out with various restriction enzymes indicated above each column. The columns at the extreme right and left ends correspond to the pattern of molecular weights (M): λ phage DNA digested with *HindIII.*

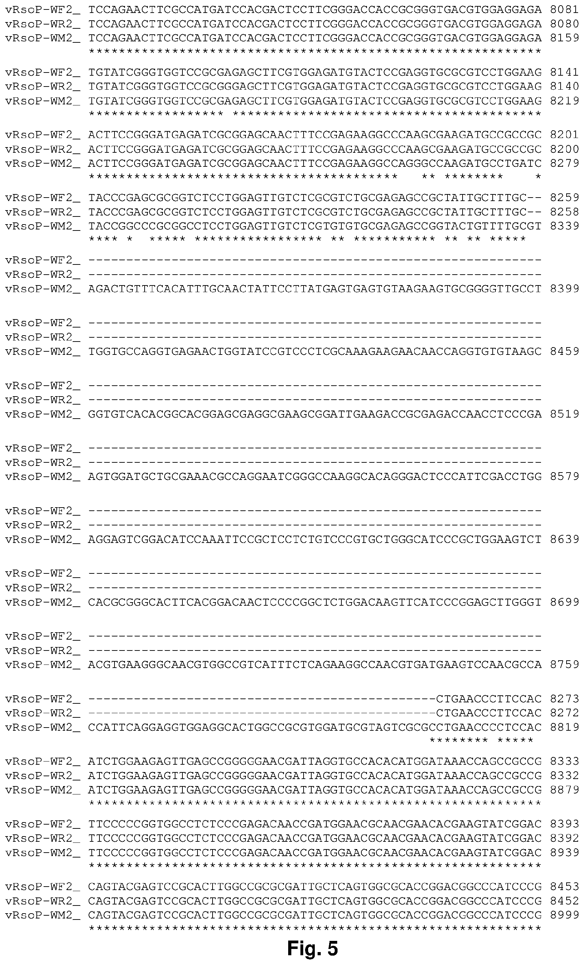

Fig. 5 shows the area in which the sequence corresponding to the vRsoP-WM2 bacteriophage presents an insertion of 468 nucleotides to the sequences corresponding to the bacteriophages vRsoP-WF2 and vRsoP-WR2, as well as areas close to this sequence. The presence of a hyphen in a sequence indicates a position where a nucleotide is absent in the aforementioned sequence with respect to one of or both of the other sequences, such absence allowing continued alignment in the same area. In the lower line below the corresponding sequence lines, the presence of an asterisk indicates coincidence between the nucleotides situated at that position in the three sequences.

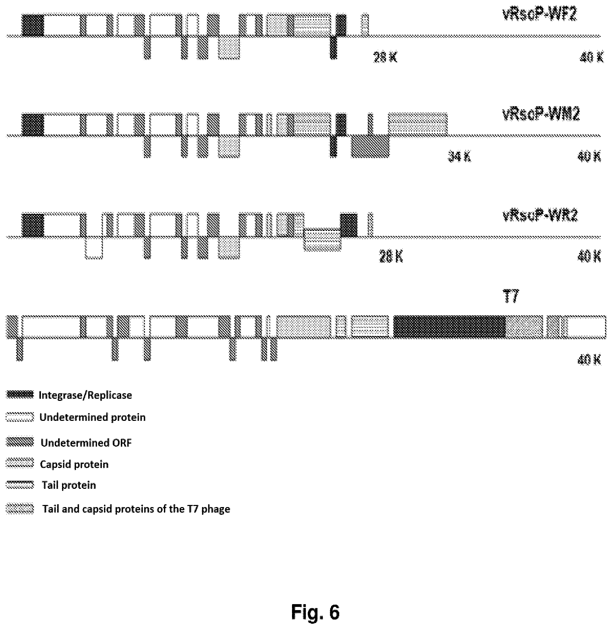

Fig. 6 shows the genomic organization of bacteriophages vRsoP-WF2, vRsoP- WM2, and vRsoP-WR2 as compared to bacteriophage T7. In various shades of grey or with weaves of parallel lines in different directions, the location of functional open reading frames (ORFs) that have been identified using the BLAST tool is indicated, as specified in the legends in the lower part of the figure. It is noted that the three bacteriophages possess a genomic organization and expression of the ORFs similar to bacteriophage T7 in part of the genome.

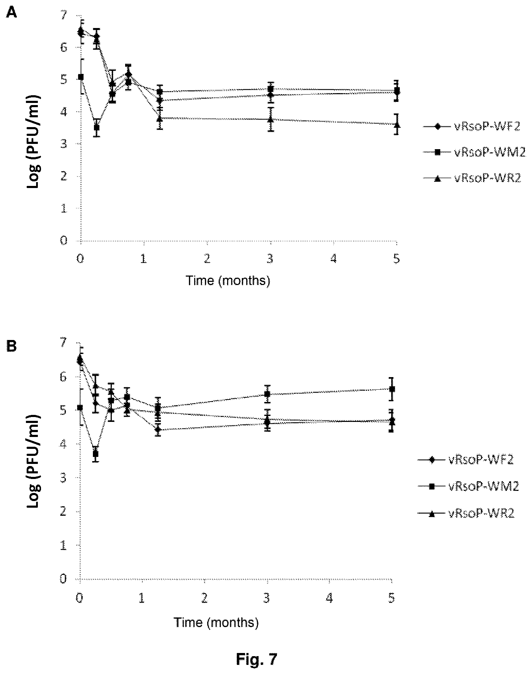

Fig. 7 shows the survival curves of bacteriophages vRsoP-WF2, vRsoP-WM2 and vRsoP-WR2, at incubated at 14°C in the absence of host cells in water from the Tormes river (panel A, top) and the Turia River (panel B, bottom). Survival is expressed as the base 10 logarithm of the plaque forming units detected per millilitre (PFU/ml) in samples taken at the times indicated on the x/y graph.

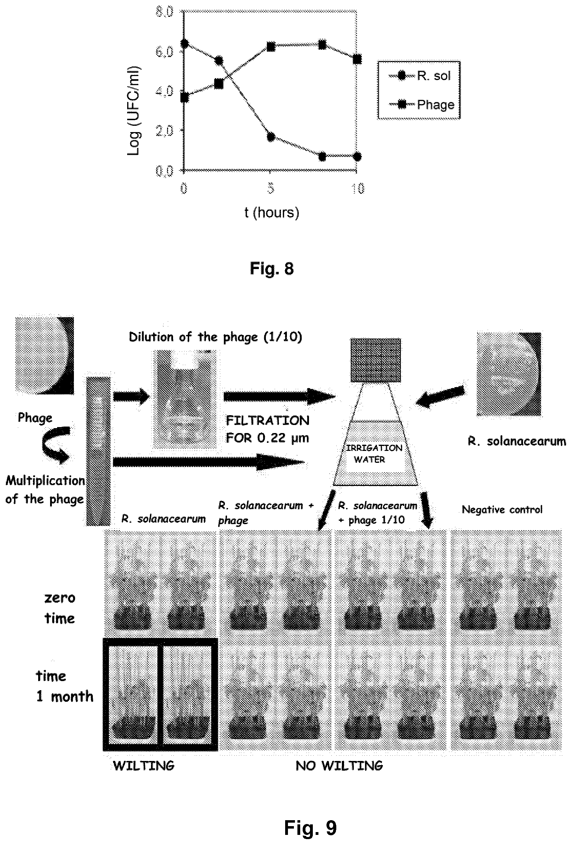

Fig. 8 shows a graph of the lytic activity of the bacteriophage vRsoP-WF2 added to an initial concentration of 10³ plaque forming units per millilitre (PFU/ml) in sterile river water, to which 10⁶ colony forming units per millilitre (CFU/ml) of *Ralstonia solanacearum* were added. A decrease within time of the CFU/ml corresponding to the bacteria (expressed in the form of the base 10 logarithm , points indicated with a filled circle) and an increase in PFU/ml corresponding to the bacteriophage (also expressed as the base 10 logarithm , points indicated with a filled square) were observed.
Fig. 9 shows an illustrating scheme of the experimental procedure of the use of the bacteriophages of the invention on irrigation water developed by the present inventors for the ability to control bacterial wilt. At the bottom, photographs of the condition of the plants at the beginning of the test (time zero, top row of photographs) and after 1 month (1 month, bottom row of photographs) are shown for each of the combinations of *R. solanacearum* and the bacteriophage vRsoP-WF2 indicated.

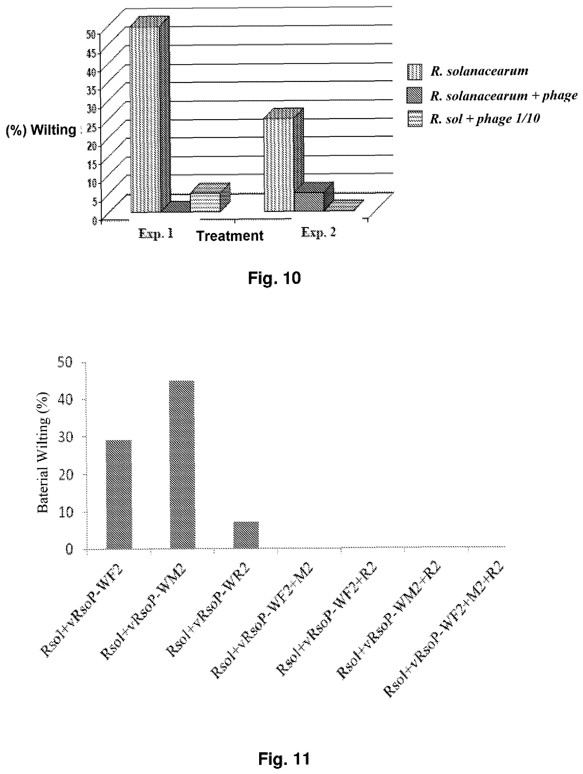

Fig. 10 shows a bar graph in which reduction of bacterial wilt, expressed as a percentage, in two different trials in tomato plants are shown. Experiment (Exp.) 1, bacteriophage concentration: 10⁹ plaque forming units per millilitre (PFU/ml) and Exp. 2 bacteriophage concentration: 10⁶ PFU/ml. In both experiments, the concentration of bacteria was 10⁵ colony forming units per millilitre (CFU/ml). Vertical frame bars correspond to those plants treated only with bacteria, without bacteriophages; bars without weaving correspond to plants treated with bacteria and bacteriophage at the indicated concentrations; bars with horizontal weaving correspond to plants treated with bacteria and 1/10 dilutions of the aforementioned concentrations of bacteriophages.
Fig. 11 shows a bar graph that represents reduction of bacterial wilt caused by *Ralstonia solanacearum,* expressed as a percentage, in trials where tomato plants were watered with water containing the combinations of bacteria (Rsol) and bacteriophage indicated under the bars. The four cases located further to the right correspond to irrigation water with binary combinations (from left to right, vRsoP-WF2 with vRsoP-WM2, vRsoP-WF2 with vRsoP-WR2, or vRsoP-WM2 with vRsoP-WR2) or tertiary combinations (vRsoP-WF2, vRsoP-WM2 and vRsoP-WR2) of bacteriophages with the bacteria.

### Detailed Description of the Invention

As mentioned hereinbefore, the invention relates to novel specific bacteriophages of *Ralstonia solanacearum* (bacteriophages of the invention), the use of at least one of the bacteriophages of the invention, or combinations , for *R. solanacearum* control in natural watercourses, natural water reservoirs, irrigation water and irrigation water reservoirs, by adding one or more of these bacteriophages to the aforementioned water or reservoirs; also it refers to the use of these bacteriophages to control *R. solanacearum* in soil, by adding one or more of these bacteriophages to the aforementioned soil via treated irrigation water; and a method for preventing or controlling bacterial wilt caused by *Ralstonia solanacearum* in plants, a composition is added to water to be used for watering plants, the aforementioned composition comprising at least one of the bacteriophages known as vRsoP-WF2 (DSM 32039), vRsoP-WM2 (DSM 32040) or vRsoP-WR2 (DSM 32041), or combinations , and the aforementioned plants are watered with the aforementioned treated water.

In the present application, the word "phage" is used as an abbreviation for the word "bacteriophage" with the same meaning. Therefore, hereinafter the two terms will be used interchangeably. "Bacteriophage" refers to a virus capable of infecting bacteria, either by producing lysis (lytic cycle) or by inserting itself into the genome and replicating itself therewith without causing lysis (lysogenic cycle).

The bacteriophages of the invention have been isolated from river water from various regions of Spain, specifically Badajoz, Salamanca and the Alpujarras (Granada).

Morphological characterization by electron microscopy and molecular characterization by DNA restriction analysis have shown that the virions belong to the *Podoviridae* family (specifically, the genus of T7-like viruses), a family of which so far only one bacteriophage has been described with lytic activity against the species formerly known as *Ralstonia solanacearum,* i.e. the bacteriophage ΦP5B1, described by Fujiwara et al (Fujiwara et al., 2011) and which has a larger genome than the bacteriophages of the present invention, whose genome does not exceed 41,000 base pairs (bp) in any of the three cases (see Table 2), while the ΦP5B1 genome has a size of 43,077 bp. Furthermore, the three inventions of other authors mentioned in the present application, relating to the use of bacteriophages to control *R. solanacearum* refer to the species formerly known as *Ralstonia solanacearum,* and, where information is available (patent documents, scientific publications, etc.), it can be confirmed that the strains dealt with are mostly reclassified as the new species *R. pseudosolanacearum.*

Therefore, the bacteriophages of the present invention do not belong to one of the most common families of bacteriophages lytic for the species formerly known as *R. solanacearum,* i.e. *Myoviridae,* but rather to a different family. In addition, they appear to be part of the same species, different from other species of T7-like viruses described so far. The discovery of viruses belonging to a new species of bacteriophages which attack *R. solanacearum* is an unexpected event.

The genome of any of the three bacteriophages of the present invention appears to have recognition targets for PstI restriction enzyme, which does not result in digestion of them, which represents a difference with lytic bacteriophages of Japanese Patent JP4532959- B2, wherein they are digested by the enzyme.

Thus, in the present invention are provided first data to identify the three bacteriophages of the present invention and distinguish them from any known bacteriophage, such as the family to which they belong, genus, the assignment of all of them to a single species, the sequence of the genome and distinctive restriction profile , obtained with several enzymes after digestion of the genome (see Examples 1 and 2, and Fig. 3, 4, 5 and 6). The isolation method used and a source of each is further described. Additionally, for clear definition , the deposit number issued by the Leibniz-Institut DSMZ-Deutsche Sammlung von Mikro-Organismen und Zellkulturen GmbH is provided, as authority for international deposit under the Budapest Treaty for each of the bacteriophages.

In addition to the structural characterization (morphological characterization of the viral particles and genomic characterization), in the present application the functional characterization (physiological and lytic) of the isolated bacteriophage is also described. As noted previously, one of the important characteristics for a biocontrol agent to be effective is to be shown to act on a wide range of strains. As shown hereinafter in the Examples section of the present application, the data previously described by Alvarez et al (Alvarez et al., 2006a, Alvarez et al., 2006b) were confirmed for a bacteriophage that was known to have been isolated from a watercourse in Spain that had not been specifically identified and for which insufficient structural data had been provided to ascribe them to a family and, much less, to a genus and specific species. In particular, the lytic capacity was confirmed for 30 strains that, at the time, were all considered to belong to the same species, i.e. the species formerly known as *R. solanacearum,* the phylotype of which was unknown, and the pH and temperature ranges in which it showed activity: between 14°C and 31°C, and a pH range of 6.5 to 8.2. These data correspond to the bacteriophage referred to in this application as vRsoP-WF2. It has been found that the other two bacteriophages of the present invention, vRsoP-WM2 and vRsoP-WR2 exhibit lytic capacity for the same strains of *Ralstonia solanacearum,* are active in the same ranges of pH and temperature, thus expanding the aforementioned characterization to the three bacteriophages of the present invention. The same results were obtained with mixtures of two of the bacteriophages (vRsoP-WF2 with vRsoP-WM2, vRsoP-WF2 with vRsoP-WR2, or WM2-vRsoP with vRsoP-WR2) or the combination of all three (vRsoP- WF2, vRsoP-WM2 and vRsoP-WR2). Thus, mixtures comprising combinations of these bacteriophages as well as the compositions comprising at least one of the bacteriophages of the present invention are a possible embodiment of the compositions of the present invention.

The specificity for *Ralstonia solanacearum* was also confirmed, no lytic activity being observed with bacteria isolated from river water with which tests were conducted, or with strains of other species of pathogenic plant bacteria. Again, these data are valid both for the three bacteriophages of the present invention and for combinations .

Therefore, the three bacteriophages separately, as well as combinations , fulfil the desirable characteristics for biological control agents such as high specificity by the host cell, and not posing a risk to the microbiota of water, soil or plants; being specific against *Ralstonia solanacearum.* Nor they pose a threat to the health of humans, animals or plants, being bacteriophages viruses that only infect bacteria. They are active also in a pH range compatible with the characteristics of different watercourses of Spain, and in a range compatible with the characteristics temperatures. This supports the use of bacteriophages of the present invention, individually or as combinations , and of compositions comprising such bacteriophages, to control *R. solanacearum,* whether in water from natural watercourses such as rivers, streams or creeks, natural reservoirs of water such as lakes, lagoons, ponds, springs and underground accumulations, artificial water reservoirs and dams, covered storage vessels, tanks, ponds (with or without surface covers), wells, irrigation water in general, or reservoirs of irrigation water as well as the aforementioned natural or artificial reservoirs.

In that sense, the field data collected by the present inventors on natural waters contaminated with *R. solanacearum* in different Spanish autonomous communities reveal that in the summer months (when the bacteria in water is detected and prohibits its use for irrigation) the highest daytime temperatures of these waters are between 13°C and 17°C and decrease at night. For example, in Salamanca and Guadalajara sampled water temperatures vary between 14°C and 4°C. In addition, in countries in central and northern Europe with environmental water contaminated with *R. solanacearum* temperatures are lower in the summer months. Therefore, the range of activity observed for the bacteriophages of the present invention is compatible with use in natural watercourses, particularly in Spain. So is the pH range of action . However, it is not easy to add bacteriophages to a river watercourse in sufficient amount to achieve effective control of microorganisms therein, especially in the particular place where this water will be drawn for irrigation, as bacteriophages will be very diluted and will be carried along watercourse so that although their survival time is very high, such use does not favour bacteriophages to come into contact with the host cell in the section of watercourse that may be of interest, unless bacteriophages are used in short watercourses and/or watercourses with reduced flow, such as rivulets, brooks and artificial pipes, especially those leading to a reservoir where the water will stay for a longer time. Therefore, it is preferred that use should take place in a natural or artificial water reservoir, such as lakes, lagoons, ponds, streams, reservoirs, covered vessels, tanks, ponds (with or without surface covering) or wells. In them, if contamination with *R. solanacearum* is suspected, it is easier to estimate the extent of such contamination and determine the amount or concentration of bacteriophages to add depending thereon. In any case, it is preferred that the water in the reservoir should be maintained at a temperature in the temperature range of 4°C and 30°C inclusive, interval in which the bacteriophages of the present invention survive prolonged periods, keeping their lytic activity, both alone and as part of compositions containing at least one of them. This temperature range also comprises the ambient temperatures of survival and/or multiplication of the pathogen *Ralstonia solanacearum,* i.e. the environmental range of 4°C and 24°C, so that the lytic activity of the bacteriophages of the present invention is effective at the temperatures that such bacteria presents a real threat of development of disease in crops. Since temperatures approaching 30°C are not common in reservoirs of environmental water, and taking into account fluctuations in daily and seasonal environmental temperature, conditions where the average water temperature in the reservoir is between 4°C and 24°C inclusive are preferred.

It should be noted that the tests described in Example 4 of the present application confirm the applicability of bacteriophages of the present invention via irrigation water and the usefulness in reducing damage caused by *R. solanacearum* wilting in plants. That is why the present invention also provides a method for preventing or controlling wilt caused by *Ralstonia solanacearum* in a plant, comprising the steps of adding to the water be used to water the plant a composition comprising at least one of the bacteriophages of the present invention, or combinations , and watering the plant with the aforementioned treated water.

The proposal of pre-treatment of irrigation water before use for irrigation is an option not considered in previous studies of Japanese authors discussed hereinbefore and is an option of great interest, since the main crops affected by *R. solanacearum* are irrigated crops. Thus, although it is compatible with the invention that plant cultivation may be carried out in any of the conditions under which cultivation is possible, a possible embodiment of the invention which may be very important use on plants growing in a field, in a nursery, in a greenhouse or any other type of substrate, or hydroponics, where it can be easy to plan and implement the method of the invention within the irrigation system and also do so in ways that benefit many plants simultaneously. Moreover, but perfectly compatible with the aforementioned embodiment, within the possible application to any crop of a species susceptible and/or tolerant to *R. solanacearum,* one embodiment of the invention of great interest is that in which the plant is a species belonging to the family of the *Solanaceae* (*Solanaceae* family) and in particular one in which the plant is selected from among tomatoes (*Solanum lycopersicum*), potatoes (the two crops most frequently affected) (*Solanum tuberosum*), sweet peppers (*Capsicum annuum*) or aubergines (*Solanum melongena*). The application of the method of the invention is perfectly compatible whether the plant is in a growing area dedicated to plants of a single species, or growing areas where there are plants of different species, usually with specific sections for each, as is the case of traditional orchards, usually with an irrigation system common to them all and a common irrigation water reservoir. The characteristics of the bacteriophages of the present invention allow for individual application (plant by plant), as is the case with the applications proposed by Japanese authors and with other biocontrol agents is not necessary, to be unnecessary.

Irrigation can be performed by any known system, such as traditional systems of partial or total flooding, drip irrigation, subsurface irrigation via perforated pipes, by exudation via porous pipes, or spray irrigation.

As discussed above, it is desirable that, prior to irrigation, the water which the composition with one or more bacteriophages of the present invention will be added to should be maintained at a temperature in the range of 4°C and 24°C which can be considered a usual environmental range, although, as bacteriophages of the invention are active up to a temperature of 31°C, this range can be extended to the range of 4°C and 30°C inclusive, although the latter value is unusual in environmental water reservoirs. As discussed above, conditions where the average water temperature in the reservoir is between 4°C and 24°C inclusive are considered suitable, given the daily and seasonal fluctuations of ambient temperature.

It is also desirable that the water pH should be in the range of 6.5 to 9.0 (both inclusive) to favour the lytic activity of the bacteriophage of the present invention.

For the same reasons discussed above, it is preferable that, prior to irrigation of the plant with water, irrigation water should stay in a reservoir, natural or artificial, from the time when the composition comprising one or more bacteriophages of the present invention is added; this approach is consistent with the addition of the bacteriophages when the water is not necessarily in such reservoir, but it is a watercourse that feeds or pours into the reservoir, especially when it is a pipe or a natural watercourse with a low flow rate that flows off of a natural watercourse with a high flow rate or a large reservoir, natural or artificial, such as a lake or a dam reservoir.

As for the reservoir itself, can be a storage vessel with or without surface coverage, including tank-type or pond-type; it can also be a natural accumulations of water, such as those that occur in the upwelling of certain springs, or artificial, natural or semi-natural wells as those formed in certain natural cavities, which are accessed by man at a later time.

On the other hand, it is important to note, as previously commented that one of the key points that determine the effectiveness of bacteriophages as biocontrol agents is their survival in the environmental conditions in which they are intended to be applied. In general, survival of the bacteriophages outside the host, as discussed previously, is extremely variable and depends on the particular nature of each bacteriophage, being strongly influenced by the surrounding environment, by conditions such as pH of the medium, temperature or sunlight (Iriarte et al., 2007). Because sunlight is a factor that often adversely affects the survival of bacteriophages under natural conditions, in considering the use in surface water, it is important for them to survive adequately in the presence of this factor. In this regard, the bacteriophages of the invention were isolated from different Spanish watercourses exposed to different levels of sunlight, unlike Japanese bacteriophages isolated from soil and plant material. Moreover, as bacteriophages of the invention were isolated from water samples in which host cells were present, the unexpected survival in water in the absence of host cells was unknown.

For all these reasons, survival of the bacteriophages isolated by the present inventors is considered an important factor, nearly a crucial feature that is an important advantage for use as a biocontrol agent in water. As described below in Example 3, the aforementioned survival was tested in the absence of the host cell in natural water from two Spanish rivers (the Tormes River in Salamanca and the Turia River in Valencia) of different chemical composition and pH, and at different temperatures. These two types of water show substantial differences in the main physico-chemical parameters referenced in the composition : specifically with the water of the Turia River values were comparatively 100 times higher in Mn, 10 times higher in Fe, between 5 and 10 times higher in chlorides and triple in nitrates; with water from the Tormes River values being approximately 4 times higher in phosphate; the average values of pH were around 8.13 in the water from the Turia River and 7.36 in the water from the Tormes river. Temperature ranges of water ranged from 3.5°C to 20.9°C for the Tormes River and from 11.5°C to 22.0°C for the water from the Turia River, i.e. temperature ranges for both environmental waters are within the temperature values used for testing survival of the bacteriophages, which were: 4°C, 14°C and 24°C, and the pH values were 7.2 for water from the Tormes River and 8.1 for the water from the Turia River. Of these rivers, the Tormes River is contaminated with *R. solanacearum* and the use of the water is prohibited for irrigation, while contamination has not been observed in the Turia River so far. For tests of the present invention, the aforementioned natural water was filtered through a 0.22 µη filter and sterilized, so that survival tests were performed in the absence of the host. As demonstrated in Example 3, the three bacteriophages of the present invention were active and at high levels of lytic activity for more than 5 months, longer than three months considered good survival period for bacteriophages of aquatic bacteria that affected fish and which are therefore in their natural environment (Pereira et al., 2011). This high survival was observed at the three temperatures tested (4°C, 14°C and 24°C), which were intended to cover the environmental range of interest for use in watercourses and natural reservoirs of water, artificial reservoirs and irrigation water. Subsequently, as mentioned in Example 3, the test was continued, and it was found that, after 3 years in natural water, they remain active. This long period of survival with maintenance of lytic activity is unexpected and surprising, particularly for a lytic bacteriophage of *R. solanacearum* because it is not a native bacteria from aquatic environments, but rather its natural environment is the xylem of plants and often the ground, and it was not expected that this bacteriophages specific of such bacteria would present a high survival rate in water outside the host. In fact, in the studies of Fujiwara et al (Fujiwara et al., 2011), for example, stability was monitored only for 15 days, in which clear differences were observed among the stability of the three bacteriophages tested, more pronounced in a buffer than in the presence of soil, with marked differences observed in the survivability of the two bacteriophages of the *Myoviridae* family, ΦRSL1 and ΦRSA1.

As discussed previously, survival outside the host varies greatly between different bacteriophages, even among those belonging to the same serotype/genotype (Brion et al., 2002) or even among those who share a common natural habitat such as water (Pereira et al., 2011), habitat in which survival of aquatic bacteriophages of at least three months previously was considered a suitable characteristic for selecting good candidates for the control of bacterial fish diseases transmitted via water. Thus, the survival of the bacteriophages isolated by the present inventors was not predictable at all, especially considering that not even the family to which they belong was known and a high survival rate in water was not expected, since the usual habitat of its host are plants and soil, not water.

Therefore, the survival results in the absence of the host are noteworthy of the bacteriophages of the invention obtained at 24°C because the previous results of the present inventors in survival studies of *R. solanacearum* in which lytic activity of other bacteriophages was tested in the presence of the aforementioned host bacteria indicated that the temperature of 24°C promotes the rate of disappearance of the pathogen with respect to a temperature of 14°C (Alvarez et al., 2007). However, at a temperature of 14°C, the bacteriophages of the invention maintain lytic activity on the host in natural water even after three years of the absence, and it has been observed that, in conditions similar to natural conditions, studies by the present inventors with other bacteriophages of this pathogen, lysis also causes a significant reduction of the populations of this bacterium (Alvarez et al., 2007). In addition, 14°C is a temperature closer to those recorded in most aquatic habitats where the pathogen has been detected in Spain and other European countries.

Moreover, it is noteworthy that it is common conserve biocontrol agents, prior to their use, at low temperatures, preferably at 4°C, but they can also be stored at 14°C, as well as 24°C. Therefore, it is noteworthy that the bacteriophages of the present invention remain active and at high levels at all three test temperatures for more than 5 months and the survival with lytic activity can be as long as a period of three years.

Therefore, a particularly novel feature of the bacteriophages of the present invention is the survival for more than 5 months in natural water in the absence of the host cell. This is an adequate and very advantageous feature for a biological control agent, which must have features that enable it to survive in the medium in which it is intended to be applied, in this case, water.

As a result, it is compatible with the use of the method and use of the present invention that the composition containing bacteriophages should be maintained during storage and/or use, preferably at a temperature in the range from 4°C to 24°C, inclusive, which can be considered a regular environmental range, however, since bacteriophages of the invention are active up to 31°C, this range may extend to a range from 4°C to 30°C inclusive, despite the latter value not being usual in environmental water reservoirs. As discussed above, an average temperature of the water in the reservoir from 4°C to 24°C inclusive, given the daily and seasonal fluctuations of ambient temperature, is also considered to be a suitable condition. This enables the compositions of the present invention to be easily preserved for a long time prior to their use in the form of suspensions in which the bacteriophages are in an aqueous vehicle which can be water (environmental, natural, distilled, previously sterilized, or subjected to another usual treatment for aqueous vehicles) or an aqueous solution (such as sterile saline, phosphate buffered saline, etc.) and ready to use and apply directly where needed. Therefore, the compositions of the present invention may comprise any carrier or excipient agronomically acceptable, and may be in liquid form, e.g. as an aqueous suspension, which can be prepared in water or in an aqueous solution and/or dilutions . In this way, they can be used to control *R. solanacearum* and can be applied with the method of prevention or treatment of wilt caused by the aforementioned bacteria and can be therefore ready for direct use from the stored and marketed form .

The high survival rate, with maintained lytic activity on the host, of the bacteriophages of the present invention, favours the use in the field because they can be transferred directly via water, a natural and simple way, without encapsulating or adding other physical, chemical and/or biological mediums to protect their viability until coming in contact with the target cell. This facilitates the production process, lowers costs and eliminates the need for complex formulations for implementation as well as the addition of chemicals to the environment. Thus, the high survival rate of the bacteriophages in water in the absence of the target cell reduces the implementation costs by decreasing the number of uses required over time, and increases long-term product efficiency in the agricultural systems where they are intended to be applied, and can thus more effectively prevent outbreaks of disease caused by *R. solanacearum.* All this leads to a product with more "added value" for farmers and nursery keepers, the major potential consumers of the aforementioned product, i.e. the bacteriophages of the present invention and/or compositions comprising them.

Moreover, this high survival in natural water while in the extracellular state facilitates combination with other control strategies (chemical and/or physical, and even biological) for the same plant pathogen or others, which may be an additional optional step of the method of the present invention. The method of the present invention is also compatible with the use of copper compounds, antibiotics and/or soil fumigants, whose application to the soil where plant is growing can also be considered an additional optional step of the method of the present invention.

The present invention is also compatible with additional use not only an agent of chemical or physical control, but rather as one or more additional biological control agents other than any of the bacteriophages of the present invention (other microorganisms such as bacteria, fungi and other bacteriophages, etc.). One possibility is any of the lytic or lysogenic bacteriophages previously known, which have activity against the aforementioned bacteria. For use, the additional agent may be further comprised in a composition of the present invention, or may be applied separately.

Although the preferred form of the compositions of the present invention is the liquid form, in aqueous medium, especially when applied to water to control *R. solanacearum* and/or preventing or reducing bacterial wilt caused by the aforementioned bacteria in plants which are to be irrigated with the aforementioned water, other forms of the composition are also compatible with the invention, especially those known to those skilled in the art for the conservation of bacteriophages, such as in a lyophilized form (which facilitates preservation at room temperature) or as a refrigerated and/or frozen aqueous suspension, preferably from 4°C to -20°C, and even lower temperatures, such as - 20°C to -80°C.

As already mentioned, the compositions of the present invention may contain one of three bacteriophages whose isolation and morphological and genomic characterization is described in the present application (vRsoP-WF2, vRsoP-WM2 or vRsoP-WR2), or combinations (vRsoP-WF2 and vRsoP-WM2, vRsoP-WF2 and vRsoP-WR2, vRsoP-WM2 and vRsoP-WR2, or vRsoP-WF2, vRsoP-WM2 and vRsoP-WR2). The tests performed and described in Example 4 suggest that combinations, either two of or all three of the bacteriophages of the present invention are more effective than the use of the bacteriophages separately, so they may be a good choice for use against *Ralstonia solanacearum* in water to be treated, and particularly in water that is to be used for irrigation in order to prevent or reduce wilt caused by this bacteria. In compositions with combinations of several bacteriophages, each may be at the same concentration as in Example 4 of the present application, but different concentrations are also compatible with the invention.

Moreover, an advantage to consider of using the combination of two or more bacteriophages of the present invention is that mixtures prevent the appearance of strains of *R. solanacearum* that are resistant to the lytic action of any one of them.

Regarding the total concentration of bacteriophages in the compositions of the present invention, there are no limitations except those imposed for chemical reasons, resulting in the suspension being saturated and bacteriophages precipitating or settling. However, in practice, this is highly unlikely. One option is for the total concentration of bacteriophages of the invention to range between 10⁵ and 10⁹ plaque forming units per millilitre (PFU/ml), which are concentrations that have been tested in the Examples section of the present application and which can also be a suggested range of concentrations in order to choose the final concentration of bacteriophages desired to be present in the irrigation water. However, concentrations may be higher or lower than those included in that range, with concentrations of 10³ PFU/ml being able to be maintained and/or used as in section 4.1 of Example 4, or even lower, as the present inventors have obtained lysis data in liquid medium with bacteriophages of the present invention at concentrations of about 10² PFU/ml. Thus, the range 10² to 10⁹ PFU/ml or 10³ to 10⁹ PFU/mL are also possible concentration ranges of the compositions of the present invention or the conditions of action of the bacteriophages of the present invention, as well as other upper or lower limits, since the bacteriophages multiply inside the bacteria.

As far as the different bacteriophages of the present invention, under the envisaged use and/or maintenance conditions, survival data may lead to a preference for vRsoP-WM2, while macrotests carried out on plants described in Example 4, specifically on tomato plants, can lead to a preference for vRsoP-WR2, because in such tests a greater reduction in bacterial wilt was observed from applying this bacteriophage individually with respect to the other two bacteriophages of the present invention.

The invention will now be explained in more detail by the Examples and Figures described hereinafter.

### Examples

### - Example 1. Origin and Isolation of the Bacteriophages.

Lytic bacteriophages against *R. solanacearum* were isolated from several rivers of Castilla-Leon, Extremadura and Andalusia, in the vicinity of fields affected by bacterial wilt. A selection of these bacteriophages was purified and their lytic activity was tested in the laboratory against *R. solanacearum,* as shown in Fig. 1.

Among them, three bacteriophages (vRsoP-WF2, vRsoP-WM2 and vRsoP-WR2), from different origins, were chosen for further characterization.
- VRsoP-WF2: isolated from the Tormes River in the vicinity of Salamanca.
- VRsoP-WM2: isolated from the Cayo River in the province of Badajoz.
- VRsoP-WR2: isolated from the Yator River in the area of the Alpujarras, in the province of Granada.

The three bacteriophages were purified via successive plaque passages in general LPGA medium (yeast extract [5g] - peptone [5 g] - glucose [10 g] - agar [20 g], dissolved in distilled water [1 litre]; the glucose is sterilized by filtration and subsequently added to the rest of the medium sterilized by autoclaving) with host cells of a standard strain of *R. solanacearum* (IVIA 1602.1 strain, deposited at the French Collection of Plant-associated Bacteria [CFBP] under CFBP number 4944 and at the DSMZ free access collection under the number DSM 100387). This method is also preferred for amplification of any of the aforementioned bacteriophages of the present invention on a solid medium.

Once purified, the lytic activity, pH and temperature range, and range of hosts of them were characterized as described hereinafter.

### 1.1. Temperature Range.

The lytic activity observed of the characterized bacteriophages against the selected strain of *R. solanacearum* (IVIA-1602.1) is observed between 14°C and 31°C in all three cases.

Within this range, the routine incubation temperature of the bacteriophages to multiply in the host, both on solid medium and in liquid medium, under laboratory conditions, is between 28-30°C, because they are the values that are considered optimal for the growth of *R. solanacearum* in these conditions.

Mixtures of two (vRsoP-WF2 - vRsoP-WM2, vRsoP-WF2 - vRsoP-WR2, vRsoP-WM2 - vRsoP-WR2) or all three bacteriophages (vRsoP-WF2 - vRsoP-WM2 - vRsoP-WR2) were also tested. Each of the four mixtures of bacteriophages also showed activity in the same temperature range.

### 1.2. pH range.

The lytic activity observed of the characterized bacteriophages against the selected strain of R. solanacearum (IVIA-1602.1) tested in different irrigation water, river water, canal water and lake water, was positive in all of the aforementioned types of water at pHs ranging between 6.5 and 9.0. Similarly, the mixtures of bacteriophages showed lytic activity in the same irrigation water and, thus, within the same pH range. The minimum and maximum pH values for the lytic activity of the bacteriophages have not yet been determined.

### 1.3. Salinity

Since it has been reported that *R. solanacearum* can grow in the presence of NaCl at concentrations of 1%, and even sometimes up to 2%, the lytic activity of bacteriophages in brackish water of different origins was tested, with salt concentrations of about 1.5%. Lytic activity was observed with all three bacteriophages. The four mixtures of bacteriophages of the invention mentioned in point 1 .1 also showed lytic activity in the tested salinity conditions.

### 1.4. Visible Light

Since visible light can sometimes affect the lytic activity of bacteriophages, their activity was tested in conditions of light and darkness. It was observed that after 48 hours of uninterrupted exposure to intense light (approximately 15,000 lux), the lytic activity was similar to that observed in dark conditions, so the presence of light does not affect the lytic activity of the bacteriophages of the invention. Similarly, the different mixtures of tested bacteriophages had similar lytic activity both in the presence of light and in darkness.

### 1.5. Aeration

Since *R. solanacearum* is an aerobic bacteria and typically grows in liquid medium with stirring (aeration), the effect of the absence of aeration on the lytic activity was determined, as in field conditions aeration is not always assured (by example in tank storage). Activity was observed both in the presence and absence of stirring, of the same magnitude, being faster with aeration. Similarly, the mixtures of the bacteriophages showed lytic activity both with and without aeration.

### 1.6. Specificity

Specificity was tested in Petri dishes against bacterial lawns of *R. solanacearum* strains in the LPGA general culture medium, on which two drops of a suspension of each of the three bacteriophages (Fig. 2) were poured. The lytic activity was visualized by the appearance of areas of clearance formed by lysed bacteria in the bacterial lawn where drops of the suspensions with bacteriophages were placed (Fig. 2, corresponding to the test on *R. solanacearum* strain IVIA - 1602.1).

According to experimental data, the lytic activity of the characterized bacteriophages was positive for 35 strains of *R. solanacearum* of different origins, hosts and years of isolation (Table 1). Among the aforementioned, 13 are international in scope and/or standard. The remaining are all strains isolated in Spain, belonging to the collection of the Valencian Institute of Agricultural Research (IVIA).

**Table 1. R. solanacearum strains sensitive to the lytic action of the three bacteriophages of the present invention.**

| **STRAIN CODE** | **COUNTRY OF ORIGIN** | **HOST** | **YEAR** |
|---|---|---|---|
| **International Strains** | | | |
| NCPPB^{a} 1115 | United Kingdom (Ex Egypt) | Potato | 1961 |
| NCPPB 1584 | Cyprus | Potato | 1963 |
| NCPPB 2505 | Sweden | Potato | 1972 |
| NCPPB 2797 | Sweden | *Solanum dulcamara* | 1974 |
| BR 264 | United Kingdom | *Solanum dulcamara* | 1993 |
| Bordeaux 11-47 | France | Aubergine | 1994 |
| Nantes 9-46 | France | Tomato | 1994 |
| 550 | Belgium (Ex Turkey) | Potato | 1995 |
| IPO-1609 | Netherlands | Potato | 1995 |
| Port 448 | Portugal | Potato | 1995 |
| W 12 | Belgium | Potato | 1996 |
| WE 4-96 | United Kingdom | River Water | 1996 |
| Tom 1 | United Kingdom | Tomato | 1997 |

| **Strains from Spain** | | | |
|---|---|---|---|
| IVIA^{b} -1602.1 | Canary Islands | Potato | 1996 |
| IVIA-2049.53 | Canary Islands | Soil | 1999 |
| IVIA-2068.58a | Canary Islands | Potato | 1999 |
| IVIA-2068.61a | Canary Islands | Potato | 1999 |
| IVIA-2093.3.1 | Canary Islands | Potato | 1999 |
| IVIA-2093.5T.1a | Canary Islands | Potato | 1999 |
| IVIA-2128.1b | Castile-Leon | Potato | 1999 |
| IVIA-2128.3a | Castile-Leon | Potato | 1999 |
| IVIA-2167.1a | Castile-Leon | River Water | 1999 |
| IVIA-2167.2b | Castile-Leon | River Water | 1999 |
| IVIA-2528.A₁₋₂ | Castile-Leon | River Water | 2001 |
| IVIA-2528.A₃.1 | Castile-Leon | River Water | 2001 |
| IVIA-2528.54.A₂ | Castile-Leon | River Water | 2001 |
| IVIA-2751.11 | Extremadura | River Water | 2003 |
| IVIA-2762.1 | Extremadura | Tomato | 2003 |
| IVIA-2762.4 | Extremadura | Tomato | 2003 |
| IVIA-3090.1 | Andalusia | Tomato | 2005 |
| IVIA-3090.5 | Andalusia | Tomato | 2005 |
| IVIA-3205.A.22 | Castile-La Mancha | River Water | 2006 |
| IVIA-3243 | Andalusia | Tomato | 2006 |
| IVIA-3359.9 | Castile-La Mancha | River Water | 2007 |
| IVIA-3359.10 | Castile-La Mancha | River Water | 2007 |

| | | | |
|---|---|---|---|
| ^{a}NCPPB: National Collection of Plant Pathogenic Bacteria, United Kingdom. ^{b}IVIA: Bacteria Collection of the Valencian Institute of Agricultural Research, Spain. | | | |

Strains from the NCPPB are available in this international collection. The other strains are available in the IVIA collection of phytopathogenic bacteria.

Specificity was also tested against other species of plant pathogenic bacteria and various bacterial isolates of river water to assess the possible impact of the isolated bacteriophages on the microbiota of natural water.

The lytic activity was negative for the 14 bacterial isolates of river water in the tests, which were selected from various water samples and presented different colonial morphologies from each other and with respect to the host. The activity was also negative for the 11 tested phytopathogenic bacteria strains belonging to other genera, demonstrating the specificity of the selected bacteriophages against *Ralstonia solanacearum.* The same results were obtained with the four possible mixtures of the aforementioned bacteriophages.

### - Example 2. Structural Characterization: Morphological and Molecular Characterization.

### 2.1. Morphological Characterization.

A study of the morphology of the selected bacteriophages was carried out via transmission electron microscopy of the viral particles after negative staining with phosphotungstic acid. It is observed that they present the characteristic morphology of the *Podoviridae* family: polygonal, non-enveloped heads 40 to 60 nm in diameter and short tails (Fig. 3). The bacteriophages of this family are also characterized by a genome of double-stranded DNA, a fact which was confirmed in the tests described below.

### 2.2. Molecular Characterization.

### 2.2.1. Extraction of the DNA of the Three Bacteriophages.

Concentrated capsid suspensions were obtained from the three types of bacteriophages from the corresponding bacterial lysates (filtered and treated with DNAse and RNAse to degrade the bacterial nucleic acids), by polyethylene glycol capsid precipitation protocol. After treatment of the aforementioned capsids with proteinase K, extraction of genomic DNA was performed after the addition of phenol, chloroform and isoamyl alcohol. After confirming that concentration and purity were adequate, the obtained DNAs were analysed by electrophoresis in agarose gel to verify the integrity as a preliminary step to the restriction analysis (see section 2.2.2) and purification for subsequent sequencing (see section 2.2.3).

### 2.2.2. Restriction Analysis of the Genomes of the Three Bacteriophages.

From the obtained genomic DNAs of the three bacteriophages, restriction analysis was carried out with various restriction enzymes, chosen to give a banding pattern belonging to T7 genus bacteriophages of the *Podoviridae* family. These enzymes were *KpnI, ScaI, SpeI and XmnI. PstI* was also tested because it is an enzyme used to cut the genome of bacteriophages of the species formerly known as *R. solanacearum* described in Japanese Patent JP4532959-B2 (publication number JP2005278513).

As shown in Fig 4, the profile bands obtained with these five restriction enzymes is apparently the same for the three bacteriophages: complete digestion with *XmnI* and partial digestion with *KpnI, ScaI and SpeI* was observed, while no *PstI* digestion was appreciable. These results indicate the genetic proximity of the three bacteriophages to each other, and the difference from the bacteriophages of Japanese patent application JP4532959-B2, whose genomes themselves are cut by *PstI.*

### 2.2.3. Mass Sequencing of Genomic DNAs of the Three Bacteriophages and Bioinformatic Analysis.

From the resulting genomic DNA belonging to each of the three bacteriophages, we proceeded to the massive sequencing of the nucleotide bases and subsequent bioinformatic analysis and complete annotation of the genomic sequences found (SEQ ID NO: 1, corresponding to vRsoP-WF2; SEQ ID NO: 2, corresponding to vRsoP-WM2 and SEQ ID NO: 3, corresponding to vRsoP-WR2). This part was entrusted to the Valgenetics, S.L. (University of Valencia Science Park, Valencia, Spain).

The main findings were as follows:
The assembly of sequences obtained by massive sequencing yielded final sequences assembled with 100% fidelity, whose sizes are shown in Table 2.

**Table 2. Size of the Genomic Sequences Obtained for Each Bacteriophage.**

| SEQ ID NO: | Bacteriophage | Number of Base Pairs (bp) |
|---|---|---|
| 1 | vRsoP-WF2 | 40,409 |
| 2 | vRsoP-WM2 | 40,861 |
| 3 | vRsoP-WR2 | 40,408 |

The results indicated that each of the majority sequences included in the samples of SEQ ID NO: 1 (corresponding to vRsoP-WF2), SEQ ID NO: 2 (corresponding to vRsoP-WM2) and SEQ ID NO: 3 (corresponding to vRsoP-WR2) is readily identifiable as a complete genome of a bacteriophage belonging to the genus of T7-like viruses, which is the type species of enterobacteria bacteriophage known as T7 (enterobacteria phage T7), which belongs to the *Podoviridae* family.

Comparing the genomes of the three bacteriophages, it was showed that they were 99% identical over the whole of the genome. However, analysis of these sequences made obvious the presence of small genomic differences such as mutations, insertions and deletions distributed throughout the genome. These differences are higher in the sequence of SEQ ID NO: 2 (corresponding to vRsoP-WM2) than in the sequences of SEQ ID NO: 1 (corresponding to vRsoP-WF2) and SEQ ID NO: 3 (corresponding to vRsoP-WR2), which are almost identical. Thus, the sequence of SEQ ID NO: 2 contains an insertion of 468 nucleotides compared to the sequences of SEQ ID NO: 1 and SEQ ID NO: 3. Fig. 5 shows the sequence alignment extract corresponding to the area of the insertion. Small differences found in the nucleotide sequences indicated that bacteriophages vRsoP-WF2, vRsoP-WM2 and vRsoP-WR2 are different bacteriophages of the same viral species (Table 3).

**Table 3. Comparison of the Sequences of the Genomes of Bacteriophages vRsoP-WF2, vRsoP-WM2 and vRsoP-WR2**

| **Compared Sequence (SEQ ID NO:)** | **Pattern Sequence of the Comparison (SEQ ID NO:)** | **Coverage*** | **Identity**** |
|---|---|---|---|
| 1 | 2 | 98% | 99% |
| 1 | 3 | 100% | 99% |
| 2 | 3 | 99% | 99% |

| | | | |
|---|---|---|---|
| *Homology between sequences of compared genomes, expressed as a percentage. **Nucleotides matching within areas of homology of the genomes compared, expressed as a percentage. | | | |

Additionally, using the *BlastN* and *Blast2Seq* analyses carried out with the tools accessible to the public via the website of the National Center for Biotechnology Information of the USA (http://www.ncbi.nlm.nih.gov/), it was found that the genomes of bacteriophages vRsoP-WF2, vRsoP-WM2 and vRsoP-WR2 exhibit some regions with high identity (about 70%) with bacteriophages: *Ralstonia* RSB1, *Vibrio VP4* and, especially, *Rhizobium* RHEph01, all of them being T7-like bacteriophages (Table 4). These regions (corresponding to 5-23% of the entire genome of bacteriophages vRsoP-WF2, vRsoP-WM2 and vRsoP-WR2) belong to highly conserved regions.

**Table 4. Comparison of the Sequences of the Genomes of Bacteriophages vRsoP-WF2, vRsoP-WM2 and vRsoP-WR2 with Several T7-like Bacteriophage Genomes.**

| **Compared Sequence (SEQ ID NO:)** | **Genome of the Virus Pattern Sequence of the Comparison (GenBank Access Number)** | **Coverage*** | **Identity**** |
|---|---|---|---|
| 1 | Φ *Ralstonia* RSB1 (AB597179.1) | 2% | 84% |
| 1 | Φ T7 (NC_001604.1) | 5% | 67% |
| 1 | Φ *Rhizobium* RHEph01 (JX483873.1) | 19% | 68% |
| 1 | Φ *Vibrio* VP4 (NC_007149.1) | 5% | 70% |
| 2 | Φ *Ralstonia* RSB1 (AB597179.1) | 15% | 66% |
| 2 | Φ T7 (NC_001604.1) | 5% | 67% |
| 2 | Φ *Rhizobium* RHEph01 (JX483873.1) | 23% | 68% |
| 2 | Φ *Vibrio* VP4 (NC_007149.1) | 4% | 70% |
| 3 | Φ *Ralstonia* RSB1 (AB597179.1) | 15% | 66% |
| 3 | Φ T7 (NC_001604.1) | 5% | 67% |
| 3 | Φ *Rhizobium* RHEph01 (JX483873.1) | 22% | 68% |
| 3 | Φ *Vibrio* VP4 (NC_007149.1) | 2% | 70% |

| | | | |
|---|---|---|---|
| *Homology between sequences of compared genomes, expressed as a percentage. **Nucleotides matching within areas of homology of the genomes compared, expressed as a percentage. | | | |

These results reveal that, except in these conserved regions within the T7-like bacteriophages, the genomes of bacteriophages vRsoP-WF2, vRsoP-WM2 and vRsoP-WR2 contain a highly divergent nucleotide sequence with respect to other bacteriophages deposited in GenBank. Therefore, these high differences in nucleotide sequence guarantee that bacteriophages vRsoP-WF2, vRsoP-WM2 and vRsoP-WR2 correspond to a new species within the genus of T7-like viruses.

Moreover, the identification of open reading frames (ORF) and characteristic features of the bacteriophages revealed that the sequences of the genomes of bacteriophages vRsoP-WF2, vRsoP-WM2 and vRsoP-WR2 have genomic organization and ORF expression that is mutually similar, and similar to T7-like bacteriophages in part of the genome (Fig. 6).

In summary, the three bacteriophages of the present invention are three isolates of the same viral species, being a new species classified as belonging to the genus T7 of the *Podoviridae* family, with organization very similar but distinct from the T7 bacteriophages deposited in GenBank (Fig. 6). The novel bacteriophages have different sequences to T7 bacteriophages, only resembling in some highly conserved areas, such as those related to replication and encapsidation.

### - Example 3. Survival of the Three Bacteriophages in Natural River Water.

The survival of the three selected bacteriophages was tested in two different types of river water: Tormes, from Salamanca, and Turia, from Valencia, both in Spain. These two types of water show substantial differences in the main physico-chemical parameters analysed of the composition: specifically with the water of the Turia River values were comparatively 100 times higher for Mn, 10 times higher for Fe, between 5 and 10 times higher for chlorides and triple for nitrates; with water from the Tormes River, values were approximately 4 times higher in phosphate; the average values of pH were around 8.13 in the water from the Turia River and 7.36 in the water from the Tormes river. Temperature ranges of water ranged from 3.5°C to 20.9°C for the Tormes River and from 11.5°C to 22.0°C for the water from the Turia River, i.e. temperature ranges for both types of environmental water are within the temperature values used for testing survival of the bacteriophages, which were: 4°C, 14°C and 24°C, and the pH values were 7.2 for water from the Tormes River and 8.1 for the water from the Turia River.

Surprisingly, it was observed that all the bacteriophages maintained their lytic activity against *R. solanacearum* after more than five months under these conditions, in the absence of the host since, prior to inoculation, the water had been filtered through a 0.22 µm filter and autoclaved.

Fig. 7 shows graphics for the evolution of plaque forming units per millilitre (PFU/ml) of the bacteriophages in both the water from the Tormes River (panel A) and from the Turia River (panel B), in samples incubated at 14°C. It is observed that PFU/ml are maintained in the absence of *Ralstonia solanacearum.* The survival curves of the three bacteriophages samples kept at 4°C and 24°C were similar.

Thus, it is noteworthy that the three bacteriophages are active and have high lytic activity at all three temperatures tested for more than 5 months.

In addition, survival and maintenance of the lytic activity of all bacteriophages were confirmed at 4°C and 14°C for a period of time as long as three years. This result is important for conservation within this temperature range when such long storage periods are required.

### - Example 4. Biocontrol of Bacterial Wilt Caused by R. solanacearum.

### 4.1. Ability to Control Bacterial Populations in Natural River Water.

Since the three bacteriophages of the invention had similar lytic activity at the different tested temperatures and pH values in natural water, initially one of them was chosen as a model (bacteriophage vRsoP-WF2) to perform biocontrol tests of bacterial wilt caused by *R. solanacearum.*

A bacteria-bacteriophage coinoculation test was carried out in sterile river water, in a closed system controlled in the laboratory, for simultaneous quantification of the population levels of both microorganisms over time. To do this, the bacteria was inoculated at a concentration of 10⁶ of colony forming units per millilitre (CFU/ml) in the liquid medium (sterile river water) and the bacteriophage was added at a concentration of 10³ plaque forming units per millilitre (PFU/ml). As shown in Fig. 8, it was confirmed that populations of the inoculated bacteria (reference strain IVIA-1602.1 of *R. solanacearum*) descended substantially in a few hours due to the lytic activity of the inoculated bacteriophages (bacteriophage vRsoP-WF2), the aforementioned pathogenic bacteria virtually disappearing after about 10 hours.

### 4.2. Tests of Biocontrol of Bacterial Wilt in Host Plants: Bacteriophage vRsoP-WF2.

The ability of the river water bacteriophage vRsoP-WF2 for biocontrol of the disease caused by *R. solanacearum* was tested in two independent experiments, watering plants of a susceptible host (tomato plants) with a concentration of the standard bacterial strain IVIA 1602.1 (10⁵ CFU/ml) and two different concentrations of the aforementioned bacteriophage (10⁶ and 10⁹ PFU/ml), and the decimal dilutions (10⁵ and 10⁸ PFU/ml, respectively), in conditions of optimum temperature and humidity for the development of the disease. The experimental procedure is shown in Fig. 9.

The results of both experiences are shown in Fig. 10. Overall, the disease incidence decreased to 0-5% in plants irrigated with the pathogen and the bacteriophage, in independent experiments, while in the controls without bacteriophages wilt incidence was 25-50%.

### 4.3. Tests of Biocontrol of Bacterial Wilt in Host Plants: vRsoP-WF2, vRsoP-WM2, vRsoP-WR2, and their Combinations.

Similar to the experiments of biocontrol in plants carried out with the bacteriophage vRsoP-WF2 described in section 4.2, a macrotest was carried out, which could simultaneously study the ability for biocontrol of each of the three bacteriophages vRsoP-WF2, vRsoP-WM2 and vRsoP-WR2, separately as well as in combinations of two and a mixture of all three. This is considered a macroassay, since it is performed with a large number of plants requiring sufficient room for incubation and people qualified for carrying it out. In all cases, tomato plants, which are a host susceptible to the pathogen, were tested, wherein approximately 35 plants per experimental condition were inoculated, which is approximately 315 plants. Inoculated plants were kept in a climatic chamber of suitable size, in day/night cycles of 16 hours of light at 26°C and 8 hours dark at 22°C and a humidity of about 70%, in conditions of biological containment, in a BSL3 laboratory. In the cited macroassay, the concentration of *R. solanacearum* (strain IVIA 1602.1) in irrigation water was 10⁵ CFU/ml, while the total concentration of bacteriophages was 10⁷ PFU/ml in all the tested experimental conditions.

The graph of Fig. 11 shows the results obtained. These results indicate that:
- The bacteriophage vRsoP-WR2 is the most effective of the three, resulting in a greater decrease of bacterial wilt when added to irrigation water at the same concentration as the other two bacteriophages.
- Any mixture of the bacteriophages (either binary combinations, or the combination including the three) are more effective than the separate bacteriophages.

All these experiments demonstrate the lytic potential of the bacteriophages of the present invention, isolated at different places in Spain, for the biocontrol of *R. solanacearum* and, therefore, the applicability of the aforementioned lytic activity in both the treatment of environmental water for agricultural use that has been contaminated with the aforementioned pathogen, or other uses, such as in the prevention and/or control of the disease caused in the field. This biocontrol ability is especially important, since it is considered that there is currently no effective control methods available via soil or water. And, in this case, as previously discussed and demonstrated in the aforementioned experiments, the biocontrol agents provided by the present invention have the unexpected feature of a high survival rate in water under normal environmental temperatures in Spain, which it is an advantage for use on plants via irrigation water and for the control and prevention of the presence of *R. solanacearum* therein, and for easy and prolonged maintenance of the marketed forms of the bacteriophages of the invention prior to use. Such maintenance may take place in an aqueous medium for a long time without severe loss of lytic activity and not even require, prior to the application to water, pre-dilution of the bacteriophages or their mixtures with any kind of physical or chemical vehicle to facilitate the interaction with the target bacteria or to ensure the stability before coming into contact with it, so that applying bacteriophages to irrigation water, water streams or water reservoirs can be simply by pouring them into *R. solanacearum* contaminated water to be controlled.

### Deposit of Microorganisms

The bacteriophages vRsoP-WF2, vRsoP-WM2 and vRsoP-WR2, with ability to lyse *R. solanacearum* cells, have been deposited in the German Collection of microbial cultures Leibniz-Institut DSMZ-Deutsche Sammlung von Mikro-organismen und Zellkulturen GmbH, Inhoffenstrasse 7B, 38124 Braunschweig, Germany, following the rules of the Budapest Treaty for the deposit of microorganisms for patent purposes, on the following dates and assigned the following access numbers (Table 5).

**Table 5. Data on the Deposit of Bacteriophages in the DSMZ German Collection.**

| **Material** | **Deposit Date** | **Access Number** |
|---|---|---|
| vRsoP-WF2 | 15 April 2015 | DSM 32039 |
| vRsoP-WM2 | 15 April 2015 | DSM 32040 |
| vRsoP-WR2 | 15 April 2015 | DSM 32041 |

### References

Addy, H.S., Askora, A., Kawasaki, T, Fujie, M. & Yamada, T. 2012. Utilization of filamentous phage RSM3 to control bacterial wilt caused by Ralstonia solanacearum. Plant Diseases. 96:1204-1209.
Álvarez, B., Biosca, E.G. & López, M.M. 2006a. River water biota affecting Ralstonia solanacearum survival: characterization of specific bacteriophages and its potential use for biocontrol in irrigation water. The 4th International Bacterial Wilt Symposium. Abst. p. 46. York (UK).
Álvarez, B., Biosca, E.G. & López, M.M. 2006b. Caracterización de fagos líticos de Ralstonia solanacearum aislados de agua de río: uso potencial en biocontrol. XIII Congreso de la Sociedad Española de Fitopatología. Abst. p. 62. Murcia
Álvarez, B., López, M.M. & Biosca, E.G. 2007. Influence of native microbiota on survival of Ralstonia solanacearum phylotype II in river water microcosmos. Appl. Environ. Microbiol. 73:7210-7217.
Álvarez, B., López, M.M. & Biosca, E.G. 2008. Survival strategies and pathogenicity of Ralstonia solanacearum phylotype II subjected to prolonged starvation in environmental water microcosms. Microbiology. 154:3590-3598.
Álvarez, B., Biosca, E.G. & López, M.M. 2010. On the life of Ralstonia solanacearum, a destructive bacterial plant pathogen. En: Current research, technology and education topics in applied microbiology and microbial biotechnology. Mendez Vilas, A. ed., pp. 267-279. World Scientific Publishing, Singapore.
Anonymous. 1998. Council Directive 98/57/EC of 20 July 1998 on the control of Ralstonia solanacearum (Smith) Yabuuchi et al. Off J Eur Communities L235, 1-39.
Anonymous. 2000. Council Directive 2000/29/EC of 8 May 2000 on protective measures against the introduction into the Community of organisms harmful to plants or plant products and against their spread within the Community. Off J Eur Communities L169, 1-112.
Anonymous. 2006. Commission Directive 2006/63/EC of 14 July 2006: amending Annexes II to VII to Council Directive 98/57/EC on the control of Ralstonia solanacearum (Smith) Yabuuchi et al. Off J Eur Communities L206, 36-106.
Bae, J.Y., Wu, J., Lee, H.J., Jo, E.J., Murugaiyan, S., Chung, E. & Lee, S.-W. 2012. Biocontrol potential of a lytic bacteriophage PE 204 against bacterial wilt of tomato. J. Microbiol. Biotechnol. 22(12):1616-1620
Balogh, B., Jones, J.B., Iriarte, F.B., & Momol, M.T. 2010. Phage therapy for plant disease control. Curr. Pharm. Biotechnol. 11(1):48-57.
Brion, G.M., Meschke, J.S. & Sobsey, M.D. 2002. F-specific RNA coliphages: occurrence, types, and survival in natural waters. Water Research. 36:2419-2
Bhunchoth, A., Phironit, N., Leksomboon, C., Chatchatwankanphanich, O., Kotera, S., Narulita, E., Kawasaki, T., Fujie, M. & Yamada, T. 2015. Isolation of Ralstonia solanacearum-infecting bacteriophages from tomato fields in Chiang Mai, Thailand, and their experimental use as biocontrol agents. J.Appl. Microbiol. 118:1023-1033.
Caruso, P., Palomo, J.L., Bertolini, E., Álvarez, B., López, M.M. & Biosca, E.G. 2005. Seasonal variation of Ralstonia solanacearum biovar 2 populations in a Spanish river: recovery of stressed cells at low temperatures. Appl. Environ. Microbiol. 2005. 71:140-8.
Fujiwara, A., Fujisawa, M., Hamasaki, R., Kawasaki, T., Fujie, M. & Yamada, T. 2011. Biocontrol of Ralstonia solanacearum by treatment with lytic bacteriophages. Appl. Environ. Microbiol. 77(12):4155-4162.
Hartman, G. L. & Elphinstone, J. G. 1994. Advances in the control of Pseudomonas solanacearum race 1 in major food crops. En: Bacterial wilt: the disease and its causative agent, Pseudomonas solanacearum. Hayward, A. C. & Hartman, G. L. eds., pp. 157-177. Wallingford: CAB International.
Jones J.B., Jackson, L.E., Balogh, B., Obradovic, A., Iriarte, F.B. & Momol, M.T. 2007. Bacteriophages for Plant Disease Control. Annu. Rev. Phytopathol. 45:245-62.
Kawasaki, T., Shimizu, M., Satsuma, H., Fujiwara, A., Fujie, M., Usami, S. & Yamada T. 2009. Genomic characterization of Ralstonia solanacearum phage ϕRSB1, a T7-like wide-host-range phage. J. Bacteriol. 191:422-427.
Lopez, M. M. & Biosca, E. G. 2005. Potato bacterial wilt management: new prospects for an old problem. En: Bacterial wilt disease and the Ralstonia solanacearum species complex, Allen, C., Prior, P. & Hayward, A. C. eds., pp. 205-224. APS Press St. Paul, MN.
Marco-Noales, E., Bertolini, E., Morente, C. & López MM. 2008. Integrated approach for detection of nonculturable cells of Ralstonia solanacearum in asymptomatic Perlargonium spp. cuttings. Phytopathology 98(8): 949-955.
McFeters, G.A. & LeChevallier, M.W. 2000. Chemical desinfection and injury of bacteria in water. En: Nonculturable microorganisms in the environment. Colwell R.R. & Grimes J.D: eds., pp 255-275. American Society for Microbiology Press, Washington, DC.
Montesinos, E., Badosa, E., Bonaterra, A., Peñalver, R. & López, M.M. 2008. Aplicación de la biotecnología al control biológico de bacterias y hongos fitopatógenos. En: Herramientas biotecnológicas en fitopatología. 2008. Pallás, V., Escobar, C., Rodriguez-Palenzuela, P. & Marcos J.M. eds., pp. 317-343. Ediciones Mundi-Prensa.
Oliver, JD., Dagher, M. & Linden K. 2005. Induction of Escherichia coli and Salmonella typhimurium into the viable but nonculturable state following chlorination of wastewater. J Water Health. 3(3):249-57.
Pereira, C., Silva, YJ., Santos, AL., Cunha, A., Gomex, N.C.M. & Almeida, A. 2011. Bacteriophages with potential for inactivation of fish pathogenic bacteria: survival, host specificity and effect on bacterial community structure. Mar. Drugs. 9:2236-2255; doi: 10.3390/md91112236.
Safni, I., Cleenwerck, I., De Vos, P., Fegan, M., Sly, L. & Kappler, U. 2014. Polyphasic taxonomic revision of the Ralstonia solanacearum species complex: proposal to emend the descriptions of Ralstonia solanacearum and Ralstonia syzygii and reclassify current R. 5 syzygii strains as Ralstonia syzygii subsp. syzygii subsp. nov., R. solanacearum phylotype IV strains as Ralstonia syzygii subsp. indonesiensis subsp. nov., banana blood disease bacterium strains as Ralstonia syzygii subsp. celebesensis subsp. nov. and R. solanacearum phylotype I and III strains as Ralstonia pseudosolanacearum sp. nov. Int. J. Syst. Evol. Microbiol. 64:3087-3103. 10
Santander R.D., Catalá-Senent J.F., Marco-Noales, E. & Biosca, E.G. 2012. In planta recovery of Erwinia amylovora viable but nonculturable cells. Trees. 26 (1):75-82. Yamada, T., Kawasaki, T., Nagata, S., Fujiwara, A., Usami S. & Fujie, M. 2007. New bacteriophages that infect the phytopathogen Ralstonia solanacearum. Microbiology. 153:2630-2639. 15
Yamada, T., Satoh, S., Ishikawa, H., Fujiwara, A., Kawasaki, T., Fujie, M. & Ogata, H. 2010. A jumbo phage infecting the phytopathogen Ralstonia solanacearum defines a new lineage of the Myoviridae family. Virology. 398(1):135-47.

## Claims

1. A bacteriophage able to lyse cells of *Ralstonia solanacearum* selected from the group of the following:
a) DSM 32039 (vRsoP-WF2), DSM 32040 (vRsoP-WM2) or DSM 32041 (vRsoP-WR2), or
b) a podovirus whose genome has the sequence of SEQ ID NO: 1 (corresponding to vRsoP-WF2), SEQ ID NO: 2 (corresponding to vRsoP-WM2) or SEQ ID NO: 3 (corresponding to vRsoP-WR2).

2. A composition comprising at least one of the bacteriophages of claim 1, or combinations thereof.

3. The composition of claim 2 comprising one of the following combinations of bacteriophages:
a) vRsoP-WF2 and vRsoP-WM2;
b) vRsoP-WF2 and vRsoP-WR2;
c) vRsoP-WM2 and vRsoP-WR2;
d) vRsoP-WF2, vRsoP-WM2 and vRsoP-WR2.

4. Composition according to claim 3, wherein each of the bacteriophages of the combination is present in the same concentration.

5. Composition according to any one of claims 2 to 4, wherein the total concentration of bacteriophages able to lyse *R. solanacearum* cells varies between 10² and 10⁹ plaque forming units per millilitre (PFU/ml).

6. Composition according to any one of claims 2 to 5,
comprising:
a) an agronomically acceptable carrier and/or excipient; and/or
b) a chemical agent for the control of *R. solanacearum* or a biocontrol agent of *R. solanacearum* different from a bacteriophage of claim 1;
and which optionally is in the form of a suspension in water or an aqueous solution.

7. Use of a bacteriophage of claim 1 or a composition of any one of claims 2 to 6 for the control of *R. solanacearum* in soil, by adding one or more bacteriophages of claim 1 or a composition of any one of claims 2 to 6 to the soil via irrigation water with which the soil is irrigated, which has been previously treated with one or more of the aforementioned bacteriophages or with the aforementioned composition.

8. Use of a bacteriophage of claim 1 or a composition of any one of claims 2 to 6 for controlling *R. solanacearum* in natural watercourses, channelled water streams, natural reservoirs of water, artificial reservoirs of water, irrigation water and irrigation water reservoirs, which will be used to irrigate crops.

9. Use according to claim 8, wherein the bacteriophage of claim 1 or the composition of any one of claims 2 to 6 is added to a natural reservoir of water or to an artificial reservoir of water, and wherein the water in the reservoir is maintained at a temperature between 4°C and 30°C, or the average water temperature in the reservoir is between 4°C and 24°C, both included.

10. A method for preventing or controlling wilting caused by *R. solanacearum* in a plant, comprising the steps of:
a) adding a composition of any one of claims 2 to 6 to the water to be used to irrigate the plant;
b) watering the plant with said water.

11. Method according to claim 10, wherein the water to which the composition of any one of claims 2 to 6 has been added previously to irrigation, is maintained at a temperature between 4°C and 30°C or an average temperature of between 4°C and 24°C, both included.

12. Method according to any one of claims 10 or 11, wherein the plant is a species belonging to the *Solanaceae* family and is susceptible to and/or tolerant to *R. solanacearum* or any other species susceptible to and/or tolerant to *R. solanacearum.*

13. Method according to claim 12, wherein the plant is selected from the group of potatoes (*Solanum tuberosum*), tomatoes (*Solanurn lycopersicum*), sweet peppers (*Capsicum annuum*) and aubergines (*Solanum melongena*).

14. Method according to any one of claims 10 to 13, further comprising:
a) a prior step to the application of the aforementioned method in which the irrigation water is subjected to one or more other strategies such as chemical, physical, and/or biological control for the same plant pathogen or others; and/or
b) an optional step in which copper compounds, antibiotics and/or soil fumigants are applied to the soil where the plant is growing.

15. Method according to any one of claims 10 to 14 or the use according to any one of claims 8 or 9, wherein the water pH is in the range of 6.5 to 9.0, both included.

## Patentansprüche

1. Bakteriophage, der in der Lage ist, Zellen von *Ralstonia solanacearum* zu lysieren, ausgewählt aus der Gruppe der folgenden:
a) DSM 32039 (vRsoP-WF2), DSM 32040 (vRsoP-WM2) oder DSM 32041 (vRsoP-WR2), oder
b) ein Podovirus, dessen Genom die Sequenz von SEQ ID NO: 1 (entsprechend vRsoP-WF2), SEQ ID NO: 2 (entsprechend vRsoP-WM2) oder SEQ ID NO: 3 (entsprechend vRsoP-WR2) aufweist.

2. Zusammensetzung umfassend mindestens einen der Bakteriophagen nach Anspruch 1 oder Kombinationen davon.

3. Zusammensetzung nach Anspruch 2 umfassend eine von den folgenden Kombinationen von Bakteriophagen:
a) vRsoP-WF2 und vRsoP-WM2;
b) vRsoP-WF2 und vRsoP-WR2;
c) vRsoP-WM2 und vRsoP-WR2;
d) vRsoP-WF2, vRsoP-WM2 und vRsoP-WR2.

4. Zusammensetzung nach Anspruch 3, wobei jeder der Bakteriophagen der Kombination in der gleichen Konzentration vorhanden ist.

5. Zusammensetzung nach einem der Ansprüche 2 bis 4, wobei die Gesamtkonzentration der Bakteriophagen, die in der Lage sind, Zellen von *R. solanacearum* zu lysieren, zwischen 10² und 10⁹ plaquebildenden Einheiten pro Milliliter (PFU/ml) variiert.

6. Zusammensetzung nach einem der Ansprüche 2 bis 5,
umfassend:
a) einen landwirtschaftlich annehmbaren Träger und/oder Hilfsstoff; und/oder
b) einen chemischen Wirkstoff zur Kontrolle von *R. solanacearum* oder einen biologischen Wirkstoff zur Kontrolle von *R. solanacearum,* der sich von einem Bakteriophagen nach Anspruch 1 unterscheidet;
und der gegebenenfalls in Form einer Suspension in Wasser oder einer wässrigen Lösung vorliegt.

7. Verwendung eines Bakteriophagen nach Anspruch 1 oder einer Zusammensetzung nach einem der Ansprüche 2 bis 6 zur Kontrolle von *R. solanacearum* im Boden durch Zugabe eines oder mehrerer Bakteriophagen nach Anspruch 1 oder einer Zusammensetzung nach einem der Ansprüche 2 bis 6 zu dem Boden über Bewässerungswasser, mit dem der Boden bewässert wird und das zuvor mit einem oder mehreren der vorgenannten Bakteriophagen oder mit der vorgenannten Zusammensetzung behandelt wurde.

8. Verwendung eines Bakteriophagen nach Anspruch 1 oder einer Zusammensetzung nach einem der Ansprüche 2 bis 6 zur Kontrolle von *R. solanacearum* in natürlichen Wasserläufen, kanalisierten Wasserströmen, natürlichen Wasserreservoirs, künstlichen Wasserreservoirs, Bewässerungswasser und Bewässerungswasserreservoirs, die zur Bewässerung von Kulturen verwendet werden.

9. Verwendung nach Anspruch 8, wobei der Bakteriophage nach Anspruch 1 oder die Zusammensetzung nach einem der Ansprüche 2 bis 6 einem natürlichen Wasserreservoir oder einem künstlichen Wasserreservoir zugegeben wird und wobei das Wasser in dem Reservoir bei einer Temperatur zwischen 4 °C und 30 °C gehalten wird oder die durchschnittliche Wassertemperatur in dem Reservoir zwischen 4 °C und 24 °C liegt, beides eingeschlossen.

10. Verfahren zur Prävention oder Kontrolle der durch *R. solanacearum* verursachten Verwelkung in einer Pflanze, umfassend die Schritte:
a) Hinzufügen einer Zusammensetzung nach einem der Ansprüche 2 bis 6 zu dem Wasser, das zur Bewässerung der Pflanze verwendet werden soll;
b) Bewässern der Pflanze mit diesem Wasser.

11. Verfahren nach Anspruch 10, wobei das Wasser, dem die Zusammensetzung nach einem der Ansprüche 2 bis 6 vor der Bewässerung zugegeben wurde, bei einer Temperatur zwischen 4 °C und 30 °C oder einer Durchschnittstemperatur zwischen 4 °C und 24 °C, beide eingeschlossen, gehalten wird.

12. Verfahren nach einem der Ansprüche 10 oder 11, wobei die Pflanze eine Spezies ist, die zur Familie der *Solanaceae* gehört und gegenüber *R. solanacearum* anfällig und/oder tolerant ist, oder jede andere Spezies, die gegenüber *R. solanacearum* anfällig und/oder tolerant ist.

13. Verfahren nach Anspruch 12, wobei die Pflanze aus der Gruppe der Kartoffeln (*Solatium tuberosum*), Tomaten (*Solatium lycopersicum*), Paprika (*Capsicum annuum*) und Auberginen (*Solatium melongena*) ausgewählt wird.

14. Verfahren nach einem der Ansprüche 10 bis 13, ferner umfassend:
a) einen vorhergehenden Schritt vor der Anwendung des oben genannten Verfahrens, bei dem das Bewässerungswasser einer oder mehreren anderen Strategien wie chemischer, physikalischer und/oder biologischer Kontrolle desselben oder anderer Pflanzenpathogene unterzogen wird; und/oder
b) einen optionalen Schritt, bei dem Kupferverbindungen, Antibiotika und/oder Bodenbegasungsmittel auf den Boden, auf dem die Pflanze wächst, aufgebracht werden.

15. Verfahren nach einem der Ansprüche 10 bis 14 oder Verwendung nach einem der Ansprüche 8 oder 9, wobei der pH-Wert des Wassers im Bereich von 6,5 bis 9,0 liegt, beide eingeschlossen.

## Revendications

1. Un bactériophage capable de lyser des cellules de *Ralstonia solanacearum* sélectionné dans le groupe suivant :
a) DSM 32039 (vRsoP-WF2), DSM 32040 (vRsoP-WM2) ou DSM 32041 (vRsoP-WR2), ou
b) un podovirus dont le génome a la séquence de SEQ ID NO: 1 (correspondant à vRsoP-WF2), SEQ ID NO: 2 (correspondant à vRsoP-WM2) ou SEQ ID NO: 3 (correspondant à vRsoP-WR2).

2. Une composition comprenant au moins l'un des bactériophages de la revendication 1, ou leurs combinaisons.

3. La composition de la revendication 2 comprenant l'une des combinaisons de bactériophages suivantes:
a) vRsoP-WF2 et vRsoP-WM2;
b) vRsoP-WF2 et vRsoP-WR2;
c) vRsoP-WM2 et vRsoP-WR2;
d) vRsoP-WF2, vRsoP-WM2 et vRsoP-WR2.

4. Composition selon la revendication 3, dans laquelle chacun des bactériophages de la combinaison est présent dans la même concentration.

5. Composition selon la revendication 3, dans laquelle chacun des bactériophages de la combinaison est présent dans la même concentration.

6. Composition selon l'une des revendications 2 à 5, comprenant :
a) un porteur et/ou excipient agronomiquement acceptable ; et/ou
b) un agent chimique pour la lutte contre *R. solanacearum* ou un agent de biolutte contre *R. solanacearum* autre qu'un bactériophage de la revendication 1;
et qui se présente éventuellement sous la forme d'une suspension dans de l'eau ou d'une solution aqueuse.

7. Utilisation d'un bactériophage de la revendication 1 ou d'une composition de l'une des revendications 2 à 6 pour la lutte contre *R. solanacearum* dans le sol, en ajoutant un ou plusieurs bactériophages de la revendication 1 ou une composition de l'une des revendications 2 à 6 au sol via l'eau d'irrigation avec laquelle le sol est irrigué, qui a été préalablement traitée avec un ou plusieurs des bactériophages mentionnés ci-dessus ou avec la composition susmentionnée.

8. Utilisation d'un bactériophage de la revendication 1 ou d'une composition de l'une des revendications 2 à 6 pour la lutte contre *R. solanacearum* dans les cours d'eau naturels, les flux d'eau canalisés, les réservoirs d'eau naturels, les réservoirs d'eau artificiels, l'eau d'irrigation et les réservoirs d'eau d'irrigation, qui seront utilisés pour irriguer les cultures.

9. Utilisation selon la revendication 8, dans laquelle le bactériophage de la revendication 1 ou la composition de l'une des revendications 2 à 6 est ajouté à un réservoir d'eau naturel ou à un réservoir d'eau artificiel, et dans laquelle l'eau dans le réservoir est maintenue à une température comprise entre 4 C et 30 C, ou la température de l'eau moyenne dans le réservoir est comprise entre 4 C et 24 C, ces deux températures incluses.

10. Procédé de prévention ou de lutte contre la flétrissure causée par *R. solanacearum* chez un végétal, comprenant les étapes consistant à :
a) ajouter une composition de l'une des revendications 2 à 6 à l'eau devant être utilisée pour irriguer le végétal ;
b) arroser le végétal avec ladite eau.

11. Procédé selon la revendication 10, dans lequel l'eau à laquelle la composition de l'une des revendications 2 à 6 a été ajoutée avant l'irrigation, est maintenue à une température comprise entre 4 C et 30 C ou à une température moyenne comprise entre 4 C et 24 C, ces deux températures incluses.

12. Procédé selon l'une des revendications 10 ou 11, dans lequel le végétal est une espèce appartenant à la famille des *Solanaceae* et est sensible à et/ou tolérant à *R. solanacearum* ou toute autre espèce sensible à et/ou tolérant à *R. solanacearum.*

13. Procédé selon la revendication 12, dans lequel le végétal est sélectionné dans le groupe des pommes de terre (*Solanum tuberosum*), tomates (*Solanum lycopersicum*), poivrons (*Capsicum annuum*) et aubergines (*Solanum melongena*).

14. Procédé selon l'une des revendications 10 à 13, comprenant en outre:
a) une étape préalable à l'application du procédé mentionné ci-dessus au cours de laquelle l'eau d'irrigation est soumise à une ou plusieurs autres stratégies telles qu'une lutte chimique, physique et/ou biologique pour le même agent pathogène des végétaux ou autres; et/ou
b) une étape optionnelle au cours de laquelle des composés de cuivre, des antibiotiques et/ou des fumigants de sol sont appliqués au sol dans lequel pousse le végétal.

15. Procédé selon l'une des revendications 10 à 14 ou l'utilisation selon l'une des revendications 8 ou 9, dans lesquels le pH de l'eau est compris dans la gamme allant de 6,5 à 9,0, ces deux pH inclus.


### SEQUENCE LISTING

<110> UNIVERSITAT DE VALENCIA (Estudi general)
   Instituto Valenciano de Investigaciones Agrarias (IVIA)
<120> Method for the prevention and/or the biological control of
   bacterial wilt caused by Ralstonia solanacearum, via the
   use of bacteriophages suitable for this purpose and
   compositions thereof.
<130> P-101377
<150> ES201530730
   <151> 2015-05-26
<160> 3
<170> BiSSAP 1.3
<210> 1
   <211> 40591
   <212> DNA
   <213> T7-like viruses
<220>
   <223> /host="Ralstonia solanacearum" /isolate="vRsoP-WF2" /isolation_source="Tormes River, Salamanca, Spain" /note="Genome of the isolate vRsoP-WF2"
<400> 1
<210> 2
   <211> 41117
   <212> DNA
   <213> T7-like viruses
<220>
   <223> /host="Ralstonia solanacearum" /aisolate="vRsoP-WM2" /isolation_source="Cayo river, Badajoz, España" /note="Genome of the virus vRsoP-WM2"
<220>
   <221> misc_feature
   <222> 8340..8805
   <223> /note="insertion with regard to vRsoP-WF2 and vRsoP-WR2"
<400> 2
   ccccatgttc actctactag tcctcctctc aggtgtcccc gtggtgttcc ttctgggtct      60
   cgttctgtat ggcctgttgg acaactgatg gtgtccctga agtgcccctt agggggaaaa     120
   cttccgacgc aaaaatttga aagccccact cgaaattcga cgcgggcaga ttccccccgt     180
   gccccctccg cggcccggcc ctcgtggccc ctgccgaccc acctccgggc accctccagg     240
   ctgtacgctc cgctgactcc tggcacatct tctggcacac tctgccgtaa ctccctgatt     300
   actaagggga tgcactagct tacgaagcta ctgcgaccca ataagcctca cgcatgagca     360
   ctcactggct cactcgtggg gctttttttt ctattctgtc cccatttccg cgcccccctg    420
   ttcggccatc agtttgcttt ggtttctcct agggtttccc ctaagtgtct ccttggcgtg     480
   catcgctacg attctcccaa cggcccactt gcggcccacc actggagaac atcatgcaac     540
   tgcaatactt ccgcgacttg gcaatcggca cagcgttcac tatcgctggc acgccctacg     600
   tgaagaaaag cgcacggact gcgtacaccg ctcccggcca ccctgggcat tgggaaggcc     660
   gctggttctg gtttggtcag actgaactgg taatggccta agggagcaca ccatgagcaa     720
   agtccgagca ctcgcctact tcttcgctgc aaccacgctc gcactcacct acgtgggcgc     780
   aagggcagca catgcggcca tctcaagcct cctcgtgatg cacctgcatt gatcccactc     840
   agaacaccct ccttggcggc aaagccgcta cagaagcctc cagatcaacg tctgggggct     900
   tttttgtttg ctcctggggc tgacctacct gcgtcccact gcgtggctcc tagggcttcc     960
   tatcgttcct tcggagcaac gctcctgata tcggaactat tgcagtgatt gaaaaataca    1020
   attgggcagt ctccgatgtt tcgtatgtaa ttcggtctca ccagggggac acgcccctga    1080
   agacaaaaaa gcgtgggacc ggggcggacg ccagcagtca gggacaaccc gagtcaatcc    1140
   aagagtaagc acattgcgag tccttccagt gtgctcatca ctggagagac atcatgcaat    1200
   cattcaccct gaacattggc cttatcccga gcaagaaatc ttcgcgtacc gctcgcatca    1260
   ctgcatcgga agttaaggcc gcacttcgtg gcgctggctt cttcgtgtcg ggctttcgca    1320
   tggcccagtc ggccaccgag cctaccgcag tggtccgcgt gatcgcacgt cagccaatga    1380
   gctatcacca agcgctctac aacgtgtccc tggcgctggt gcaggactgc atcgcggttg 1440
   tccctgacac ggtacggggc gcgttgattg gcccggatgc ggctgagtgg ggtgagttca 1500
   atccggccta cttcatcccg tttgatgtcg aaccgcaggc aatcgctgcg tgacacttag 1560
   ggtgcccctt caggggctcc aggagtagcc gcattgcgct gtgcagtgcg cctatcactg 1620
   gaggacaaca tgtacggaaa ctttgacccg agcacgaacg catggccgtt cagtgtggag 1680
   tttgtggacg ctgtaggctg gcaagtggag gacaaccggg accccaccaa tgtcgtggtg 1740
   atggtcgctg gtctcacctt cgaggaagcc aaacagcgcg cgtctgaact caacctgaac 1800
   cacttccggg ggtcctgaca tgccgactct caaggaagcg agcgtgaatg ctcagagacc 1860
   acgcggaggc gtccaagcgt ggagcgtagg ggacacctac ccggtcactg tagtgggcct 1920
   gggcaatggc ccccgcgtgc aatggtacgc ggagaacctg cacacgggcg aacgtggccc 1980
   cgtgcgagat gcccagggtg atgcagtggt ggaccagtat cgtctttggg cggagttcaa 2040
   cagaaatcgc ctacaggcgt aattcggtgg ccctgttcat gtgtcgtgaa cagggctcca 2100
   ggagtgaacg cattcaatcg tgagtgcggt catcactgga gaatgcaaca tgcaaacgaa 2160
   agaacagcgc atcgaactaa tcgccgcgat gtttggtgag caagaaacgg gcctgatcgg 2220
   taagcaactc cgcgtgctgg ataactccca aagcggggcg ttctacaatg ttggtgatgt 2280
   cggtaccgta gtcctcgtgg acgatgacgg tgaaatctgg gtggactttg gcccggatgg 2340
   cttcaaaggc gatggtacgg catacccggt ctgggccgct ggttcgctgg gcgcagacga 2400
   ccatgagttt ctggaaaact gacatgggcg tcatctggca cgaactcatc tacgccctgg 2460
   gagccctcgt ggttgtcggg gtcctcattc tgatcctcac cgagggagac tgacatcatg 2520
   cgcacctttg caatcgactt catgctcaac ggcaagcgcg ttgggcgtga ctacgtgacg 2580
   gcttccaacg agaagcaagc caccatcatc gcagaacgca ctgcacccgt gacgctgtat 2640
   gacgaggttg tggtcgcacc gctgtgatgg accatcgggc tcccttgtgg agcccccagg 2700
   agtggaccct ttcaattccg agagtgtcca tcactggaga gaatcatgtc ggacaaagcc 2760
   aagcaatcca tcgagttcgt tcgcaacggc ctgggcgagg aaaacttcaa caagctcctg 2820
   agcatcacgg gagtacgtga catcgaactg gctgccgcgt tcctggcgac caccaaggag 2880
   gagcgtgact ctgtgaagac aggtgacgac ctcatgcgcc tgctgggccg caagcacgct 2940
   gagaaccgcg tggccatggc tctggtgcgc gcgggtgtgc cggtggagga tgccgtgtct 3000
   ttcgtgcgtg aaaccgctgc aagcctgtaa gccccaaggt gccccttagg gggcctctag 3060
   gagtgagccg ctggaatcgt ccgaagtctc atcactggag atcgctatgt ctgcacaagc 3120
   cgaacaaacc caaaccgccc cgaccatcat cgccctgctg tctgctgcga atatggctca 3180
   gacgggcccc ggcgtcttcg ctggcgtcat caaccaagcc acacctgagg agcgcgcggg 3240
   tgtgaagaac atgaaggacc tcctggcgct gtacttcaag gttcatgcgc gagtggtggc 3300
   cgaaatctcc gcggaagtgg aagccaccac ggaccatcgg gctcctctgg tggacctgtc 3360
   cgacttcgct gagaccctgg cggagtactt cagccgtgcc gatgaagtgg tgccggaagg 3420
   cgtcacgctg caataacgct gggtgccccg aaaggggctc caggagtgga tgtcttcatt 3480
   gtgaggacct ccatcactgg agaaagcaat ggcacagatg cgcgcctggg tctacaaggc 3540
   gcactggagg cggcacctgg ccgcgcaagg catcgtcctg cgcaaatacg aggtggacaa 3600
   ggagtacatg caccgcggca tgacgcaggc catcttccgc cgcaacaaag cgaagttggt 3660
   ggccgagtac acggagttct gacatggaca tcgtagacga actggagata ggaccctctt 3720
   acgtcctgaa ctcggacgag aagtggctcc gcaagagagc cgctgaggaa atccgcaggc 3780
   tccgaaagca actggcggac gctggttggg ctctcgaagc ggcccgtgaa ctcgaagacc 3840
   aacgagacaa cgggggctgg ctatgaaacc cgctgacggt caacccaagc gcttcaagct 3900
   gcacaccaag tatccccaca acaggtccga gggtttgact catcggacca acaaggggac 3960
   cgcgcttcaa gttctaccga agaggtaacg ccatgaagat cactctgaca ctggaggaca 4020
   ccgctgatgg tgtcgctgtg aactggaccg aggagcaatc tgaagctcag aacaaaccca 4080
   gcgagagcct ggccaccatc atcgctgcca agttcattct tgagataaat caatctcacc 4140
   gtatgggaat tttacggctg tccggcactg cattgggcgc agatcgcgca tagctagtat 4200
   gaggtgtgtt gcgtagagtg cgaaccagtt ttatttggtt cgccatgccc gcatccagaa 4260
   gctcatcgca acagtagagg agtagcaatg ccggtcatca aacgcgggaa caagtaccag 4320
   gccagtgtgg gctctggtac tgatcgctgg cgcaagatgt tcgacaccca ggaggaggcg 4380
   gagaccgcag aactggcaga gaagctgcgc aggaaggccg ctgggaagga cgagaagggg 4440
   gctacaagct ccgcaaatgg ggcgaaggta cagaagaccc taaaggaggc ttacgaccgc 4500
   accttggccc tgatttggaa gggcaccgct gcggagaaga cccacatcat caactcgaac 4560
   tccgtgatgg cggagttggg caaggacacg ctcctgtccg acatcgccac cgaggacgta 4620
   acggagatga tcctggctct ggaggagaag ggcaactcag gcagcacggt gaacaagaag 4680
   ctgtcctgcc tgtccatgat cctcaagacc gcctcggatg agtggcctgg gtgcatcgtg 4740
   gagatgccca agctgaagcg gcgcaaggag gggtctcacc ggctccggtg gatcaacgag 4800
   gccgaggaga agcggatgct ggaggccgcg gagcacctgg ggctctacga cctccgggac 4860
   tacatcatcg ttggcatcga caccgggttc cgccgcggag aactcctcgg gttccccctg 4920
   aaggactacc agggcggtct catgatcctc cacgatggtg agaccaagag cggcaagggg 4980
   cgcgccatcc cggtcaccaa gcgggtccac gagatcatcc agcggaggag caactactcg 5040
   tacctcttcc aggactacac ggtccacaag ctgcgttggc agttcgacca actgaagctc 5100
   cacatggggc tccaggagga cacgcagttc gtggtccaca ccctgcggca cacctgtgcc 5160
   agccggatgg ttcaacgtgg ggtgcccctg aaggtggtcc aggagtggat gggtcacgcc 5220
   accatcgcca cgaccatgcg ctacgcgaag ctagctccga gcagcctgct gatggcgaag 5280
   aaggccctgg aggaagaacc ccaggaactc acattcattc ctcccccgca gatggatgtg 5340
   gtggagcttc acgacttcta aggaaaggaa ttggaacacc tcagagagac gttcaaggga 5400
   aaggtacaga gacaggcagg acgagatggg tgctgggttt ggcaaggctc taagacggac 5460
   aggggatatg ggaacctgtg ggacccaaaa accaagaagc ctgtctcagc acatcgactg 5520
   tcctaccaac tccacaaggg acaaatcccg gaggggttga tggttctcca ccggtgcgat 5580
   aacagggctt gtgtgaaccc aaagcacctg tttgtgggga ccgcccagga caatacggtt 5640
   gacatgtacc tgaagggtag aggaacagtt ccacattagg ttccacataa ggataaccct 5700
   gaagggaaac ctaatgtgta aatcctaagt gtttatcttc atagatagac actattaatg 5760
   atatctactt agagagaaca ctttagttga cactatgact acccaacaag tggacaacga 5820
   gaacgaagac ctggtgacta ttcagcttcg tctcgaagaa gagatgaccc agcggggagc 5880
   agaccggtac atccgggggg tatccaaggc catcgagaag ggccgtgagg atgacaccgc 5940
   ctacggcaag caaatcctgg ccgggaggtt ggcgaagctg gcccaggcca tcgctgagtg 6000
   gaaggcggag gtggcctctg gtaagcctgg ccggaagcac tcggcctgga agctcatcaa 6060
   ggacacggac gacaacatcc tcgccttcct ggccctcaag cacgttctct cgggggtctc 6120
   cgcagtccgc accgtccagt acgtggccgt ggccatcggc accgcggtgg aggacgagat 6180
   gcggttcgcc aaggtccgtg aggcggagcg gaagaagttt gagcagctag tcaccggggc 6240
   agcgaagcgg accagccagc actacaagca cgtctacgcc acccgcgtgg ctgaggacgt 6300
   gacggagtgg gacaagtggt cccggactga ccgcctccac gtgggggtca agctcctgga 6360
   cctcctgatg cagtccatcg gcctggtgga ggtgtccacg aacctggaca acagcgagca 6420
   ggggctcaag tacgtgaagg ccctcccgga gaccctggag tggatcgaac ggaagaacga 6480
   ggtgaccgcc ctgctgcgcc cggtctatga gccgatggtg gttcagccgc gggattggac 6540
   caacccgttc gatggcggct acctgtcctc gaacatcaag ccgctgaagc tggtgaagac 6600
   gaagaacaag gcgtacctgg aggaactccg cggcgctgac atgcccatcg tctacgaggc 6660
   agtgaacgcc atccagcgca cggcctggca gatcaactcc caggttctca cggtgatgcg 6720
   gcacctgtgg gactcaggct ccgagcttgg tggtcttccc cctcgggagg gactgccgat 6780
   gccaccgaag ccctacgaca tcgacaccaa cgatgactcg aagaaggcgt accgcatcgc 6840
   cgcagcgaag gtccacatgg agaacctctc cattctgggc cagcgcatcg gctttgacat 6900
   ggccctgggc attgcgggcc gctacgagaa gtaccggcgc atctacttcc cgtaccagtt 6960
   ggacttccgg gggcgcatct acgcggtccc gcacctgaac ccgcaggggt ccgactacca 7020
   gaaggctctc ctcagattcg ccaacgggaa accgctgggc tccgaggggt ggaagtggtt 7080
   ggccatccac ggtgcgaacc tggcgggcta tgacaaggtg agtttggagg accgcgtgga 7140
   gtgggtcctg gagaacgaag atgagattct cagaatcgca agtgatccct acgaccatcg 7200
   tggttgggca tcggaagtgg ggggggttaa gatcgacaag ccctggcagt ttcttgcctt 7260
   ctgctttgag tgggctgggt tcgttgagca tggtgagtcg ttcgtatcaa agctgcccgt 7320
   ggctatggac ggttcatgct ctggcatcca gcacttcagc gcgatgctcc gggacgaacg 7380
   aggcggggcc gcagtcaacc tcgtacccca ggacctccca gccgatgtct atagagccgt 7440
   cgctgagaga gtcattgaac aggctgaaag tgatctcgct cacggttccg aggacgaact 7500
   gaagcacaac ggccagggca tcgcttacct gtctgagggc tccaagacca tcgcccagca 7560
   gtggatcaag ttcggcatca cccgcaaggt caccaagcgg agcgtgatga cgctggccta 7620
   cggctccaag gagtacggct tcaaggagca actcatggag gacatcctgt ggccagcgaa 7680
   gagggcagcg atgcggcctg atgggtccat cgacacggag aagttcccgt tcagcgggga 7740
   tggctaccgt gcggctctct ggatggcgaa ggcaatctgg aacgcggtga acgcagtgct 7800
   ggtgaaagct ggcgaggcga tgcactggct ccaggaggtg gcagcactgg ccgcgaagga 7860
   ggaactgcct gtccgctgga caaccccggt ggggttcccg gtgatgcagg cgtatccggc 7920
   cctggaggca cgtagggtga agaccgccat caacggcatg gtgctgaagc tcctcatgaa 7980
   ccaggagaag gactccctgg acaagcggaa gcaggggcag ggcatctcgc ccaacttcgt 8040
   ccactcctgc gatgcggcgc acctgatgct cacggtggtc cgcgcgaagc aggaaggtat 8100
   ccagaacttc gccatgatcc acgactcctt cgggaccacc gcgggtgacg tggaggagat 8160
   gtatcgggtg gtccgcgaga gcttcgtgga gatgtactcc gaggtgcgcg tcctggaaga 8220
   cttccgggat gagatcgcgg agcaactttc cgagaaggcc agggccaaga tgcctgatct 8280
   accggcccgc ggcctcctgg agttgtctcg tgtgtgcgag agccggtact gttttgcgta 8340
   gactgtttca catttgcaac tattccttat gagtgagtgt aagaagtgcg gggttgcctt 8400
   ggtgccaggt gagaactggt atccgtccct cgcaaagaag aacaaccagg tgtgtaagcg 8460
   gtgtcacacg gcacggagcg aggcgaagcg gattgaagac cgcgagacca acctcccgaa 8520
   gtggatgctg cgaaacgcca ggaatcgggc caaggcacag ggactcccat tcgacctgga 8580
   ggagtcggac atccaaattc cgctcctctg tcccgtgctg ggcatcccgc tggaagtctc 8640
   acgcgggcac ttcacggaca actccccggc tctggacaag ttcatcccgg agcttgggta 8700
   cgtgaagggc aacgtggccg tcatttctca gaaggccaac gtgatgaagt ccaacgccac 8760
   cattcaggag gtggaggcac tggccgcgtg gatgcgtagt cgcgcctgaa cccctccaca 8820
   tctggaagag ttgagccggg ggaacgatta ggtgccacac atggataaac cagccgccgt 8880
   tcccccggtg gcctctcccg agacaaccga tggaacgcaa cgaacacgaa gtatcggacc 8940
   agtacgagtc cgcacttggc cgcgcgattg ctcagtggcg caccggacgg cccatcccga 9000
   tgacactcgc cgctgaactg atgcaacagg gctatgacgt atccgccctg gaagcacgtc 9060
   acatgacctg aaccaacaat ggcagaaaag aaacaacgca acccgagctt cacctcgccg 9120
   cgcggcatcg cccgctaccc ggccctcaac aagcccgact acggcaacga acagttcccg 9180
   aagccggatg gtgagtacaa ggtccaactc atcctgagcg aggccgaggc ccagccgctc 9240
   atcgagaagc tccagccgct ctatgacgcg gccatcgagg aaggcaaggc gaagttcaag 9300
   gaactgaagg tggagcagcg caagaagctg ggcgcgctga aggagaacga cctctacgcc 9360
   accgagtacg accaggagac cgaggagccg accggcaacc tcatcttcaa gttcacgatg 9420
   caggccggcg gcaagaacaa gaagggtgag ccgtggtctc gcaagcccgc gctgttcgac 9480
   gcgaagggca agccgctgcc gaagaatgca ccggccatct ggggcggttc ggaagtcaag 9540
   gtctcgttcg aggccgctcc gtacttcatc cccggcacgg gtgctgctgg tctgaagctg 9600
   cgtctccagg cagcgcaggt gctcgaactg gtgactggtg gccagcgcag tgccgatgcc 9660
   tacggcttcg gtgccgaaga cggctacgag gcagacgaca acaatgaaga gggcgatgaa 9720
   gccccggaca ctgatggcaa gagcggcagc ggcgaagacg agttctaaat cactgactgc 9780
   caaacaggtg gccctgaagt acggcttcag gagcggcctg gaagagaaga tcgccgcgga 9840
   cctcacctcg aaagggatgg ggttcacgta tgaggagcta accatccctt acgtgaagcc 9900
   cgcgaagccc tcaaagtaca caccggactt cgaccttctc aagaacggca tcatcgtgga 9960
   gtccaaggga cggttcctaa cagaggaccg ggccaagcac ctgctggtga aagcccagca 10020
   cccagacctg gacattcgtt tcgttttctc gaattcaaag gcaaagatca acaagcgaag 10080
   cccgaccacc tatgcgatgt ggtgcgagaa aaacggcttc gcatatgcgg acaagagcgt 10140
   gcccgaggca tggctcaaag agccgccgaa cctgaagtcc ctaacagcca tcgagaggct 10200
   gcggggagca tgacatggca tacacttcca acaccaagaa gcgggcaagc acggactacc 10260
   tggtggtcca ttgctccgca acgaagccct ccgctgacat cggagccgcg gacatcgacc 10320
   gctggcaccg gaagcagggg tggcgctgca tcggctacca cttcgtcatc cgccgtgatg 10380
   gcaccatcga agaaggccgt tacgctgacg ttatcggcgc acacgtagaa ggccacaacg 10440
   agaactccct gggcatctgc ctggcgggtg gtgtctccga gaaggatgtg aacgttgccg 10500
   agaacaactt cacgcccgag cagttcgcca gcttacagaa gctcctgacg gacctccgag 10560
   cgaagtatcc caaggccacc atccagggtc accgcgattt ccctggtgtg gcgaagtcgt 10620
   gcccctcctt cagtgcgaag gattgggcca agcaaaacgg tttctgacac accacgagga 10680
   gcaaccatga aggcatggcg taaagaaccc aatcagggcg cagtccgtat tggtcgcaag 10740
   accatcaacg cgaagcgtgt gatgaacaag ttcaaaccga gcatggtcaa ccatggctcc 10800
   gtcctgtttc agcggatgat gctccaggcc ggtatctggg cgctctaacc taaaccatct 10860
   ccagtggtac ttcgggccgg tccttcgggc tggcccccct tttatgctca agatttgtaa 10920
   gaggtgcggt gaatgcaagc cgtttagcga ctttcacaaa gcacccgcag gaaaattcaa 10980
   gctccagtca tattgcaagc agtgcaagaa ggaatacacg cgggacactg gagctaacat 11040
   cctaccctcc attcgtcaga gagcacgaaa gcagggagtc cccttctcgc ttaccaaaga 11100
   gaacctccca cccatccccg aagtgtgccc ggtcttaggg gttccccttc gacggacact 11160
   cggctttgcg gacgacaact cgccatcgct ggatcgattg atccctgagc ttgggtacgt 11220
   gcctgggaat gttgagtgga tgagctaccg agctaatcga atcaagaacg actcaaccta 11280
   tgaagaactc gaaagggtca ctgcctgggt ccgagagcga gtttctacga cacatcccat 11340
   gtgagggctg cggttcctca gacgggaaca gtctcttcag tgatgggcac cagtggtgct 11400
   tcgtctgtga aacctacgtg cccggtgatg gcagcgaacc aacaatagga acaacgaaga 11460
   agcggatgga agggctgcta accggggagt ttcgccccct actgaaacgg aagatcaccg 11520
   aggagacggc gcgcaagttc tcgtatcaag tcggtgagtt caagggaaag acggtgcaac 11580
   tcgcgccgta ctttgacaat gcaggtgtga tggtggctca gaaggtccga ttcccggaca 11640
   aggagttcac cgtagttggg gatggcaagg ccatctctgg aatcctcttt ggccagaacc 11700
   tatgggctcc tggcggaaag aagatcgtgg tcaccgaagg cgagatcgat gccatgtcgg 11760
   tgagccaagc gcagggcaac aaatggcctg tggtctccgt accaaacgga gcacaaggcg 11820
   cgaagaagtc gcttcagaag gcactcgaat acctggagag ctttgatgaa gtgattttga 11880
   tgttcgattc cgatgatgca ggcaagaagg ccgctgctga gtgcgcggag ttgttctcgc 11940
   ccggcaagtg caagatcgcg tccatcccga tgaaggacgc caacgaattg ctgaaggctg 12000
   gccgtgagca ggagatcatc actgcaatct ggcaggccaa ggagtaccgc cccgatggca 12060
   tcatctcggg agcggaactg tgggaggcgg tgtcagcatc tcaggatatc gtagagtccg 12120
   ttccgtaccc ctgggacgca ctgaatgaag tcacgaaagg cgcgcgtaca ggcgagcttg 12180
   tgactctcac tgcgggttcc ggcatcggca aatctgccgt ggtacgcgag atcgctcacc 12240
   acctcctgag gcgtggagag acggttggca tgttgatgct cgaagagaac ccgaagcgca 12300
   ccgcgctggg tctcattagc atctccctca acaggcctct ccacatagac cgtgaaggtg 12360
   tcagcaagga tcaactgaag gtagctttcg atgatacggt aggctctggc cgactattcc 12420
   tctacgacca cttcggctcc agcgacatcg acaacctggt gtcccgtgtc cgcttcatgg 12480
   cgaagggcct ggggtgcaag tgggtcatcc tcgaccacct gagcattgtt gtctctggcc 12540
   tcggtgacgg agacgaacgg cgactcatcg acaacgcaat gacgatgctg cgtaccctcg 12600
   tggaggagac cggcatcggc atgtttgtgg tgtcacacct ccgccgaccg gagggtgacc 12660
   gcggccacga acagggagca cgtacctcgc tcacccaact ccgcggttcc catagcatcg 12720
   cgcaactgtc ggacatggtg attggtctcg aacggaacca gcagggtgag aacccgaacg 12780
   tcaccacgct ccgtgtgctg aagaaccgct tctccggtga gaccggtgag gccgggttcc 12840
   tgctgtacga ccgggagacc ggacgcctgg aagagacgga cgcacctgct gcgcccttca 12900
   aagacgaaac caaatcggac gttcagtccg agttctaacc aaaggttaca tcatgagtct 12960
   gatttcgctg ttcacgcagt ccgctgctga ccaacgtgct gccgcgcccc gtgctgcccg 13020
   tgtccgcgcc aagatcgcgg acctgatcga ctaagcggga gtctctgtga tcgatgacac 13080
   ccgcctccaa gagttccgag aaatcctcga tgtagtccgc tgggagttcc ccggttcaca 13140
   ccccgtgatt gggggcgggg ctctccgcga ttcctaccat ggtcgcccaa tcaaggacgt 13200
   ggacgtgttc atgcgcaggc gtgaccacga gacgctgaac tcggaactca cccgcttcat 13260
   ccgcccgccg atcctcgtgg cccacggcta tggccgtccc gacatgcacg gcgcatggga 13320
   cctgatgcag tccgttgctg gctacgaggt gcaactcatc ctcgcggact tcgagaacct 13380
   ggaagacctg gccggtacgt tcgacctggg gattgcccga gccaccttcg atggtgaccg 13440
   gctgttcctc catccggact tcctccagga ctccacggat aaggtcttcc gcatccgtcg 13500
   cgcggacaac ctgttcgaga aggcgcgaag cctgaagcgc atcaagcggc tggcagagaa 13560
   gtacccggac ttttcaacac cggacttcga gcattgccct gtctgcgcac aacccatcat 13620
   cgagttccgc aatgctgcca gcgtccgaga gcaccaaatc tccgggctct gccagcaatg 13680
   ccagtactcg gtgttcgaca aggactgacc atgaacacct tcctcattct cctggtcctc 13740
   atcggaggcc aaatcgaagg ccgcgtgatc gctgagttcg acactccccg tgagtgcgaa 13800
   gcagcgaagg aacacgtgag ggtcatcaac caaccccctg tcgtcgcgtc cacgttggtg 13860
   tgcgcaaggg atggccgcgc gtaatcacca aggacggtat gaagctattc gacattgaaa 13920
   caaacggtct gctggatacc gtcaccaagg ttcactgtct cgtcatcaag gatcgcacca 13980
   ccgggaggaa gttccgctgc atccccgcag gcttcccgat gcaagcggac atgaccatcg 14040
   agcaagggct ggagcttctc aagtccggcc ccatcggtgg ccacggaatc ctcaggtacg 14100
   acatcccggt cctggagaag ctgtacccgg acttcaccta cgacaaggac caggtgttcg 14160
   acaccctggt ggccgcgcgt ctcatctgga cgcacatcaa ggacatcgac aacgggctcc 14220
   tcaaaaagaa gcaaatcccc ggctccctct acggctccca ctcgctggaa gcctggggtt 14280
   accgcctgaa gctccagaag ggcgagtacg cggctgagtt caaggcgcgc atgggggacg 14340
   cttacgaggg gggcatggag tggcgagagc tttctcctga gatgctcgac tactgcgacc 14400
   tggacgtgga tgtcacggac gcactgttcg accggatcga aggcaagaac tactccgcgg 14460
   aggcgctgga gcttgagcac cgcatcgcct ggctgatggc tcaacaggaa cgcaatgggt 14520
   tcccgtttga cgtgacgaag gccagcgcgt tgtacgccaa gctcgcgcaa cgccggggcg 14580
   aactggagcg agaactgaaa gagttcttcc gtttctggtt cgctccggct ggaacagtga 14640
   ctccgaaggt tggaaacaag gcgcgaggaa ctgtagccgg tgtcccgtac accaaggtga 14700
   agatcgtgga gttcaacccc ggctcccgcg accacatcgc taatcgcctt gtcacgctct 14760
   acggctggaa accggaggtg ttcaccgatg gcggtaagcc tcgggttgat gaagatgtga 14820
   tggcacgcct ggactacccg cccacgaaac tcctcacgga atacctgctg gtctccaaga 14880
   gaatctctca gctagctgaa ggtgaccaag cgtggctcaa ggttgtacgt gacggaaaga 14940
   ttcatggctc cgtgaatccg aatggcgcgg ttacaggaag atgcacgcac gctttcccga 15000
   acgtggccca ggtgccagcc gtaggttccc cctatggtga ggagtgccgg ggattgttcg 15060
   gggcacctaa gggttggctg ctggttggct ccgatgcttc cgggttggag cttcgctgtc 15120
   tagcccactt catggccagg cacgatggcg gcaagtatgg aaaggtgatc cttgagggag 15180
   acatccacac ggagaatcag aaggccgctg gactgcccac acgaaacaac gcgaagacct 15240
   tcatctacgc gttcctctac ggagccgggg acgccaagat tggtaagatc gttggtaagg 15300
   acgctgctga aggaaagaag ctcaaggccg cgttcctgaa gaagaccccc gcactcaaga 15360
   agctcctcga agctgtccgt gagtctgcca agcgcggcta cctggttggc ctcgacaagc 15420
   gacaactcca tgtccgctct cagcacgccg cattgaacac cctgctgcaa tccgcaggtg 15480
   ccctcatctg caagtattgg gttgtccgca cggcagagcg aatggaagct ctgggctaca 15540
   agcacggatg ggatggggac ttcgcgttcg tcgcctatat ccacgatgag cagcaggttg 15600
   cagtacgaaa tgaggaagtc gccaaggtcc tcgttgagca ggttgcattg gccatgaagg 15660
   acgccgaagc gtgggccgga ttccggtgcc cgctggcctg tgagtccaag gtcggtacgg 15720
   attgggcttc aacacactaa agtaatcaga caccaacatg agcatgttcc gagacgacct 15780
   actcaaagaa gtcctctacg aggcgttcaa gactcccttc aagctccagt ccgacttcgc 15840
   ccgagagttc gctcaggaag tcgccgctct ggcctcgatg ggatacatct cgacctacga 15900
   ggggccgcag cagttcggca agaagtggcg cgtcaccggc atcggcctgg acaagctgcg 15960
   caagctgggg atgctgtgag tgaagcccta cgcccccatt cgctgaggat catgggccgg 16020
   aagttccggg tctcttacaa ggatgacctg gacggtgacc tgggatactg cgaacccacc 16080
   aagtgtaaga tcgagattga gaacgggcag caccccgtgg aggaggccga tacggtcctc 16140
   catgaggtgc ttcacgcggt gttctatctg atggacattg ggctctccgc ggaggaggag 16200
   gagcacgtgg tccgtaaggt tgtcaccgga ctcacccagg tattccagga caacccccgg 16260
   ctcctgacct acttggcaaa cgccaagtga tggaccatat agccaagttt gattctctcc 16320
   aggaggaact catgacggac aagaagtgga ccatcacggt taacgtggac acccccgagg 16380
   gccaccggga gcggaccatc gagttccccc accggcccac cgaggaggag cttggtctca 16440
   agctggcgca gttcttcagc cggatgaact tccgattcaa cgaacacctg aaggaggtga 16500
   aggggtgtgc gctcctgaca cctcggagac cgtatgaaag tagcgctgat tgatgctgac 16560
   gttctggtct tccaggcggc tgtagtcgct gagaaggcaa ccgattgggg ggacggtgtt 16620
   tggaccctcc acgcagacga gggtgacgga gaacgaatcg ttcgccagtc cgtcatcacc 16680
   ctccaggaga agaccggtgc ggataaggtc atcctggcat tctccgatga ggagaactgg 16740
   cgcaaggcca tactgcccac ctacaaggcc aaccgagcgg gttcccgcca gccgatcatc 16800
   cgcgcgcatc tgaagcggtg ggcttccgac gaatacgaga gcttcacccg gccaaccctc 16860
   gaaggggatg acgtgctggg catcctggcc acccgcgagg gcaagccagg cgagaacttc 16920
   atcgtgtgct ccatcgacaa ggacatgcga accatccctg gcacccactt caacttcggc 16980
   aagaacgaag agttcgtggt gacggaggag ggggcagact actggcatct cttccagacc 17040
   ctcacgggtg acccggtgga tggctacgca ggctgtcccg gcattggccc ggtggccgcg 17100
   aagaagattc tcgacaagag ccccacctgg ggtgccgtgg tctctgccta cgacaaggca 17160
   ggcttcggtg aagaggaagc tctcgtgcag gcccgagtgg cgcgcatctg ccgcgctgaa 17220
   gactacgact tcaagaagaa acaagttcga ctgtggaccc caaagaaatc ctgaaagaac 17280
   tggaacagca gcaacgccgc aagttcgaga aaggccctct caccggcaaa cgcgccgatg 17340
   tcatcatcat ggacgacatc caggacacca aggacaccaa cccgaaggac gccatcggct 17400
   ccaccaagct ccccctcgac ctcgttcctg actcgctctc ggtcttcgcc gcgctggcgt 17460
   tcaccgaggg tgccaccaag tacggtgcct acaactggcg tgtcgctggt gtccgtgcgt 17520
   ccatctacaa ggccgcgctg gagcgtcacc tgaagaagtg gtggaacggt gagtgggccg 17580
   acccgaagac gaaggtgccg cacctggcca gcgtcatcgc gtgtgctgcg atcatcctgg 17640
   acgcggacct cgcaggcaag ttgacggatg accgccctcc ggcaatcgac ctgagttcct 17700
   tcatcgactc ccttgaggag accgtgaagc acctcaagga actgcacaag gacaagaacc 17760
   cgaagcacta caccgaactc aacgtatgaa cccgaagcga aacactctga ccggctgggt 17820
   catctatgat gcagagcggg cgactggccg aagcaccgcg attgcgctga gtcttctagg 17880
   caaggccatt gcaaatccag gtgtggccgt acaaatccga gaacatcacg gtactcgtcc 17940
   ggctgacgag agtctgatgc gcctgatgcg ggatatggtc tttcggctgg gcctcaaggg 18000
   catgacgttc agccagaacc tgactgtgac gttcaacctt tgggagcctg tgtgagccag 18060
   agccgaaagg gctctctcat tgaggccctc atcaacaccg caatcggctt cgggatcaac 18120
   ttcacggcga acctcatcat cctcccactg ttcggcttca ccagtttgac ggtgcagacg 18180
   aacctggtga ttggcgtggt ctacacgctc atctccgtgg tgcggagtta cgtggttcgc 18240
   cgctggttca acgcacacat cgtccgagcc gccaagaaac tctcaggggc ctgaaggtct 18300
   ctttaggttc cacaatagga gaatcaaatt ggcgaacgac aagtttccgc cgattcccaa 18360
   agaattactt gaggcgcttg agaagcggtt cccggagaca ccactcgaaa atatcgggtc 18420
   tgtggatcaa cttcgattgg ctcagggtga gctacgtgtt gtccggtttc tccgagccca 18480
   attcgagaag cagaccaaga acattttgga gaacacatag tgtgcatgtc tcaaccgtcc 18540
   gccccacctc cggccccacc gccaccgcca cctccgcccc cgcccgtcga tccgattccg 18600
   gtccaacctg cgcagcaaac cggtggagcg gtgaccagcg gcaagagcaa gggacgcgac 18660
   tccctccgta tcgacctggc ccagaagaca tcgggtggtg gcgccggtct gaacatcccg 18720
   atgtaacgaa gggcagggat ggaacaagaa aagaaaacct gcgcctccct ctaccagaaa 18780
   ctcaccaccg accgagaccc gttcctgaag cgggcctacg actgcgccga actgacgatt 18840
   ccctccttgc ttcctcgtga gggacacaac ggctccacca aactcgtcac tccgtggcag 18900
   ggcattggtg ctcgtggggt gaacaacctc gcatccaaac tcctgctgac gcagcttcct 18960
   cccggaactc ctccgttcaa gttgtcgatt gacgacttca cgctggagga actgacgaag 19020
   caggaaggga tgcgggcgaa ggtagaggag gggctcaaca agatcgaacg cgcggttcag 19080
   actgagatcg aagcgaacta catccgcgtg gctgccttcg aggcgctgaa gcatctcatc 19140
   gttagtggca atgccctgct gtacattccg cctgaaggtg gactgagagt attccacctg 19200
   gaccgctacg ttgtccgccg tgacccgatg ggcaacgtgc tggacatcat caccaaggag 19260
   aacgtctccc gagacgcact ccccgacaac ctcgtcctcc ctgatgacac cgaggagaac 19320
   caggagcccg cggctggtac gaaggatgtg gagctttaca cccacgtcta tcgccagggc 19380
   cgcaggtgga aggtctacca ggaagtcaag ggtgtccgca ttcccggcac cgagggttcg 19440
   tacccgctcg ataagagccc gtggattccc gttcgcttca cgcagatcga cggtgagagc 19500
   tacggacgcg gttacgtgga ggagtacatc ggggacctga agagtctcga aggactctcc 19560
   caggccatcg ttgagggctc cgctgccgca gcgaagatcc tgttcctggt gaacccgaat 19620
   ggcaccacgg acatggctga cgtgtccgag gctgagaacg gtgcgttccg cgagggtgtc 19680
   gcaactgaca tcacggtcct ccagcttcag aagcacaatg acttccgcgt tgctctggag 19740
   accatgaagg acatcaccga gcgcctggcg tttgcattcc tgctgaactc cgcagtgcag 19800
   cgcaacggcg aacgggtgac cgcagaagaa gtccgctaca tggcgaacga gttggagtct 19860
   gcgctgggtg gtatctactc catcctctcg caagagttcc aactgccgct catcaagcgg 19920
   atcatgtacc agatggaacg gcagaagcgt ctgcccgtcc ttcccgaagg gaccgtcaag 19980
   ccaatcatcg tgactggcat cgaggccctc ggacgtggaa acgacctgaa caagctgatc 20040
   cagttcgtcc agatcgccgc acaggcagcg aatcttcctc ccgagatcga caaggccgac 20100
   ttcctcaagc gtgctggtac ggcgctgggg atcgacatga agggtctcgt gttgccgcct 20160
   gaggtggtag ctcagaacaa ccagcaggcc atgatgatgc agatgatgca gcagggtgtg 20220
   aaccccgcca tcacgcaggc tggacagcta atgaaacaag gaatgcagaa tgccgcgcaa 20280
   cccgcaggcg ggcagtaagg ctcccgaggc caacactgcc gaagcccccg tggtcaccgt 20340
   tgaagactcg gtggccgaac agcaacccaa gcccgcagcg aagccggtca aagtgaccga 20400
   actacctggt ggcgtgaaga tcgaagactt ctgatgagtg tggattccgt agtcatcaag 20460
   cagccggacg ctccggtgga agaccaggcc cacatcgatg cgatggtggc caaggtggat 20520
   gctgccaata cttcgaccga accggacact cccgggatgc ccgcagaggg acgcccgcag 20580
   tggctcccgg agaagttcaa gtctcccgag gacttggcca aggcatatgc cgaactggaa 20640
   ggcaagctgg gtgggaagaa ggatgatgcc actccacccg ctgacgacaa ggccgcgaag 20700
   tctgacgaaa ccccggaccc aagcaaggcc acccaggacg atgcctcgaa ggctctctct 20760
   gagaagggcc tgagcttcga tgagttctcc gctgagtttg cccagaaggg tgaactgacc 20820
   gccgagagct acgagaagct ggagaaggct ggcatcccga aggccgtggt ggaccagtac 20880
   atcgctggcc agcaggccct cgctgagtcg taccgcaagg acgtgacctc ggttgccggt 20940
   ggcgatgaaa gcttcgctga gatggtcaca tgggccgctg cgaacctctc gaaggaagag 21000
   atcgccgcgt acaacaaggc cgtggactcc ggtgacatca accaggcgaa gctggtcgtg 21060
   gccggtgtgt accagaagtt cgacgctgct ggccgcggtg gtgagcctgc cctggtgact 21120
   ggcgctggcg gtaaggtctc gggcgatgtc tatgagtccc tggctcagat gcagaaggac 21180
   atggcctcgc cggagtacaa gaccgacccc gcattccgca agaaggtgga gcagaagatc 21240
   gcccgctcga acatcttgta aggaaccatc atgatcctgg agagcatcct gggttcggtg 21300
   gtggtccccg ctatcatcga cctcgtgaag ggtgctggtg gggccattag ccgcaagttc 21360
   tttggtctgt cggttgacga ccagatcaag attcaaaatg ccgacatcga gaagctcaag 21420
   gctctcgctg ccctcgacaa tccgtatggc acccccagcc agtgggtggt ggacctccgc 21480
   gcatcgttcc gatacatcgg cgctgccgcg gtcatcgctg tcggctgtgt caccctgtat 21540
   gccggtgtcc agaccaacat cgaagacgtg aaggagatgg gtttcgccct cgtgggcatg 21600
   cccttcggtt tcatcttcgg tgaacgcctg tacctcggcc tgaggggcaa gagcaagtaa 21660
   gcactgccgg gaagcagcgc attctgtacg gttctgatcc cgtaaccgtt acccacggga 21720
   tcacactgcc attgaagtga aaggtcctag ccgcactgcg ctcctgcgcg gtggctctgc 21780
   tgcatccaaa gaacaccaca acagaacctt ggcccgctga ggcggacaac cctgtgtgac 21840
   gtgtgagttc ccggaagccg ctcaacacga ctttcaactc acttccaaaa caaaaatggc 21900
   aaacgcagtt ccgtctcgcc tgggccaggc aaacctggca ggcgatccga aggccctgtt 21960
   cctgaaggtc ttcgctggcg aagtcatgac ggccttcgct gaaaacaaca tcgtacttca 22020
   gtacgtccgc cagcgcacga ttagttctgg caagtcggct tgacccaacc ttatgaactg 22080
   ggccgactct aaacaccccg taaattcggt ggaaccccat gggggcaata ccgagccaag 22140
   acttcgcagt acgcgagatg gtgtagagac tagacacggg gaacccacaa agacctgcgc 22200
   atgttgcaac gtcgagaagc ccgcccgtga gttctataaa aaggacgcac agacaggaag 22260
   gctcgatgga atttgcaagt cctgccgaat catcaagacc cgagagaaaa ccttaggggt 22320
   cactgaagat gactatcggc ggatgtatca tgtccagggc ggtcgatgtg gaatctgcca 22380
   acggcgcttg tactcaaaga ggtacaagag ttttgcagtg gaccatgatc acgagacagg 22440
   aaaagtccgt ggcttgttgt gccataattg caaccgcgga ttaggcatgt tccgagacga 22500
   cccgactgcg cttaggcgtg ctatcgactg ggttaaggta tagtccgatc ctcacagcaa 22560
   tgtgagtagg ggaagcagtt ccccgtaatt ggtaaggcta ccgccgcgta ccacacgccc 22620
   ggtaacgaaa tcaacggcag caacatcgcc cacaacgaag tggtcatcac catcgatgac 22680
   ctgctgctgg ccaacacctt catcgccaac atcgatgaag cgatgaacca ctacgatgtt 22740
   cgttcggtct attcgagcga actcggcaag gccctggcca accagcttga ccgccacctg 22800
   ctgcaactgg ctgtcctggc cgcccgctct gctgcccgta tcacgggcga acagggtggt 22860
   tcggtcatca ccgatgctgc tgccggtacc gactcgaacg cactggtcgc ggacatcttc 22920
   tccgcggctc agaagctcga tgagaaggat gtcccggctg atggccgtgt gtgcttcctg 22980
   cttccggccc aatactacgc cctggcacag aacaccaaga ttctgaacaa ggattggggt 23040
   ggtgccggtg tgtatgcgga tggcaaggtc ctccgtgtgg ccggtgtgga gatcgtgaag 23100
   acgaaccacc tgccgaacac gaacatcgct tcgggttcga ccgcggctgg tactggcgat 23160
   aagtacattg gcaacttcac gaccaccgtt ggtgtggtca cccagaagtc cgccctgggc 23220
   accgtgaagc tcatggacct ggcgatggag tctgaatacc agattcagcg tcagggcacc 23280
   ctgatggtcg ccaagtacgc aatgggtcac ggcgttctgg ctccgcaagc ggctgtcgaa 23340
   atcaagaccg cataagcgtc ccctcaagcc tcgggaggtt ctcttcaaga gttcctcctg 23400
   gggctttttt ttctgctctc aaggatcacc aattggcaac caagactcaa actgatcgcg 23460
   ccaaggacgg tcaggatttc ttccagcttc cggcctacaa ggacacgccc acggtcaccg 23520
   tgaatggcac cgtccgtgct cgcacgactg tctcgagtgg cgtccagctg gcaacccctg 23580
   cggctcagga tgacatcgta gcgatcacgt tcaactcggc tgaccctggg aatacccgcc 23640
   gcgaggtctt caccccggcc actggcgcaa ccatcacacc caccgagttc tgcatcgagg 23700
   cccgcattgt acccgcaggc accattgcgg ccctcaccat caccttcccc ccgaacccct 23760
   cgaaggaagg ccagcagttc cgtgctgtca ccacgcagac catcaccgcg gtgacctgga 23820
   ctggtggctc tcgtctcaac gctcccacca cgctagccgc tggccgtgct gccaccttcg 23880
   agtggagcgt ggcgaagcag gagtgggtct tcatcaacta aggaaaacgc atgaccacca 23940
   tcgtcactcc gaccacggag cttgaggcgg tcaacctgat gctcgatgtc atcggggaga 24000
   gcccaatcag caccctggag aacagcgctg tggtggacgc ggtgaaggcc aaggcggtcc 24060
   tctccgaggt gtcccgcgct gtacaaacga agggctggca cttcaacacc gagaaggggt 24120
   tcgagctagt ccccacggtc ttcgagaagg agatcatcgt ccccgccaac tgcctgcgca 24180
   ttgatacggt ctacccggac gagggcatcg atgcagttca ccgtggcact cgcctctatg 24240
   accgccgcag gcacacctac cagttcgaca agagtgtgaa ggtggacatg gtggtcaacc 24300
   tccaattcga ggaactcccg gaatccgccc gccgctacat cgccatccgt gccgcacggg 24360
   tcttccaggc ccgcatagtg ggctctgaga gcctctacca gttcaccgca gaggacgaga 24420
   gggacgcccg agcggacctc aagaaggctg agggcatcac gggggactac aacattctga 24480
   cggacagctg ggctgttcgt cgcgtcatcg atcgctgata tgcccctcgt ttcttcttcc 24540
   atcgccaaca tggtgaacgg ggtctctcag caacccttca cgctgcgtct cgcgtctcaa 24600
   gctgagttgc aggagaacgg cctcagtacc gtggctcagg ggttgaagaa gaggccccca 24660
   accaagcaca tcaaacgcct cggcagtgcc atcaccggct ctgcctacat ccacaccatc 24720
   aaccgtgact ctgtggagcg gtatgaggtg gtcatcacga acggtgacct gaaggtctac 24780
   gacacggcag ggaaccagaa gacggtgaac ttcccgaatg ggaaggcgta cctgaactcc 24840
   acggaccctg ctacgtcctt cagggccgtc actgtggcgg actacacgtt tatcgtgaac 24900
   aagaagactg tcaccgcggc cagtgccacg aactccccaa cgcggccctt cgagtccctc 24960
   gcaaacgtga aggttgggct ctactcgaag acctacacca tcaccgtctc cggtgtgggc 25020
   acggccacct atagtacccc cgatggcacc gttgcggccc acgcggcaca gatcaccacg 25080
   gactacatcg ccaaccagct tgcgaatggt ctcattaccc tcggtggatt cacctcagtg 25140
   aaccaggtag gctccgtcat ctacatcgcc cggcccaccg attacaccat ctccgcaaca 25200
   gatgggtata acaacgcggc cctgaacgtg attaagggga cggtgcagag gttctcggac 25260
   ctccccgcga atgcgaactt ccaggacttc actgtggaga tcgcagggga caacacctcg 25320
   gagtccgata actattgggt caagtttgac aagaccggga acaactccgg tgtctggcgc 25380
   gagaccatca agccaggcat ctcggttggt cttagtccca gcacgatgcc gtgggtactg 25440
   gtccgtgagt cggacggcac gttcaccttc aaacccatct cctggacgaa ccggctggtg 25500
   ggtgatgaag actccgctcc acacccatcg tttgtgggcc gcaccatcca ggatgtgttc 25560
   ttctaccgga accgcctggg cttcctcgcg gatgaggcgg tggtgatgtc ggaggctggc 25620
   cagttcttca acttctaccc gaccacggtg acgcaactcc tggattccga ccgcatcgac 25680
   gtatcagcat cccacacgaa agtctcgaac ctgaacttcg cggtggcctt caacaaggac 25740
   ctcctgctgt tctcctcgca gactcagttc tcggtggaat caggtgacct cctgacaccc 25800
   aagagcgtct ccatcaagcc caccacggag ttcgagtgca gcacccttgc gcctcccgtt 25860
   gggattggac gcaacgtcta cttcgcggtc cctaagggtg agttcgaggg cttccgtgag 25920
   ttctacgtag cggacaacgc aggcaccaat gatgcggctg agatcaccgg ccacgtcccg 25980
   aagtacatcc cgaagggggc ctacaagatc gctgcggctc tcaacgagga ctttcagggg 26040
   aacccaacgc gatgtatgcg tacaagttct actggaacag caacgagaag ctccaaagct 26100
   cctggtccaa gtggaccttc ccgagcacgg acacgattct ccacgcggag ttcatccagt 26160
   cggaactgtt catcctcatc aaccggcccg atggtctcta cctggagaag ctcagtgtgg 26220
   ctctcgggga catcgggacg aacgagccct acaacgtcca cctggaccgc aagctgacgg 26280
   tgccgaaagc aagcctcacg tatgacggca cgtacaccat catctcctcc gcggctctcc 26340
   cgtggaaccc aacggatgga acgtacacgg cagtggtggc caccagtcag ccgcagaagg 26400
   ctggcgtcct ctacccggtc atttgggatg ggacgaacgc caagattctc ggtaaccgtg 26460
   tggactccga cctcatcgtt ggtaggcgct acgccttccg ctatcgcttc tcgccgctac 26520
   tggtccgcca gcagtccggc cagggccaga aggcggacac ggttgcacgt ctccagattc 26580
   gcaacatgca agtcaacttc tcggagagtg gcaacttcca ggcaaaggtc acgccttacg 26640
   ggcgggacac ctacacgtac acctactcag gaaagaccct cgggctgcct tcggcaaaca 26700
   tcggggccat cggaattgaa gatggcaagt tccggttccc ggtgatgtcg cagaacacca 26760
   ccgtggacat cgaactcttc tcggactcgc cgctcccctg cgccttcttg agtgcagatt 26820
   gggaaggcta ctatgtccga cgaagccagg cggtctaaac catacgtccg tcctgcaaca 26880
   cgcgaagact gcatcatcct cgcaaggaac ctccgacagg aagacgcgga ggagatcgct 26940
   catgtgaacg gtctccccgc ggagatgaat ctcttgctgg ggttccgcac ctccgctcga 27000
   ctttatgcgg tggtgtgggg ggatgagacc gtggccgtgt tcggcatcgg gggagtgcct 27060
   ggcgtcatcg gcttcccctg gatgctcgct tcgccctccc tctcgaaaat ccgcaagagc 27120
   ttcctgaggg agtgccgcgg gtacgtggag gggatgctcc aggagtatcg ccacctggag 27180
   aactacgtgt gggcaaagaa cgaagtccac atccagtggc tcaagtggct ggggttcgag 27240
   ttcgagccag cagcaccatt cggtatcaat gacgaaccct ttcacagatt ttataggagc 27300
   atgtgatgtg cggaccagcc gcagttccaa tcgccatgct gggtatcagc gctgtgggca 27360
   ctgccgcttc gattagcgcg cagtcgcagc agcagaaggc acaggatgcc ttcaaccagc 27420
   gccagtatga aaacgacatg accgcgtacc gaggcaacct cgccaacatc gaggtgcaac 27480
   ggaaccaggc gcgggaagat gcagtagcgc agaagcagca gaacgacatg gcaggaaggc 27540
   gcgcaacagc aaccgccacg actgccgcag gtgaggcggg tgtctcaggc gcctcggtgg 27600
   atgcactgct gcgggacctc gctggccagg ctgcctacga caacaccaac gtggatgaga 27660
   actatctgcg ccaggacagg gctctgaacg cccagcgtga gaacgccttc aacagcactg 27720
   caagccagat caaccagctt cgcccctcga tgtccccgga ctatctcggc gctggtctcc 27780
   gcattggcca ggctgctgcg ggtgcttaca gccagtacca gcagaacctc gactacgagc 27840
   ggaaccagag cgtcccacgc cgaggagcat aaatggcacg agttcagaca gactatcgaa 27900
   cccgaggtac agggcttcag gacatctcgc ccccaatgct tcagccgcag caggcagggt 27960
   tagacaatgg tgccgctgag tctgccgcac ggctggccca ggcgttaggg gctgttgacc 28020
   tgtctccgct ggtaaccgcc aagcgatacc aggatgtgga ggaggcggag aaggcacggg 28080
   cctacgccaa ctccctcacc gtggaggagc ttgggaagca gatcaaggat gggaccctca 28140
   tggcgtccca ttcgcctgtc ttcagggcaa cggtcgaaca catccacggt gagaacacgc 28200
   tcaacacgtt cgagcgggac acactctcga agctcacccg cggggaactg aagttcgaca 28260
   ccccgcaggc catggatgag tacctcacga agtaccgcaa cgaggccctc acgggatcca 28320
   gcaagttcac cactgcgggc ttcgataagg gctacggcac gttccgtgag cgagccatcg 28380
   cggttaacgt gaaggtggcc gatgaagagg ccgtgaagcg cggcagccag gaagcctcgg 28440
   acaacctcgg caacctgacc ctgcaagtca ccgacccgat gtacaagggt gacgctgcgc 28500
   aggccatcgt ggaccgctac cagcttcttc ggaagacctc tctgctgcgt gacgatgccg 28560
   cgaaggaagc tctctcgggt gtcgctgcga accttgcagc ctccggcaac aaggccctcc 28620
   tgggttctct gctggacaag aagttggaca gcggtgtctc cgtcaaggcc gctctggggg 28680
   acctgaaggc catccagttc acgcaacacg ctgaacgtga gtatgaccag gcgcagcacc 28740
   aacggattga cgttgagatt cgtccgttcg tggagcaggc cgacaagggt gaactgaagc 28800
   gggatgcctt cgacaagtgg ggggccgcga atgagaagta cgtcaccacc cccaccatcc 28860
   acgccatcat caagggcaac gaggcggcca tcgagcggca acagaagctc atcgctcaga 28920
   acgccctcct ggcccaggcc gaagcaacac aggctcaggc aacgcaggca gcccgcacgg 28980
   ccatcgacca gggcaacctg gcgttcctcc cgcagcagaa ggtgatgaca cctcaggggg 29040
   aacagaagaa cttcgatacg aaggccgctg ctgtcccgta catccaggaa cggattgcac 29100
   gggagaacat gccgttcggt aagcaggtgg agttctggtc caccaacggg gtggagaatc 29160
   ccgagtggga gaaacagatc aagggtggcc tctcgaacct cgcctccgcg ggctggacct 29220
   tcgatggcaa gaccattggc caactgaaca accagggcca ggccgcaatc gacaccttca 29280
   tccgcatcaa cagcaccaac cccggctacg ctgagaagtt ggtgggcggt gacaaggact 29340
   acaagaagct ctccgacatc cagttcctca tggagaaggg cggcttcccg aacgtcaacg 29400
   atgctgcggc actcatcaac cagattgacc gcgctgacat caaggcatcg gactacggtt 29460
   cgatgaagca gaaggtggcc tcctcggtgg acgatgtggt gaaccagcat tggtactcag 29520
   gcgccaccag ttggttcagt ggcctcttcg gcaatgacca ggtgaacctc accgctgtct 29580
   ccgctgacat tcgccgcagg gctgaactcc tggtgatgtc tggccaggtg cccgatgcga 29640
   acgccgcggt gaaggccacg gtggaatacc tggcgaaccc cgcagtcacc acgcggatca 29700
   acaatacgct ctacttcaac aaggaccttc cggtggtccc gaagggcgag gacaccgggc 29760
   agtggatggg gcggttcatc aaggacgttc cccagcagat cgccaaggcg aacaacctcg 29820
   gtgatgctcg cctggagccg aaccagtacg gaggcttcac ggcctggact ggtggtgtcc 29880
   cgatgacgga cggcaccggt aaggtggtca cctacacgcg ggatgacatc tcgaagtggg 29940
   tggacaacac catcaccgct gaccgccaca aggccgctgc tgatgccaac ttcaagagct 30000
   accaggaccg cctcgtgaag gaactccgcg atgaaaagca gaaggacccc tacgtgatgg 30060
   agcggatgtt cgacgcgact gccaacggca tgtggtggaa ccgccaactc tacagccgcg 30120
   aaggctatga gcaggttctc cgtgacggca acacaggcaa gccgctcaac gaactgttcc 30180
   aaatctacaa agacaaacgc ttcaaggata agtaatggcc gcatcgatcg ctctggggga 30240
   tgtccagagg attacctccg agacggagaa gaagtacggg ctccctgaag ggacgctgtt 30300
   caagatcgga aacatcgagt cctcgttcca ggatggccag gtgagcccga agggagccaa 30360
   gggctacttc cagttcaccg atgacaccgc aaggcgctac ggcctggatg atccgttcga 30420
   cttcgagaag tcatccgatg ccgcgggccg gtacatgcga gacaacctgg ccaagtacca 30480
   gggcaacatg gacctgtccc tcgcggacta caacggtggc ccgaaggccg ctaaggctct 30540
   cgccaagggg aagccctggg cagagacttc ggactacctg gcgaagttct acggcaacaa 30600
   gtccgagccg ctctcgcagc aattcaccac gggctccgaa gtccctctta ctgcctcccc 30660
   ctccgcctcc cagctatatc gagacgcacg gcagcaggag tctgagtatg gaggggttgg 30720
   caataacatt ctcaatctgc ctcgtgctat tggcctgggc tttcaagtcg ataattcggt 30780
   ctacaatttc tggcaggagc gaggactctc cagcgtagac cccgacttcc gctgggacga 30840
   tgacttctcg aagcagatgc ttgatggggt ccctgagcgt cattggggat acctgctgca 30900
   atccaagtcg aagcaggaag cggaactccg ccgtgcccgt ctgttggaca cgatggagaa 30960
   ggaagtcgaa ctctccaaga tgggtgtggc cggtttcggt ggtcgcctgg tgggcaacct 31020
   ggtggatcta cctacgctca tctcgttcgt ccctgggttc ggtggtgcgg gcctcctcac 31080
   gaccacttca cgcatcgcca atgctgcccg catggctgcc ctcggtgctg ctacgaacgt 31140
   agccttcgat gctgcaacga tgcagttccg ccccacggcc accccggatg acctctacat 31200
   ctccgctgcg atgggcctgg gtctcggtgc tgctggtggc ctctcggtga atcctgcccg 31260
   cctggccgcg caacgtctcg ctgctgagaa ccgccgcctc ggtgagttcg gtctccgtga 31320
   atccggcaag gcgcagatca aggagcttgg cgacaacggc ttcaacttcg gtgctggccg 31380
   tgaggagttc gcacggcgca tccaaggcaa gcccgatgag ccggtggaga tcaagtaccc 31440
   aggcggtgca atcgtgctgc cgcggggcga tggtgagcct ccgaagattt tccaccctgg 31500
   tgatccccct gaggttcgca agccagggaa catcaacgag ccgcttcctc ccgaagctcc 31560
   tccagctact cctccggcca ccggcccggt tgctcccaag gctcctccag cagaggcacc 31620
   taagggcaag ggctggacct ccgagtggga cactccgcgg tacgcctcag gcggtggcaa 31680
   cgagcaactc ctcgtgctgc ctccggcaaa gcgtgtgagt cagttggctg agtatgtccg 31740
   ccagttctcg aagaacgggg acatcgtgaa ggtgatggac cgggtgctga agggcatcga 31800
   cctccgcaag ttggagttca aggtcatcga gaagggtcag cgtttcggcc agcgtgacat 31860
   ggacaacgaa atcctcggcg cgaagggcgc tgtaggtact ccgcgaggtt ccattggtga 31920
   caacatcatg atgttcctgc ggggccactc gtgggagatg cctggtgtca acccgatgca 31980
   cacggtgggt ctcaacgagg agacgttcgt tcacgaactc gttcacgttg ccaccatcta 32040
   caagctccgc ggtgttgagc ctggcatggg tgtacgcatc acggaccctg ttgtgcgcag 32100
   ggctgctgat gacctggcga acctccacgg ggacatcctc gaccacgcca ggcaaacctt 32160
   cggggccaac tggaaaggtg aactccaggg acgcctcggt gccaacctgg agaacgagaa 32220
   ggaactcatc gcctatggtc tgacgaaccg gaacttccag gagtggctca agacggtgcc 32280
   cgttgagggt ggccctgaga agaacctgtg ggaccgcttc gtgcattccc tgcgcaagct 32340
   cctgggcatc ggcccgaagg aacacaacgc cttcacccgg ctgatcgaac tgtccgcccc 32400
   tctcacgaag aagggcgact tcgttgagcg catcaagacg aacccagagt tggaagcaac 32460
   gggtgggttt gttgacgctg acaccgtgaa ggccgcgaac gaagctgacc tggctccggt 32520
   ctatggctgg ggtctcggcc tggagaacag gctgggtggt gctaaggctc cctccgctgt 32580
   tcgtcagttg gcctcgaagc tgttcggcac caccatcggc tacaaggaca acgcggtggt 32640
   gaagctcaac gcttgggacg acaccacgaa gtgggctgac tcctgggccg tggagatgcg 32700
   caagggcacc tatccgcagt tcgaggagtg gctcaagggc tctcagtaca agtggcacga 32760
   gaagggcaag gcgttcgatg acttcggcgc acaggtgtcc aactacatcc gcggcttcga 32820
   gggtgattac ccaccgcagg tggtcaaggc tggcgagcac atgcgcaaga ccctggccaa 32880
   cgtggtggac tacatcaaca gcccactgaa ggacgaaggc cgagccaaga ttggtctcac 32940
   cgagacggac atccgagacc cggagaccgg caaggtggag cgggtaggga cgctggagaa 33000
   gaacccgaac tacctcccgc gcaagcacga catcaacaag tggaactcga tggtctccaa 33060
   cttcggcagg gatgccgtgg aagggtggtg ggcacgggcc taccaggctg gccgtgaggg 33120
   aatctctgac gaggccgctg cgaagtgggc caagtggtat gtccgcacgg tggaggaggc 33180
   tcacgccaac cgcactcagg acatgctcga tgacctcctg aagggcaccg atagggacgc 33240
   cctgaagaac tccctgatgc tcaacggagg ctactccgaa gcggaggctc tgcggatcat 33300
   ggacgacatg attcctggta gggccaccga tgcaggccgc acgatggcca gcctgaagca 33360
   ccgcaacacc atccgggaaa cgcacaccga gcagtggacc acgaaggacg ggacgaagat 33420
   ggaggtgagt ctgaacgact tcatccactc gaacgccttc gacgtggttg agccgtacct 33480
   ccgcaggacc gcgggcagtg tggcgctggc caagcatctc gacatctaca agatggggga 33540
   cattgaccgc gttatcgctg aggccaccgg caacaagctt gggcaggagt tcaagtccac 33600
   ccccgatatt cagaagctcc gcaaggacct gaagttcgcc ttcgagcgag tccaagggct 33660
   tcccctggag gagttctcca cgctgaacaa gagcctggag atgtggcgca acttcaacgt 33720
   tatccgcctg atgggtggag cagtctggaa ccaggccacc gaactcagcc agatcatcgg 33780
   cacgatgggg tggaagacta cgcttgcggc tctccctgag cttcgagcac tgcgccgtga 33840
   catcgccacc ggcaaggccc cgcatgacat cctggaccac ctggagaaca ccattggtgg 33900
   cgtagggtcc gagtacgtgg cccgcctgga gttcaaggct ggtgacgatt gggtccgcaa 33960
   caagggggac accaggttca accgctggct ggactctgct gacaccggca ccaggaagct 34020
   ggcgaaaggt gtgctggatt acaccggcat gactccgctg atgattcagc agaagcgtgt 34080
   ccacgcgatt gcgttggtga accacttcgt caacgtggcg aacggcaagg ctgctgggtt 34140
   cctcacgaag gatcgcctgg cctggatggg tatgagcgcg gatgacttcg gcaaggtcct 34200
   gtctggcatc aagcagttca ccaagcccgc tgatggtgag ttctcgaaga ccttcaagat 34260
   ggacttcgcg ggctggcaga aggcggaccc ggagagctac tcgaagttca tgacggccat 34320
   ccaccgtgaa tcccgcaggg tcatccagga gaacgacctg ggctccatga tcccgctcat 34380
   gggcaccacg ctgggcaaga cggtcttcca gttcatgaac ttctcgatgc acggctggaa 34440
   caagtcgctg atgttcgcca tgaaccaccg cgactggtcc acactgtcca ccgtacttca 34500
   cggctcactc ttcgcgtcca tcgcctacat ggggcggacg ctgctgggtg ccggtggcat 34560
   ggaagcggac aagcgccagc agtatctcga caagcggatg tccgttggcc agatcgttac 34620
   caacagcttc gggcgcatct ctcaggcgtc cgtgctgccc aacatgttcg acaccatctc 34680
   accgtatccg ctgttcagcg gaatgcggac cacgagtgac ctctccagtc tggcatcgaa 34740
   cccgacctac caggccatca acggactcat ctcgatgaag aagctgattc ggaatggtgt 34800
   gtcggatgag taccaaacca cggagaagga catccgcacc tggggcaggc tactgcctct 34860
   caacaacgtc ttcccggtga ccacgttcct gaaccacctg gcgaacgatt atccgcacgg 34920
   cgaaaagcaa caataaacgg gtagccctcg gcacgaccgg gggcaacctc ttttggagaa 34980
   tagatagtgc cttacagtta cgttcttctc tcggggaacg gctctgcgac caacttcggc 35040
   ttcagcttcg gttatctcag caagttccac atcggagtga aggtgaacgg tgtagtcacc 35100
   accttcacct gggtgacgga cttcaccatt ggcatcacac cggccccggc caacggtgca 35160
   gtcatcgagg ttcgacggac gactccgttg aatcaacccg ccgtggactg gtcagatggc 35220
   tccacgctca ccgaagcgga catggacctc aacactcggt tctctctgta cactgctcag 35280
   gaggccgctg atggtgttgc agcatccatc actcagaact ccctggggca gtggggcggc 35340
   cagaaccgca gggccgtcaa cttcgcagac ccggttgatc cacaagacct ggtgaacaag 35400
   cgatacttcg aggacgtgta cacacctcag ttggacgcga aggtcaccga agccaccaac 35460
   caggccaaca acgcggcctc cagcgccgcc actgcgcagg gctatgctct cgctgcggac 35520
   aactccgcgg acctcgctgc ggccctcctg gcgaccttca aaggccagta cctcggtgcc 35580
   cttgcatcta accccacgct ggacggtaac ggccagccgg tgactgctgg tgacctctac 35640
   ttcagcacca ccgataacct gatgaaggtg tacaccgggt ccgcgtggat caacgctggg 35700
   tcaaccgtcc agtccaccat caaacgtcct gtcacaccca tcgtggcaac cgcaggccag 35760
   accgtgttcc cggtgtctgg tgggtacgac gccccataca ttctcgtgtt tgtgaatggg 35820
   gttgaggtgg cttctccaga tgtggacgtg actaacggca gcaccatcgt attctccagc 35880
   ggcctgactg ctggagataa agtggattac gcagcgtttg gtgcgttcca ggtggccaac 35940
   ccggttatcg atgggaccag cgccgcagac ttcatcaaga cacgcaatgc ccgtgtagtt 36000
   acctctattg ccgacctgaa ggccctcaat aagaacacct acaacttcgt tctcgtcact 36060
   ggcttctatg cttcagggga tggtggcggc ggtttcttcc ttcaggttcc cacgatgccc 36120
   accaacggta tcgttcaggt cgggaatgac ggaggcatct ggcagttggt ggttgatcgg 36180
   gattatgttt ccgcgaaaca actcggtgcg agactggacg gttcaacgga tgactcctct 36240
   ctcctgaaca acgccaagtc cactctcgat gctcttggta agaggctgta tatcccgtct 36300
   ggggtttgca gaatctcaac agcaatcact ccaccaaagg ctggtgtgtt tggggatagt 36360
   cctcaagcgt ccatcatcca gtgtaacaac tgctctgcat tcctattccc agcaaatttt 36420
   gggctctctc gtccggcttg tgtcattgag aagttgggga ttcagtccta cagcaacacc 36480
   tgcgatgggc tatacgcttt ccgtgcccct ggggtggcat caggagcatc gcccgtctac 36540
   aacagcggcc taactgttag ggatgttgag attggtacgg gcggacgatt cggtggcggt 36600
   ttctcactga aggacttctt ccgagtgaac gtagagaaca ttggcatgac tgatgtgagt 36660
   tccgccgtat tgctcaccgg gtcagttgtg caggcagtat tccgaaatgt caccgcaaac 36720
   ggtgataacg caccaactgt tcttaaccgg tatggtttcc aaacagccgc agcttcctat 36780
   tccagcggta cgctaggtcc tgaacacatt agtacgtggg attgcagctt cattcgctat 36840
   acacgcggtg ttcaacacga tgctgggctc atggtctcgt tcaacaatac ggacctggaa 36900
   actttcacac acggcttcta tctctcgcag ccctgcactg tgcgtggtgg tattagcgcc 36960
   ccggctccgg cagcttcagg gactgctgcg tggattgggc ttttcaaagc tatttctgat 37020
   tttgacgtag ccaacggcac tctgatcgat gaccttgaga tcaacacgct aaacacccca 37080
   ggaactccag cctcttcgta tggggttctc attggcaaca atgtgaataa gtgcgttggt 37140
   actacaatcc gtagtcccag gattcgaggt aacactagtt caatggtcgg tgggattgtc 37200
   gctaatctag ctggaggtga catcgttatc gaggatgcca tcatcaacgg cagtgtggtt 37260
   actggaacta cggtgtctgt gagcaatgct tcctatgcaa gggttgtggg caatcgaagc 37320
   gccaccggtg ggactgtaaa tggttccctg tcaatcacag ataacggtgt tggttccatt 37380
   ggtgatgttc gtggaaatgc gtttgccacc attaccaaca ccctcaatgc ctattccggt 37440
   acatggacgc ctggaacaat tcctaacgga acaccagcag caacaacggt ggccgtccct 37500
   ggcgcagtgg ttggtgacaa agtagtggtc ggcctttcca gcctgaccgg atcggccaac 37560
   tgcatcattt ccggctatgt gtcttccacc ggaaatgtgg ctgtcctgtt gtataacgtc 37620
   tctggtgcat cacagacgat tccctccggg actctccagg taacagtcct caagtcgtaa 37680
   tcaagatgtc cctctaggtt tcccctggag ggacttcctc tttcaaggaa aggtatgagc 37740
   aatgcgctca acgtaagtaa gctggccaca ctcacggcaa cagaaatcaa agcataacca 37800
   aacaagaagt aatcatgccg aacatcgaca aagacgtaca gaaggatgct ctgaaggagg 37860
   ccctcacgga gtggctggac aagcagttcg ccacattcgg gaagtgggcc ttgcggtcca 37920
   tcctggccgc tgccttctca gtcctcatgt acctgtacct gacttctcaa ggctggcacc 37980
   gctgatatga ccgaaaagac caccgcttcc gaaaaggagc ttggcgaagt ccacaacgag 38040
   atggccgcat ggtgcctgga catcctcaag ggaatcccgg tcaccgacaa agacggtaac 38100
   ctcgtgattg aggatgggag agttgttcgt ctccctccgg ctcctgccta cctcaacgtc 38160
   attcgccagt tcctcaagga caacgacatc caggctgaac ccgccaaggg ctcctcgatg 38220
   ggtgacctct cggacctccc ggtgttcgag gatgacaacg ttgtgcctct caagtctcaa 38280
   tcgaaataaa cgcgattaga ggccctcaga gcgattttaa gcctccaagg tagggtagcc 38340
   tatccgggca cctgatcgcg tcctgtgggg ccatctcgca agccaagaat gaaaataaca 38400
   actgccgagg tttcggcaaa acgctgcccg aagtgcggcg aagaaaaaca cctctccgag 38460
   ttacacgcga atcacaccaa gagggacggc cacaacacca tctgcaagct ctgcatgaag 38520
   caggtggcac gagactggcg caacacacct ccgggccgct ccaagcagat gtggacgacc 38580
   tcaaagaaac gtgcggagga gaggggctgg gagttcaatc taacccccga gtggattcag 38640
   gaacgcctcg aagctggcgt gtgtgaggcc accgggattc ccttggagat gtccgcggag 38700
   gagttcaaag gctacggcca cttccgtcca tggaccccct cactcgaccg agacgatcca 38760
   acgaaagggt acacaaccga caacgtgaag gttgtgtgct ggatgtacaa ccaggccaaa 38820
   ggcgtaagca tgcacgaagc cgtcctaaga atggcccgtg ccctcgtagc gaatgacaac 38880
   taaacaacac ccagcacaga aagactttcg cgtctttatg ttcatggtgt ggcgccacct 38940
   caatctcccc gaacccacac cagtccaata tgacatcgcc cactacttgc aacacggacc 39000
   acgccgttca gtcatcgaag cgttccgtgg tgtaggtaag tcctggatca cctccgcctt 39060
   agtttgctgg gttctgtgga acgacccaca gaagaaaatc ctggtcatct ccgcctcgaa 39120
   ggaacgagca gatgccttct ctaccttcgt gaagcggctc atcaacgagc ttcccgttct 39180
   ccagcacttg aagcctaagg cggaccagcg agactcgatg atttccttcg atgttggtcc 39240
   cgcaactcct gaccactccc cctcggtcaa gtccgttggt atcaacgggc agatcactgg 39300
   ttctcgtgcc gacatcatca tcgctgatga cgttgaggtt cccaataact ccgccacgca 39360
   gatgatgcgc gacaagctct ctgaggcggt gaaggaaatg gatgcggtca tcaaaccgct 39420
   ccagacctcc cgcatcatct atctgggcac gcctcagacg gagatgtcgc tgtacaacgc 39480
   tctccctgag cgtggatacg aagcccgcat ctggccagcg ctgtaccccg agcttcacct 39540
   cgtggccaac tacaagggcc gcctggctcc attcatcacg cgggctctgg aggccgataa 39600
   gagtctcgta ggtgctccta cggaccccag gcggttcaac gagactgacc tgttggagcg 39660
   taaggcgtcc tatggacgtg ctggcttcgc tctccagttc atgctcgaca cgagcctcag 39720
   cgatggtgac cgctacccgc tgaagatcgc ggacctcatc gtccagaacc tcaaccccac 39780
   gatggcccat gtgaagatcg cctgggctgc tgcacctgaa gtttgcatca acgatctccc 39840
   cgcggtggcc ctcacgggtg accgctacta ccggcccatg tggacggacc agcagatgtc 39900
   cgagtacacg ggctgtgtca tggccatcga cccctcgggc cgtggtgctg acgagaccgg 39960
   ctacgccatc atcaagattc tcgcaggcaa cctcttcctg gtggccgcgg gtggactctc 40020
   cggtggctac tcagatgaaa ctctggagac cctggcgaga ctcgctaaga cccaccaggt 40080
   gaaccacgtc atcatcgagg ccaacttcgg tgatggcatg tacaccaagc tcatcactcc 40140
   attcttcggg aaggtgggac acaaggtcct ggtggaggag gtgaagcact ccacgcagaa 40200
   ggaagcccgt atcatcgaca cccttgagcc tgtgctctcg actcatcgtc tcatcgttga 40260
   ccagaaggtc atcgagaacg acttcaggac ggcagagcag gacatcaagt acagcctgtt 40320
   ctaccagatg acccggatca cccgagacaa gggtgccctg gctcatgatg accgtctcga 40380
   tgcactggcc atcgctgttg cctactggac ggagcatatg tccagggaca acgataaggc 40440
   cgctgctgcg atcaaggaca aggcgctgaa ggatgaactg aagaagttcg ttcacggtgt 40500
   ccttgggcgc aaacccaagc gaacctcgtg gatgtcctcg aactcaggct ccaggtgaca 40560
   ttcggtgcca caataggaga accctacgtg ggttcttcgg gggcttcatc cgtagctgat 40620
   atggatgcca cacaccgtgt ggactcggga aacctcagtg tgtggtgatg tagtcgctgc 40680
   attctaggac acccgttagt ctccctattc ctcatctcta tgggggggta ggggggctaa 40740
   cttaggtgtt cctagtgttg atgatatagc cactgagatg tcaacctcag tgtcccttaa 40800
   gttgtctctt agggttgcat taaggagaca tcatcaccat catctcccat aaggtcatcc 40860
   tccccatgtt cactctacta gtcctcctct caggtgtccc cgtggtgttc cttctgggtc 40920
   tcgttctgta tggcctgttg gacaactgat ggtgtccctg aagtgcccct tagggggaaa 40980
   acttccgacg caaaaatttg aaagccccac tcgaaattcg acgcgggcag attccccccg 41040
   tgccccctcc gcggcccggc cctcgtggcc cctgccgacc cacctccggg caccctccag 41100
   gctgtacgct ccgctga 41117
<210> 3
   <211> 40589
   <212> DNA
   <213> T7-like viruses
<220>
   <223> /host="Ralstonia solanacearum" /isolate="vRsoP-WR2" /isolation_source="Yátor river, Alpujarras, Granada, España" /note="Genome of the isolate vRsoP-WR2"
<400> 3
   gacaactgat ggtgtccctg aagtgccccc ttagggggaa aacttccgac gcaaaaattt 60
   gaaagcccca ctcgaaattc gacgcgggca gattcccccc gtgccccccc gcggcccggc 120
   cctcgtggcc cctgccgacc cacctccggg caccctccag gctgtacgct ccgctgactc 180
   ctggcacatc ttctggcaca ctctgccgta actccctgat tactaagggg atgcactagc 240
   ttacgaagct actgcgaccc aataagcctc acgcatgagc actcactggc tcactcgtgg 300
   ggcttttttt tctattctgt ccccatttcc gcgcccccct gttcggccat cagtttgctt 360
   tggtttctcc tagggtttcc cctaagtgtc tccttggcgt gcatcgctac gattctccca 420
   acggcccact tgcggcccac cactggagaa catcatgcaa ctgcaatact tccgcgactt 480
   ggcaatcggc acagcgttca ctatcgctgg cacgccctac gtgaagaaaa gcgcacggac 540
   tgcgtacacc gctcccggcc accctgggca ttgggaaggc cgctggttct ggtttggtca 600
   gactgaactg gtaatggcct aagggagcac accatgagca aagtccgagc actcgcctac 660
   ttcttcgctg caaccacgct cgcactcgcc tacgtgggcg caagggcagc acatgcggcc 720
   atctcaagcc tcctcgtgat gcacctgcat tgatcccact cagaacaccc tccttggcgg 780
   caaagccgct acagaagcct ccagatcaac gtctgggggc ttttttgttt gcccctgggg 840
   ctgacctacc tgcgtcccac tgcgtggctc ctagggcttc ctatcgttcc ttcggagcaa 900
   cgctcctgat atcggaacta ttgcagtgat tgaaaaatac aattgggcag tctccgatgt 960
   ttcgtatgta attcggtctc accaggggga cacgcccctg aagacaaaaa agcgtgggac 1020
   cggggcggac gccagcagtc agggacaacc cgagtcaatc caagagtaag cacattgcga 1080
   gtccttccag tgtgctcatc actggagaga catcatgcaa tcattcaccc tgaacattgg 1140
   ccttatcccg agcaagaaat cttcgcgtac cgctcgcatc actgcatcgg aagttaaggc 1200
   cgcacttcgt ggcgctggct tcttcgtgtc gggctttcgc atggcccagt cggccaccga 1260
   gcctaccgca gtggtccgcg tgatcgcacg tcagccaatg agctatcacc aagcgctcta 1320
   caacgtgtcc ctggcgctgg tgcaggactg catcgcggtt gtccctgaca cggtacgggg 1380
   cgcgttgatt ggcccggatg cggctgagtg gggtgagttc aatccggcct acttcatccc 1440
   gtttgatgtc gaaccgcagg caatcgctgc gtgacactta gggtgcccct tcaggggctc 1500
   caggagtagc cgcattgcgc tgtgcagtgc gcctatcact ggaggacaac atgtacggaa 1560
   actttgaccc gagcacgaac gcatggccgt tcagtgtgga gtttgtggac gctgtaggct 1620
   ggcaagtgga ggacaaccgg gaccccacca atgtcgcggt gatggtcgct ggtctcacct 1680
   tcgaggaagc caaacagcgc gcgtctgaac tcaacctgaa ccacttccgg gggtcctgac 1740
   atgccgactc tcaaggaagc gagcgtgaat gctcagagac cacgcggagg cgtccaagcg 1800
   tggagcgtag gggacaccta cccggtcact gtagtgggcc tgggcaatgg cccccgcgtg 1860
   caatggtacg cggagaacct gcacacgggc gaacgtggcc ccgtgcgaga tgcccagggt 1920
   gatgcagtgg tggaccagta tcgtctttgg gcggagttca acagaaatcg cctacaggcg 1980
   taattcggtg gccctgttca tgtgtcgtga acagggctcc aggagtgaac gcattcaatc 2040
   gtgagtgcgg tcatcactgg agaatgcaac atgcaaacga aagaacagcg catcgaacta 2100
   atcgccgcga tgtttggtga gcaagaaacg ggcctgatcg gtaagcaact ccgcgtgctg 2160
   gataactccc aaagcggggc gttctacaat gttggtgatg tcggtaccgt agtcctcgtg 2220
   gacgatgacg gtgaaatctg ggtggacttt ggcccggatg gcttcaaagg cgatggtacg 2280
   gcatacccgg tctgggccgc tggttcgctg ggcgcagacg accatgagtt tctggaaaac 2340
   tgacatgggc gtcatctggc acgaactcat ctacgccctg ggagccctcg tggttgtcgg 2400
   ggtcctcatt ctgatcctca ccgagggaga ctgacatcat gcgcaccttt gcaatcgact 2460
   tcatgctcaa cggcaagcgc gttgggcgtg actacgtgac ggcttccaac gagaagcaag 2520
   ccaccatcat cgcagaacgc actgcacccg tgacgctgta tgacgaggtt gtggccgcac 2580
   cgctgtgatg gaccatcggg ctcccttgtg gagcccccag gagtggaccc tttcaattcc 2640
   gagagtgtcc atcactggag agaatcatgt cggacaaagc caagcaatcc atcgagttcg 2700
   ttcgcaacgg cctgggcgag gaaaacttca acaagctcct gagcatcacg ggagtacgtg 2760
   acatcgaact ggctgccgcg ttcctggcga ccaccaagga ggagcgcgac tctgtgaaga 2820
   caggtgacga cctcatgcgc ctgctgggcc gcaagcacgc tgagaaccgc gtggccatgg 2880
   ctctggtgcg cgcgggtgtg ccggtggagg atgccgtgtc tttcgtgcgt gaaaccgctg 2940
   ctagcctgta agccccaagg tgccccttag ggggcctcta ggagtgagcc gctggaatcg 3000
   tccgaagtct catcactgga gatcgctatg tctgcacaag ccaaacaaac ccaaaccgcc 3060
   ccgaccatca tcgccctgct gtctgctgcg aatatggctc agacgggccc cggcgtcttc 3120
   gctggcgtca tcaaccaagc cacacctgag gagcgcgcgg gtgtgaagaa catgaaggac 3180
   ctcctggcgc tgtacttcaa ggttcatgcg cgagtggtgg ccgaaatctc cgcggaagtg 3240
   gaagccacca cggaccatcg ggctcctctg gtggacctgt ccgacttcgc tgagaccctg 3300
   gcggagtact tcagccgtgc cgatgaagtg gtgccggaag gcgtcacgct gcaataacgc 3360
   tgggtgcccc gaaaggggct ccaggagtgg atgtcttcat tgtgaggacc tccatcactg 3420
   gagaaagcaa tggcacagat gcgcgcctgg gtctacaagg cgcactggag gcggcacctg 3480
   gccgcgcaag gcatcgtcct gcgcaaatac gaggtggaca aggagtacat gcaccgcggc 3540
   atgacgcagg ccatcttccg ccgcaacaaa gcgaagttgg tggccgagta cacggagttc 3600
   tgacatggac atcgtagacg aactggagat aggaccctct tacgccctga actcggacga 3660
   gaagtggctc cgcaagagag ccgctgagga aatccgcagg ctccgaaagc aactggcgga 3720
   cgctggttgg gctctcgaag cggcccgtga actcgaagac caacgagaca acgggggctg 3780
   gctatgaaac ccgctgacgg tcaacccaag cgcttcaagc tgcacaccaa gtatccccac 3840
   aacaggtccg agggtttgac tcatcggacc aacaagggga ccgcgcttca agttctaccg 3900
   aagaggtaac gccatgaaga tcactctgac actggaggac accgctgatg gtgtcgctgt 3960
   gaactggacc gaggagcaat ctgaagctca gaacaaaccc agcgagagcc tggccaccat 4020
   catcgctgcc aagttcattc ttgagataaa tcaatctcac cgtatgggaa ttttacggct 4080
   gtccggcact gcattgggcg cagatcgcgc atagctagta tgaggtgtgt tgcgtagagt 4140
   gcgaaccagt tttatttggt tcgccatgcc cgcatccaga agctcatcgc aacagtagag 4200
   gagtagcaat gccggtcatc aaacgcggga acaagtacca ggccagtgtg ggctctggta 4260
   ctgatcgctg gcgcaagatg ttcgacaccc aggaggaggc ggagaccgca gaactggcag 4320
   agaagctgcg caggaaggcc gctgggaagg acgagaaggg ggctacaagc tccgcaaatg 4380
   gggcgaaggt acagaagacc ctaaaggagg cttacgaccg caccttggcc ctgatttgga 4440
   agggcaccgc tgcggagaag acccacatca tcaactcgaa ctccgtgatg gcggagttgg 4500
   gcaaggacac gctcctgtcc gacatcgcca ccgaggacgt aacggagatg atcctggctc 4560
   tggaggagaa gggcaactca ggcagcacgg tgaacaagaa gctgtcctgc ctgtccatga 4620
   tcctcaagac cgcctcggat gagtggcctg ggtgcatcgt ggagatgccc aagctgaagc 4680
   ggcgcaagga ggggtctcac cggctccggt ggatcaacga ggccgaggag aagcggatgc 4740
   tggaggccgc ggagcacctg gggctctacg acctccggga ctacatcatc gttggcatcg 4800
   acaccgggtt ccgccgcgga gaactcctcg ggttccccct gaaggactac cagggcggtc 4860
   tcatgatcct ccacgatggt gagaccaaga gcggcaaggg gcgcgccatc ccggtcacca 4920
   agcgggtcca cgagatcatc cagcggagga gcaactactc gtacctcttc caggactaca 4980
   cggtccacaa gctgcgttgg cagttcgacc aactgaagct ccacatgggg ctccaggagg 5040
   acacgcagtt cgtggtccac accctgcggc acacctgtgc cagccggatg gttcaacgtg 5100
   gggtgcccct gaaggtggtc caggagtgga tgggtcacgc caccatcgcc acgaccatgc 5160
   gctacgcgaa gctagctccg agcagcctgc tgatggcgaa gaaggccctg gaggaagaac 5220
   cccaggaact cacattcatt cctcccccgc agatggatgt ggtggggctt cacgacttct 5280
   aaggaaagga attggaacac ctcagagaga cgttcaaggg aaaggtacag agacaggcag 5340
   gacgagatgg gtgctgggtt tggcaaggct ctaagacgga caggggatat gggaacctgt 5400
   gggacccaaa aaccaagaag cctgtctcag cacatcgact gtcctaccaa ctccacaagg 5460
   gacaaatccc ggaggggttg atggttctcc accggtgcga taacagggct tgtgtgaacc 5520
   caaagcacct gtttgtgggg accgcccagg acaatacggt tgacatgtac ctgaagggta 5580
   gaggaacagt tccgcattag gttccacata aggataaccc tgaagggaaa cctaatgtgt 5640
   aaatcctaag tgtttatctt catagataga cactattaat gatatctact tagagagaac 5700
   actttagttg acactatgac tacccaacaa gtggacaacg agaacgaaga cctggtgact 5760
   attcagcttc gtctcgaaga agagatgacc cagcggggag cagaccggta catccggggg 5820
   gtatccaagg ccatcgagaa gggccgtgag gatgacaccg cctacggcaa gcaaatcctg 5880
   gccgggaggt tggcgaagct ggcccaggcc atcgctgagt ggaaggcgga ggtggcctct 5940
   ggtaagcctg gccggaagca ctcggcctgg aagctcatca aggacacgga cgacaacatc 6000
   ctcgccttcc tggccctcaa gcacgttctc tcgggggtct ccgcagtccg caccgtccag 6060
   tacgtggccg tggccatcgg caccgcggtg gaggacgaga tgcggttcgc caaggtccgt 6120
   gaggcggagc ggaagaagtt tgagcagcta gtcaccgggg cagcgaagcg gaccagccag 6180
   cactacaagc acgtctacgc cacccgcgtg gctgaggacg tgacggagtg ggacaagtgg 6240
   tcccggactg accgcctcca cgtgggggtc aagctcctgg acctcctgat gcagtccatc 6300
   ggcctggtgg aggtgtccac gaacctggac aacagcgagc aggggctcaa gtacgtgaag 6360
   gccctcccgg agaccctgga gtggatcgaa cggaagaacg aggtgaccgc cctgctgcgc 6420
   ccggtctatg agccgatggt ggttcagccg cgggattgga ccaacccgtt cgatggcggc 6480
   tacctgtcct cgaacatcaa gccgctgaag ctggtgaaga cgaagaacaa ggcgtacctg 6540
   gaggaactcc gcggcgctga catgcccatc gtctacgagg cagtgaacgc catccagcgc 6600
   acggcctggc agatcaactc ccaggttctc acggtgatgc ggcacctgtg ggactcaggc 6660
   tccgagcttg gtggtcttcc ccctcgggag ggactgccga tgccaccgaa gccctacgac 6720
   atcgacacca acgatgactc gaagaaggcg taccgcatcg ccgcagcgaa ggtccacatg 6780
   gagaacctct ccattctggg ccagcgcatc ggctttgaca tggccctggg cattgcgggc 6840
   cgctacgaga agtaccggcg catctacttc ccgtaccagt tggacttccg ggggcgcatc 6900
   tacgcggtcc cgcacctgaa cccgcagggg tccgactacc agaaggctct cctcagattc 6960
   gccaacggga aaccgctggg ctccgagggg tggaagtggt tggccatcca cggtgcgaac 7020
   ctggcgggct atgacaaggt gagtttggag gaccgcgtgg agtgggtcct ggagaacgaa 7080
   gatgagattc tcagaatcgc aagtgatccc tacgaccatc gtggttgggc atcggaagtg 7140
   gggggggtta agatcgacaa gccctggcag tttcttgcct tctgctttga gtgggctggg 7200
   ttcgttgagc atggtgagtc gttcgtatca aagctgcccg tggctatgga cggttcatgc 7260
   tctggcatcc agcacttcag cgcgatgctc cgggacgaac gaggcggggc cgcagtcaac 7320
   ctcgtacccc aggacctccc agccgatgtc tatagagccg tcgctgagag agtcattgaa 7380
   caggctgaaa gtgatctcgc tcacggttcc gaggacgaac tgaagcacaa cggccggggc 7440
   atcgcttacc tgtctgaggg ctccaagacc atcgcccagc agtggatcaa gttcggcatc 7500
   acccgcaagg tcaccaagcg gagcgtgatg acgctggcct acggctccaa ggagtacggc 7560
   ttcaaggagc aactcatgga ggacatcctg tggccagcga agagggcagc gatgcggcct 7620
   gatgggtcca tcgacacgga gaagttcccg ttcagcgggg atggctaccg tgcggctctc 7680
   tggatggcga aggcaatctg gaacgcggtg aacgcagtgc tggtgaaagc tggcgaggcg 7740
   atgcgctggc tccaggaggt ggcagcactg gccgcgaagg aggaactgcc tgtccgctgg 7800
   acaaccccgg tggggttccc ggtgatgcag gcgtatccgg ccctggaggc acgtagggtg 7860
   aagaccgcca tcaacggcat ggtgctgaag ctcctcatga accaggagaa ggactccctg 7920
   gacaagcgga agcaggggca gggcatctcg cccaacttcg tccactcctg cgatgcggcg 7980
   cacctgatgc tcacggtggt ccgcgcgaag caggaaggta tccagaactt cgccatgatc 8040
   cacgactcct tcgggaccac cgcgggtgac gtggaggaga tgtatcgggt ggtccgcggg 8100
   agcttcgtgg agatgtactc cgaggtgcgc gtcctggaag acttccggga tgagatcgcg 8160
   gagcaacttt ccgagaaggc ccaagcgaag atgccgccgc tacccgagcg cggtctcctg 8220
   gagttgtctc gcgtctgcga gagccgctat tgctttgcct gaacccttcc acatctggaa 8280
   gagttgagcc gggggaacga ttaggtgcca cacatggata aaccagccgc cgttcccccg 8340
   gtggcctctc ccgagacaac cgatggaacg caacgaacac gaagtatcgg accagtacga 8400
   gtccgcactt ggccgcgcga ttgctcagtg gcgcaccgga cggcccatcc cgatgacact 8460
   cgccgctgaa ctgatgcaac agggctatga cgtatccgcc ctggaagcgc gtcacatgac 8520
   ctgaaccaac aatggcagaa aagaaacaac gcaacccgag cttcacctcg ccgcgcggca 8580
   tcgcccgcta cccggccctc aacaagcccg actacggcaa cgaacagttc ccgaagccgg 8640
   atggtgagta caaggtccaa ctcatcctga gcgaggccga ggcccagccg ctcatcgaga 8700
   agctccagcc gctctatgac gcggccatcg aggaaggcaa ggcgaagttc aaggaactga 8760
   aggtggagca gcgcaagaag ctgggcgcgc tgaaggagaa tgacctctac gccaccgagt 8820
   acgaccagga gaccgaggag ccgaccggca acctcatctt caagttcacg atgcaggccg 8880
   gcggcaagaa caagaagggt gagccgtggt ctcgcaagcc cgcgctgttc gacgcgaagg 8940
   gcaagccgct gccgaagaat gcaccggcca tctggggcgg ttcggaagtc aaggtctcgt 9000
   tcgaggccgc tccgtacttc atccccggca cgggtgctgc tggtctgaag ctgcgtctcc 9060
   aggcagcgca ggtgctcgaa ctggtgactg gtggccagcg cagtgccgat gcctacggct 9120
   tcggtgccga agacggctac gaggcagacg acaacaatga agagggcgat gaagccccgg 9180
   acactgatgg caagagcggc agcggcgaag acgaattcta aatcactgac tgccaaacag 9240
   gtggccctga agtacggctt caggagcggc ctggaagaga agatcgccgc ggacctcacc 9300
   tcgaaagggg cggggttcac gtatgaggag ctaaccatcc cttacgtgaa gcccgcgaag 9360
   ccctcaaagt acacaccgga cttcgacctt ctcaagaacg gcatcatcgt ggagtccaag 9420
   gggcggttcc taacagagga ccgggccaag cacctgctgg tgaaagccca gcacccagac 9480
   ctggacattc gtttcgtttt ctcgaattca aaggcaaaga tcaacaagcg aagcccgacc 9540
   acctatgcga tgtggtgcga gaaaaacggc ttcgcatatg cggacaagag cgtgcccgag 9600
   gcatggctca aagagccgcc gaacctgaag tccctagcag ccatcgagag gctgcgggga 9660
   gcatgacatg gcatacactt ccaacaccaa gaagcgggca agcacggact acctggtggt 9720
   ccattgctcc gcaacgaagc cctccgctga catcggagcc gcggacatcg accgctggca 9780
   ccggaagcag gggtggcgct gcatcggcta ccacttcgtc atccgccgtg atggcaccat 9840
   cgaagaaggc cgttacgctg acgttatcgg cgcacacgta gaaggccaca acgagaactc 9900
   cctgggcatc tgcctggcgg gtggtgtctc cgagaaggat gtgaacgttg ccgagaacaa 9960
   cttcacgccc gagcagttcg ccagcttaca gaagctcctg acggacctcc gagcgaagta 10020
   tcccaaggcc accatccagg gtcaccgcga tttccctggt gtggcgaagt cgtgcccctc 10080
   cttcagtgcg aaggattggg ccaagcaaaa cggtttctga cacaccacga ggagcaacca 10140
   tgaaggcatg gcgtaaagaa cccaatcagg gcgcagtccg tattggtcgc aagaccatca 10200
   acgcgaagcg tgtgatgaac aagttcaaac cgagcatggt caaccatggc tccgtcctgt 10260
   ttcagcggat gatgctccag gccggtatct gggcgctcta acctaaacca tctccagtgg 10320
   tacttcgggc cggtccttcg ggctggcccc ccttttatgc tcaagatttg taagaggtgc 10380
   ggtgaatgca agccgtttag cgactttcac aaagcacccg caggaaaatt caagctccag 10440
   tcatattgca agcagtgcaa gaaggaatac acgcgggaca ctggagctaa catcctaccc 10500
   tccattcgtc agagagcacg aaagcaggga gtccccttct cgcttaccaa agagaacccc 10560
   ccacccatcc ccgaagtgtg cccggtctta gggattcccc ttcgacggac actcggcttt 10620
   gcggacgaca actcgccatc gctggatcga ttgatccctg agcttgggta cgtgcctggg 10680
   aatgttgagt ggatgagcta ccgagctaat cgaatcaaga acgactcaac ctatgaagaa 10740
   ctcgaaaggg tcactgcctg ggtccgagag cgagtttcta cgacacatcc catgtgaggg 10800
   ctgcggttcc tcagacggga acagtctctt cagtgatggg caccagtggt gcttcgtctg 10860
   tgaaacctac gtgcccggtg atggcagcga accaacaata ggaacaacga agaagcggat 10920
   ggaagggctg ctaaccgggg agtttcgccc cctactgaaa cggaagatca ccgaggagac 10980
   ggcgcgcaag ttctcgtatc aagtcggtga gttcaaggga aagacggtgc aactcgcgcc 11040
   gtactttgac aatgcaggtg tgatggtggc tcagaaggtc cgattcccgg acaaggagtt 11100
   caccgtagtt ggggatggca aggccatctc tggaatcctc tttggccaga acctatgggc 11160
   tcctggcggg aagaagatcg tggtcaccga aggcgagatc gatgccatgt cggtgagcca 11220
   agcgcagggc aacaaatggc ctgtggtctc cgtaccaaac ggagcacaag gcgcgaagaa 11280
   gtcgcttcag aaggcactcg aatacctgga gagctttgat gaagtgattt tgatgttcga 11340
   ttccgatgat gcaggcaaga aggccgctgc tgagtgcgcg gagttgttct cgcccggcaa 11400
   gtgcaagatc gcgtccatcc cgatgaagga cgccaacgaa ttgctgaagg ctggccgtga 11460
   gcaggagatc atcactgcaa tctggcaggc caaggagtac cgccccgatg gcatcatctc 11520
   gggagcggaa ctgtgggagg cggtgtcagc atctcaggat atcgtagagt ccgttccgta 11580
   cccctgggac gcactgaatg aagtcacgaa aggcgcgcgt acaggcgagc ttgtgactct 11640
   cactgcgggt tccggcatcg gcaaatctgc cgtggtacgc gagatcgctc accacctcct 11700
   gaggcgtgga gagacggttg gcatgttgat gctcgaagag aacccgaagc gcaccgcgct 11760
   gggtctcatt agcatctccc tcaacaggcc tctccacata gaccgtgaag gtgtcagcaa 11820
   ggatcaactg aaggtagctt tcgatgatac ggtaggctct ggccgactat tcctctacga 11880
   ccacttcggc tccagcgaca tcgacaacct ggtgtcccgt gtccgcttca tggcgaaggg 11940
   cctggggtgc aagtgggtca tcctcgacca cctgagcatt gttgtctctg gcctcggtga 12000
   cggagacgaa cggcgactca tcgacaacgc aatgacgatg ctgcgtaccc tcgtggagga 12060
   gaccggcatc ggcatgtttg tggtgtcaca cctccgccga ccggagggtg accgcggcca 12120
   cgaacaggga gcacgtacct cgctcaccca actccgcggt tcccatagca tcgcgcaact 12180
   gtcggacatg gtgattggtc tcgaacggaa ccagcagggt gagaacccga acgtcaccac 12240
   gctccgtgtg ctgaagaacc gcttctccgg tgagaccggt gaggccgggt tcctgctgta 12300
   cgaccgggag accggacgcc tggaagagac ggacgcacct gctgcgccct tcaaagacga 12360
   aaccaaatcg gacgttcagt ccgagttcta accaaaggtt acatcatgag tctgatttcg 12420
   ctgttcacgc agtccgctgc tgaccaacgt gctgccgcgc cccgtgctgc ccgtgtccgc 12480
   gccaagatcg cggacctgat cgactaagcg ggagtctctg tgatcgatga cacccgcctc 12540
   caagagttcc gagaaatcct cgatgtagtc cgctgggagt tccccggttc acaccccgtg 12600
   attgggggcg gggctctccg cgattcctac catggtcgcc caatcaagga cgtggacgtg 12660
   ttcatgcgca ggcgtgacca cgagacgctg aactcggaac tcacccgctt catccgcccg 12720
   ccgatcctcg tggcccacgg ctatggccgt cccgacatgc acggcgcatg ggacctgatg 12780
   cagtccgttg ctggctacga ggtgcaactc atcctcgcgg acttcgagaa cctggaagac 12840
   ctggccggta cgttcgacct ggggattgcc cgagccacct tcgatggtga ccggctgttc 12900
   ctccatccgg acttcctcca ggactccacg gataaggtct tccgcatccg tcgcgcggac 12960
   aacctgttcg agaaggcgcg aagcctgaag cgcatcaagc ggctggcaga gaagtacccg 13020
   gacttttcaa caccggactt cgagcattgc cctgtctgcg cacaacccat catcgagttc 13080
   cgcaacgctg ccagcgtccg agagcaccaa atctccgggc tctgccagca atgccagtac 13140
   ttggtgttcg acaaggactg accatgaaca ccttcctcat tctcctggtc ctcatcggag 13200
   gccaaatcga aggccgcgtg atcgctgagt tcgacactcc ccgtgagtgc gaagcagcga 13260
   aggaacacgt gagggtcatc aaccaacccc ctgtcgtcgc gtccacgttg gtgtgcgcaa 13320
   gggatggccg cgcgtaatca ccaaggacgg tatgaagcta ttcgacattg aaacaaacgg 13380
   tctgctggat accgtcacca aggttcactg tctcgtcatc aaggatcgca ccaccgggag 13440
   gaagttccgc tgcatccccg caggcttccc gatgcaagcg gacatgacca tcgagcaagg 13500
   gctggagctt ctcaagtccg gccccatcgg tggccacgga atcctcaggt acgacatccc 13560
   ggtcctggag aagctgtacc cggacttcac ctacgacaag gaccaggtgt tcgacaccct 13620
   ggtggccgcg cgtctcatct ggacgcacat caaggacatc gacaacgggc tcctcaaaaa 13680
   gaagcaaatc cccggctccc tctacggctc ccactcgctg gaagcctggg gttaccgcct 13740
   gaagctccag aagggcgagt acgcggctga gttcaaggcg cgcgtggggg acgcttacga 13800
   ggggggcatg gagtggcgag agctttctcc tgagatgctc gactactgcg acctggacgt 13860
   ggatgtcacg gacgcactgt tcgaccggat cgaaggcaag aactactccg cggaggcgct 13920
   ggagcttgag caccgcatcg cctggctgat ggctcaacag gaacgcaatg ggttcccgtt 13980
   tgacgtgacg aaggccagcg cgttgtacgc caagctcgcg caacgccggg gcgaactgga 14040
   gcgagaactg aaagagttct tccgtttctg gttcgctccg gctggaacag tgactccgaa 14100
   ggttggaaac aaggcgcgag gaactgtagc cggtgtcccg tacaccaagg tgaagatcgt 14160
   ggagttcaac cccggctccc gcgaccacat cgctaatcgc cttgtcacgc tctacggctg 14220
   gaaaccggag gtgttcaccg atggcggtaa gcctcgggtt gatgaagatg tgatggcacg 14280
   cctggactac ccgcccacga aactcctcac ggaatacctg ctggtctcca agagaatctc 14340
   tcagctagct gaaggtgacc aagcgtggct caaggttgta cgtgacggaa agattcatgg 14400
   ctccgtgaat ccgaatggcg cggttacagg aagatgcacg cacgctttcc cgaacgtggc 14460
   ccaggtgcca gccgtaggtt ccccctatgg tgaggagtgc cggggattgt tcggggcacc 14520
   taagggttgg ctgctggttg gctccgatgc ttccgggttg gagcttcgct gtctagccca 14580
   cttcatggcc aggcacgatg gcggcaagta tggaaaggtg atccttgagg gagacatcca 14640
   cacggagaat cagaaggccg ctggactgcc cacacgaaac aacgcgaaga ccttcatcta 14700
   cgcgttcctc tacggagccg gggacgccaa gattggtaag atcgttggta aggacgctgc 14760
   tgaaggaaag aagctcaagg ccgcgttcct gaagaagacc cccgcactca agaagctcct 14820
   cgaagctgtc cgtgagtctg ccaagcgcgg ctacctggtt ggcctcgaca agcggcaact 14880
   ccatgtccgc tctcagcacg ccgcattgaa caccctgctg caatccgcag gtgccctcat 14940
   ctgcaagtat tgggttgtcc gcacggcaga gcgaatggaa gctctgggct acaagcacgg 15000
   atgggatggg gacttcgcgt tcgtcgccta tatccacgat gagcagcagg ttgcagtacg 15060
   aaatgaggaa gtcgccaagg tcctcgttga gcaggttgca ttggccatga aggacgccga 15120
   agcgtgggcc ggattccggt gcccgctggc ctgtgagtcc aaggtcggta cggattgggc 15180
   ttcaacacac taaagtaatc agacaccaac atgagcatgt tccgagacga cctactcaaa 15240
   gaagtcctct acgaggcgtt caagactccc ttcaagctcc agtccgactt cgcccgagag 15300
   ttcgctcagg aagtcgccgc tctggcctcg atgggataca tctcgaccta cgaggggccg 15360
   cagcagttcg gcaagaagtg gcgcgtcacc ggcatcggcc tggacaagct gcgcaagctg 15420
   gggatgctgt gagtgaagcc ctacgccccc attcgctgag gatcatgggc cggaagttcc 15480
   gggtctctta caaggatgac ctggacggtg acctgggata ctgcgaaccc accaagtgta 15540
   agatcgagat tgagaacggg cagcaccccg tggaggaggc cgatacggtc ctccatgagg 15600
   tgcttcacgc ggtgttctat ctgatggaca ttgggctctc cgcggaggag gaggagcacg 15660
   tggtccgtaa ggttgtcacc ggactcaccc aggtattcca ggacaacccc cggctcctga 15720
   cctacttggc aaacgccaag tgatggacca tatagccaag tttgattctc tccaggagga 15780
   actcatgacg gacaagaagt ggaccatcac ggttaacgtg gacacccccg agggccaccg 15840
   ggagcggacc atcgagttcc cccaccggcc caccgaggag gagcttggtc tcaagctggc 15900
   gcagttcttc agccggatga acttccgatt caacgaacac ctgaaggagg tgaaggggtg 15960
   tgcgctcctg acacctcgga gaccgtatga aagtagcgct gattgatgct gacgttctgg 16020
   tcttccaggc ggctgtagtc gctgagaagg caaccgattg gggggacggt gtttggaccc 16080
   tccacgcaga cgagggtgac ggagaacgaa tcgttcgcca gtccgtcatc accctccagg 16140
   agaagaccgg tgcggataag gtcatcctgg cattctccga tgaggagaac tggcgcaagg 16200
   ccatactgcc cacctacaag gccaaccgag cgggttcccg ccagccgatc atccgcgcgc 16260
   atctgaagcg gtgggcttcc gacgaatacg agagcttcac ccggccaacc ctcgaagggg 16320
   atgacgtgct gggcatcctg gccacccgcg agggcaagcc aggcgagaac ttcatcgtgt 16380
   gctccatcga caaggacatg cgaaccatcc ctggcaccca cttcaacttc ggcaagaacg 16440
   aagagttcgt ggtgacggag gagggggcag actactggca tctcttccag accctcacgg 16500
   gtgacccggt ggatggctac gcaggctgtc ccggcattgg cccggtggcc gcgaagaaga 16560
   ttctcgacaa gagccccacc tggggtgccg tggtctctgc ctacgacaag gcaggcttcg 16620
   gtgaagagga agctctcgtg caggcccgag tggcgcgcat ctgccgcgct gaagactacg 16680
   acttcaagaa gaaacaagtt cgactgtgga ccccaaagaa atcctgaaag aactggaaca 16740
   gcagcaacgc cgcaagttcg agaaaggccc tctcaccggc aaacgcgccg atgtcatcat 16800
   catggacgac atccaggaca ccaaggacac caacccgaag gacgccatcg gctccaccaa 16860
   gctccccctc gacctcgttc ctgactcgct ctcggtcttc gccgcgctgg cgttcaccga 16920
   gggtgccacc aagtacggtg cctacaactg gcgtgtcgct ggtgtccgtg cgtccatcta 16980
   caaggccgcg ctggagcgtc acctgaagaa gtggtggaac ggtgagtggg ccgacccgaa 17040
   gacgaaggtg ccgcacctgg ccagcgtcat cgcgtgtgct gcgatcatcc tggacgcgga 17100
   cctcgcaggc aagttgacgg atgaccgccc tccggcaatc gacctgagtt ccttcatcga 17160
   ctcccttgag gagaccgtga agcacctcaa ggaactgcac aaggacaaga acccgaagca 17220
   ctacaccgaa ctcaacgtat gaacccgaag cgaaacactc tgaccggctg ggtcatctat 17280
   gatgcagagc gggcgactgg ccgaagcacc gcgattgcgc tgagtcttct aggcaaggcc 17340
   attgcaaatc caggtgtggc cgtacaaatc cgagaacatc acggtactcg tccggctgac 17400
   gagagtctga tgcgcctgat gcgggatatg gtctttcggc tgggcctcaa gggcatgacg 17460
   ttcagccaga acctgactgt gacgttcaac ctttgggagc ctgtgtgagc cagagccgaa 17520
   agggctctct cattgaggcc ctcatcaaca ccgcaatcgg cttcgggatc aacttcacgg 17580
   cgaacctcat catcctccca ctgttcggct tcaccagttt gacggtgcag acgaacctgg 17640
   tgattggcgt ggtctacacg ctcatctccg tggtgcggag ttacgtggtt cgccgctggt 17700
   tcaacgcaca catcgtccga gccgccaaga aactctcagg ggcctgaagg tctctttagg 17760
   ttccacaata ggagaatcaa attggcgaac gacaagtttc cgccgattcc caaagaatta 17820
   cttgaggcgc ttgagaagcg gttcccggag acaccactcg aaaatatcgg gtctgtggat 17880
   caacttcgat tggctcaggg tgagctacgt gttgtccggt ttctccgagc ccaattcgag 17940
   aagcagacca agaacatttt ggagaacaca tagtgtgcat gtctcaaccg tccgccccac 18000
   ctccggcccc accgccaccg ccacctccgc ccccgcccgt tgatccgatt ccggtccaac 18060
   ctgcgcagca aaccggtgga gcggtgacca gcggcaagag caagggacgc gactccctcc 18120
   gtatcgacct ggcccagaag acatcgggtg gtggcgccgg tctgaacatc ccgatgtaac 18180
   gaagggcagg gatggaacaa gaaaagaaaa cctgcgcctc cctctaccag aaactcacca 18240
   ccgaccgaga cccgttcctg aagcgggcct acgactgcgc cgaactgacg attccctcct 18300
   tgcttcctcg tgagggacac aacggctcca ccaaactcgt cactccgtgg cagggcattg 18360
   gtgctcgtgg ggtgaacaac ctcgcatcca aactcctgct gacgcagctt cctcccggaa 18420
   ctcctccgtt caagttgtcg attgacgact tcacgctgga ggaactgacg aagcaggaag 18480
   ggatgcgggc gaaggtagag gaggggctca acaagatcga acgcgcggtt cagactgaga 18540
   tcgaagcgaa ctacatccgc gtggctgcct tcgaggcgct gaagcatctc atcgttagtg 18600
   gcaatgccct gctgtacatt ccgcctgaag gtggactgag agtattccac ctggaccgct 18660
   acgttgtccg ccgtgacccg atgggcaacg tgctggacat catcaccaag gagaacgtct 18720
   cccgagacgc actccccgac aacctcgtcc tccctgatga caccgaggag aaccaggagc 18780
   ccgcggctgg tacgaaggat gtggagcttt acacccacgt ctatcgccag ggccgcaggt 18840
   ggaaggtcta ccaggaagtc aagggtgtcc gcattcccgg caccgagggt tcgtacccgc 18900
   tcgataagag cccgtggatt cccgttcgct tcacgcagat cgacggtgag agctacggac 18960
   gcggttacgt ggaggagtac atcggggacc tgaagagtct cgaaggactc tcccaggcca 19020
   tcgttgaggg ctccgctgcc gcagcgaaga tcctgttcct ggtgaacccg aatggcacca 19080
   cggacatggc tgacgtgtcc gaggctgaga acggtgcgtt ccgcgagggt gtcgcaactg 19140
   acatcacggt cctccagctt cagaagcaca atgacttccg cgttgctctg gagaccatga 19200
   aggacatcac cgagcgcctg gcgtttgcat tcctgctgaa ctccgcagtg cagcgcaacg 19260
   gcgaacgggt gaccgcagaa gaagtccgct acatggcgaa cgagttggag tctgcgctgg 19320
   gtggtatcta ctccatcctc tcgcaagagt tccaactgcc gctcatcaag cggatcatgt 19380
   accagatgga acggcagaag cgtctgcccg tccttcccga agggaccgtc aagccaatca 19440
   tcgtgactgg catcgaggcc ctcggacgtg gaaacgacct gaacaagctg atccagttcg 19500
   tccagatcgc cgcacaggca gcgaatcttc ctcccgagat cgacaaggcc gacttcctca 19560
   agcgtgctgg tacggcgctg gggatcgaca tgaagggtct cgtgttgccg cctgaggtgg 19620
   tagctcagaa caaccagcag gccatgatga tgcagatgat gcagcagggt gtgaaccccg 19680
   ccatcacgca ggctggacag ctaatgaaac aaggaatgca gaatgccgcg caacccgcag 19740
   gcgggcagta aggctcccga ggccaacact gccgaagccc ccgtggtcac cgttgaagac 19800
   tcggtggccg aacagcaacc caagcccgca gcgaagccgg tcaaagtgac cgaactacct 19860
   ggtggcgtga agatcgaaga cttctgatga gtgtggattc cgtagtcatc aagcagccgg 19920
   acgctccggt ggaagaccag gcccacatcg atgcgatggt ggccaaggtg gatgctgcca 19980
   atacttcgac cgaaccggac actcccgagg tgcccgcaga gggacgcccg cagtggctcc 20040
   cggagaagtt caagtctccc gaggacttgg ccaaggcata tgccgaactg gaaggcaagc 20100
   tgggtgggaa gaaggatgat gccactccac ccgctgacga caaggccgcg aagtctgacg 20160
   aaaccccgga cccaagcaag gccacccagg acgatgcctc gaaggctctc tctgagaagg 20220
   gcctgagctt cgatgagttc tccgctgagt ttgcccagaa gggtgaactg accgccgaga 20280
   gctacgagaa gctggagaag gctggcatcc cgaaggccgt ggtggaccag tacatcgctg 20340
   gccagcaggc cctcgctgag tcgtaccgca aggacgtgac ctcggttgcc ggtggcgatg 20400
   aaagcttcgc tgagatggtc acatgggccg ctgcgaacct ctcgaaggaa gagatcgccg 20460
   cgtacaacaa ggccgtggac tccggtgaca tcaaccaggc gaagctggtc gtggccggtg 20520
   tgtaccagaa gttcgacgct gctggccgcg gtggtgagcc tgccctggtg actggcgctg 20580
   gcggtaaggt ctcgggcgat gtctatgagt ccctggctca gatgcagaag gacatggcct 20640
   cgccggagta caagaccgac cccgcattcc gcaagaaggt ggagcagaag atcgcccgct 20700
   cgaacatctt gtaaggaacc atcatgatcc tggagagcat cctgggttcg gtggtggtcc 20760
   ccgctatcat cgacctcgtg aagggtgctg gtggggccat tagccgcaag ttctttggtc 20820
   tgtcggttga cgaccagatc aagattcaaa atgccgacat cgagaagctc aaggctctcg 20880
   ctgccctcga caatccgtat ggcaccccca gccagtgggt ggtggacctc cgcgcatcgt 20940
   tccgatacat cggcgctgcc gcggtcatcg ctgtcggctg tgtcaccctg tatgccggtg 21000
   tccagaccaa catcgaagac gtgaaggaga tgggtttcgc cctcgtgggc atgcccttcg 21060
   gtttcatctt cggtgaacgc ctgtacctcg gcctgagggg caagagcaag taagcactgc 21120
   cgggaagcag cgcattctgt acggttctga tcccgtaacc gttacccacg ggatcacact 21180
   gccattgaag tgaaaggtcc tagccgcact gcgctcctgc gcggtggctc tgctgcatcc 21240
   aaagaacacc acaacagaac cttggcccgc tgaggcggac aaccctgtgt gacgtgtgag 21300
   ttcccggaag ccgctcaaca cgactttcaa ctcacttcca aaacaaaaat ggcaaacgca 21360
   gttccgtctc gcctgggcca ggcaaacctg gcaggcgatc cgaaggccct gttcctgaag 21420
   gtcttcgctg gcgaagtcat gacggccttc gctgaaaaca acatcgtact tcagtacgtc 21480
   cgccagcgca cgattagttc tggcaagtcg gcttgaccca accttatgaa ctgggccgac 21540
   tctaaacacc ccgtaaattc ggtggaaccc catgggggca ataccgagcc aagacttcgc 21600
   agtacgcgag atggtgtaga gactagacac ggggaaccca caaagacctg cgcatgttgc 21660
   aacgtcgaga agcccgcccg tgagttctat aaaaaggacg cacagacagg aaggctcgat 21720
   ggaatttgca agtcctgccg aatcatcaag acccgagaga aaaccttagg ggtcactgaa 21780
   gatgactatc ggcggatgta tcatgtccag ggcggtcgat gtggaatctg ccaacggcgc 21840
   ttgtactcaa agaggtacaa gagttttgca gtggaccatg atcacgagac aggaaaagtc 21900
   cgtggcttgt tgtgccataa ttgcaaccgc ggattaggca tgttccgaga cgacccgact 21960
   gcgcttaggc gtgctatcga ctgggttaag gtatagtccg atcctcacag caatgtgagt 22020
   aggggaagca gttccccgta attggtaagg ctaccgccgc gtaccacacg cccggtaacg 22080
   aaatcaacgg cagcaacatc gcccacaacg aagtggtcat caccatcgat gacctgctgc 22140
   tggccaacac cttcatcgcc aacatcgatg aagcgatgaa ccactacgat gttcgttcgg 22200
   tctattcgag cgaactcggc aaggccctgg ccaaccagct tgaccgccac ctgctgcaac 22260
   tggctgtcct ggccgcccgc tctgctgccc gtatcacggg cgaacagggt ggttcggtca 22320
   tcaccgatgc tgctgccggt accgactcga acgcactggt cgcggacatc ttctccgcgg 22380
   ctcagaagct cgatgagaag gatgtcccgg ctgatggccg tgtgtgcttc ctgcttccgg 22440
   cccaatacta cgccctggca cagaacacca agattctgaa caaggattgg ggtggtgccg 22500
   gtgtgtatgc ggatggcaag gtcctccgtg tggccggtgt ggagatcgtg aagacgaacc 22560
   acctgccgaa cacgaacatc gcttcgggtt cgaccgcggc tggtactggc gataagtaca 22620
   ttggcaactt cacgaccacc gttggtgtgg tcacccagaa gtccgccctg ggcaccgtga 22680
   agctcatgga cctggcgatg gagtctgaat accagattca gcgtcagggc accctgatgg 22740
   tcgccaagta cgcaatgggt cacggcgttc tggctccgca agcggctgtc gaaatcaaga 22800
   ccgcataagc gtcccctcaa gcctcgggag gttctcttca agagttcctc ctggggcttt 22860
   tttttctgct ctcaaggatc accaattggc aaccaagact caaactgatc gcgccaagga 22920
   cggtcaggat ttcttccagc ttccggccca caaggacacg cccgcggtca ccgtgaatgg 22980
   caccgcccgt gctcgcacga ctgtcccgag tggcgtccag ctggcaaccc ctgcggctca 23040
   ggatgacatc gtagcgatca cgttcaactc ggctgaccct gggaataccc gccgcgaggt 23100
   cttcaccccg gccactggcg caaccatcac acccaccgag ttctgcatcg aggcccgcat 23160
   tgtacccgca ggcaccattg cggccctcac catcaccttc cccccgaacc cctcgaagga 23220
   aggccagcag ttccgtgctg tcaccacgca gaccatcacc gcggtgacct ggactggtgg 23280
   ctctcgtctc aacgctccca ccacgctagc cgctggccgt gctgccacct tcgagtggag 23340
   cgtggcgaag caggagtggg tcttcatcaa ctaaggaaaa cgcatgacca ccatcgtcac 23400
   tccgaccacg gagcttgagg cggtcaacct gatgctcgat gtcatcgggg agagcccaat 23460
   cagcaccctg gagaacagcg ctgtggtgga cgcggtgaag gccaaggcgg tcctctccga 23520
   ggtgtcccgc gctgtacaaa cgaagggctg gcacttcaac accgagaagg ggttcgagct 23580
   agttcccacg gtcttcgaga aggagatcat cgtccccgcc aactgcctgc gcattgatac 23640
   ggtctacccg gacgagggca tcgatgcagt tcaccgtggc actcgcctct atgaccgccg 23700
   caggcacacc taccagttcg acaagagtgt gaaggtggac atggtggtca acctccaatt 23760
   cgaggaactc ccggaatccg cccgccgcta catcgccatc cgtgccgcac gggtcttcca 23820
   ggcccgcata gtgggctctg agagcctcta ccagttcacc gcagaggacg agagggacgc 23880
   ccgagcggac ctcaagaagg ctgagggcat cacgggggac tacaacattc tgacggacag 23940
   ctgggctgtt cgtcgcgtca tcgatcgctg atatgcccct cgtttcttct tccatcgcca 24000
   acatggtgaa cggggtctct cagcaaccct tcacgctgcg tctcgcgtct caagctgagt 24060
   tgcaggagaa cggcctcagt accgtggctc aggggttgaa gaagaggccc ccaaccaagc 24120
   acatcaaacg cctcggcagt gccatcaccg gctctgccta catccacacc atcaaccgtg 24180
   actctgtgga gcggtatgag gtggtcatca cgaacggtga cctgaaggtc tacgacacgg 24240
   cagggaacca gaagacggtg aacttcccga atgggaaggc gtacctgaac tccacggacc 24300
   ctgctacgtc cttcagggcc gtcactgtgg cggactacac gtttatcgtg aacaagaaga 24360
   ctgtcaccgc ggccagtgcc acgaactccc caacgcggcc cttcgagtcc ctcgcaaacg 24420
   tgaaggttgg gctctactcg aagacctaca ccatcaccgt ctccggtgtg ggcacggcca 24480
   cctatagtac ccccgatggc accgttgcgg cccacgcggc acagatcacc acggactaca 24540
   tcgccaacca gcttgcgaat ggtctcatta ccctcggtgg attcacctca gtgaaccagg 24600
   tgggctccgt catctacatc gcccggccca ccgattacac catctccgca acagatgggt 24660
   ataacaacgc ggccctgaac gtgattaagg ggacggtgca gaggttctcg gaccttcccg 24720
   cgaatgcgaa cttccaggac ttcactgtgg agatcgcagg ggacaacacc tcggagtccg 24780
   ataactattg ggtcaagttt gacaagaccg ggaacaactc cggtgtctgg cgcgagacca 24840
   tcaagccagg catctcggtt ggtcttagtc ccagcacgat gccgtgggta ctggtccgtg 24900
   agtcggacgg cacgttcacc ttcaaaccca tctcctggac gaaccggctg gtgggtgatg 24960
   aagactccgc tccacaccca tcgtttgtgg gccgcaccat ccaggatgtg ttcttctacc 25020
   ggaaccgcct gggcttcatc gcggatgagg ctgtggtgat gtcggaggct ggccagttct 25080
   tcaacttcta cccgaccacg gtgacgcaac tcctggattc cgaccgcatc gacgtatcag 25140
   catcccacac gaaagtctcg aacctgaact tcgcggtggc cttcaacaag gacctcctgc 25200
   tgttctcctc gcagactcag ttctcggtgg aatcaggtga cctcctgaca cccaagagcg 25260
   tctccatcaa gcccaccacg gagttcgagt gcagcaccct tgcgcctccc gttgggattg 25320
   gacgcaacgt ctacttcgcg gtccctaagg gtgagttcga gggcttccgt gagttctacg 25380
   tagcggacaa cgcaggcacc aatgatgcgg ctgagatcac cggccacgtc ccgaagtaca 25440
   tcccgaaggg ggcctacaag atcgctgcgg ctctcaacga ggacttcttc gtggtgctga 25500
   cttcagggga acccaacgcg atgtatgcgt acaagttcta ctggaacagc aacgagaagc 25560
   tccaaagctc ctggtccaag tggaccttcc cgagcacgga cacgattctc cacgcggagt 25620
   tcatccagtc ggaactgttc atcctcatca accggcccga tggtctctac ctggagaagc 25680
   tcagtgtggc tctcggggac atcgggacga acgagcccta caacgtccac ctggaccgca 25740
   agctgacggt gccgaaagca agcctcacgt atgacggcac gtacaccatc atctcctccg 25800
   cggctctccc gtggaaccca acggatggaa cgtacacggc agtggtggcc accagtcagc 25860
   cgcagaaggc tggcgtcctc tacccggtca tttgggatgg gacgaacgcc aagattctcg 25920
   gtaaccgtgt ggactccgac ctcatcgttg gtaggcgcta cgccttccgc tatcgcttct 25980
   cgccgctact ggtccgccag cagtccggcc agggccagaa ggcggacacg gttgcacgtc 26040
   tccagattcg caacatgcaa gtcaacttct cggagagtgg caacttccag gcaaaggtca 26100
   cgccttacgg gcgggacacc tacacgtaca cctactcagg aaagaccctc gggctgcctt 26160
   cggcaaacat cggggccatc ggaattgaag atggcaagtt ccggttcccg gtgatgtcgc 26220
   agaacaccac cgtggacatc gaactcttct cggactcgcc gctcccctgc gccttcttga 26280
   gtgcagattg ggaaggctac tatgtccgac gaagccaggc ggtctaaacc atacgtccgt 26340
   cctgcaacac gcgaagactg catcatcctc gcaaggaacc tccgacagga agacgcggag 26400
   gagatcgctc atgtgaacgg tctccccgcg gagatgaatc tcttgctggg gttccgcacc 26460
   tccgctcgac tttatgcggt ggtgtggggg gatgagaccg tggccgtgtt cggcatcggg 26520
   ggagtgcctg gcgtcatcgg cttcccctgg atgctcgctt cgccctccct ctcgaaaatc 26580
   cgcaagagct tcctgaggga gtgccgcggg tacgtggagg ggatgctcca ggagtatcgc 26640
   cacctggaga actacgtgtg ggcaaagaac gaagtccaca tccagtggct caagtggctg 26700
   gggttcgagt tcgagccagc agcaccattc ggtatcaatg acgaaccctt tcacagattt 26760
   tataggagca tgtgatgtgc ggaccagccg cagttccaat cgccatgctg ggtatcagcg 26820
   ctgtgggcac tgccgcttcg attagcgcgc agtcgcagca gcagaaggca caggatgcct 26880
   tcaaccagcg ccagtatgaa aacgacatga ccgcgtaccg aggcaacctc gccaacatcg 26940
   aggtgcaacg gaaccaggcg cgggaagatg cagtagcgca gaagcagcag aacgacatgg 27000
   caggaaggcg cgcaacagca accgccacga ctgccgcagg tgaggcgggt gtctcaggcg 27060
   cctcggtgga tgcactgctg cgggacctcg ctggccaggc tgcctacgac aacaccaacg 27120
   tggatgagaa ctatctgcgc caggacaggg ctctgaacgc ccagcgtgag aacgccttca 27180
   acagcactgc aagccagatc aaccagcttc gcccctcgat gtccccggac tatctcggcg 27240
   ctggtctccg cattggccag gctgctgcgg gtgcttacag ccagtaccag cagaacctcg 27300
   actacgagcg gaaccagagc gtcccacgcc gaggagcata aatggcacga gttcagacag 27360
   actatcgaac ccgaggtaca gggcttcagg acatctcgtc cccaatgctt cagccgcagc 27420
   aggcagggtt agacaatggt gccgctgagt ctgccgcacg gctggcccag gcgttggggg 27480
   ctgttgacct gtctccgctg gtaaccgcca agcgatacca ggatgtggag gaggcggaga 27540
   aggcacgggc ctacgccaac tccctcaccg tggaggagct tgggaagcag atcaaggatg 27600
   ggaccctcat ggcgtcccat tcgcctgtct tcagggcaac ggtcgaacac atccacggtg 27660
   agaacacgct caacacgttc gagcgggaca cactctcgaa gctcacccgc ggggaactga 27720
   agttcgacac cccgcaggcc atggatgagt acctcacgaa gtaccgcaac gaggccctca 27780
   cgggatccag caagttcacc actgcgggct tcgataaggg ctacggcacg ttccgtgagc 27840
   gagccatcgc ggttaacgtg aaggtggccg atgaagaggc cgtgaagcgc ggcagccagg 27900
   aagcctcgga caacctcggc aacctgaccc tgcaagtcac cgacccgatg tacaagggtg 27960
   acgctgcgca ggccatcgtg gaccgctacc agcttcttcg gaagacctct ctgctgcgtg 28020
   acgatgccgc gaaggaagct ctctcgggtg tcgctgcgaa ccttgcagcc tccggcaaca 28080
   aggccctcct gggttctctg ctggacaaga agttggacag cggtgtctcc gtcaaggccg     28140
   ctctggggga cctgaaggcc atccagttca cgcaacacgc tgaacgtgag tatgaccagg     28200
   cgcagcacca acggattgac gttgagattc gtccgttcgt ggagcaggcc gacaagggtg 28260
   aactgaagcg ggatgccttc gacaagtggg gggccgcgaa tgagaagtac gtcaccaccc 28320
   ccaccatcca cgccatcatc aagggcaacg aagcggccat cgagcggcaa cagaagctca 28380
   tcgctcagaa cgccctcctg gcccaggccg aagcaacaca ggctcaggca acgcaggcag 28440
   cccgcacggc catcgaccag ggcaacctgg cgttcctccc gcagcagaag gtgatgacac 28500
   ctcaggggga acagaagaac ttcgatacga aggccgctgc tgtcccgtac atccaggaac 28560
   ggattgcacg ggagaacatg ccgttcggta agcaggtgga gttctggtcc accaacgggg 28620
   tggagaatcc cgagtgggag aaacagatca agggtggcct ctcgaacctc gcctccgcgg 28680
   gctggacctt cgatggcaag accattggcc aactgaacaa ccagggccag gccgcaatcg 28740
   acaccttcat ccgcatcaac agcaccaacc ccggctacgc tgagaagttg gtgggcggtg 28800
   acaaggacta caagaagctc tccgacatcc agttcctcat ggagaagggc ggcttcccga 28860
   acgtcaacga tgctgcggca ctcatcaacc agattgaccg cgctgacatc aaggcatcgg 28920
   actacggttc gatgaagcag aaggtggcct cctcggtgga cgatgtggtg aaccagcatt 28980
   ggtactcagg cgccaccagt tggttcagtg gcctcttcgg caatgaccag gtgaacctca 29040
   ccgctgtctc cgctgacatt cgccgcaggg ctgaactcct ggtgatgtct ggccaggtgc 29100
   ccgatgcgaa cgccgcggtg aaggccacgg tggaatacct ggcgaacccc gcagtcacca 29160
   cgcggatcaa caatacgctc tacttcaaca aggaccttcc ggtggtcccg aagggcgagg 29220
   acaccgggca gtggatgggg cggttcatca aggacgttcc ccagcagatc gccaaggcga 29280
   acaacctcgg tgatgctcgc ctggagccga accagtacgg aggcttcacg gcctggactg 29340
   gtggtgtccc gatgacggac ggcaccggta aggtggtcac ctacacgcgg gatgacatct 29400
   cgaagtgggt ggacaacacc atcaccgctg accgccacaa ggccgctgct gatgccaact 29460
   tcaagagcta ccaggaccgc ctcgtgaagg aactccgcga tgaaaagcag aaggacccct 29520
   acgtgatgga gcggatgttc gacgcgactg ccaacggcat gtggtggaac cgccaactct 29580
   acagccgcga aggctatgag caggttctcc gtgacggcaa cacaggcaag ccgctcaacg 29640
   aactgttcca aatctacaaa gacaaacgct tcaaggataa gtaatggccg catcgatcgc 29700
   tctgggggat gtccagagga ttacctccga gacggagaag aagtacgggc tccctgaagg 29760
   gacgctgttc aagatcggaa acatcgagtc ctcgttccag gatggccagg tgagcccgaa 29820
   gggagccaag ggctacttcc agttcaccga tgacaccgca aggcgctacg gcctggatga 29880
   tccgttcgac ttcgagaagt catccgatgc cgcgggccgg tacatgcgag acaacctggc 29940
   caagtaccag ggcaacatgg acctgtccct cgcggactac aacggtggcc cgaaggccgc 30000
   taaggctctc gccaagggga agccctgggc agagacttcg gactacctgg cgaagttcta 30060
   cggcaacaag tccgagccgc tctcgcagca attcaccacg ggctccgaag tccctcttac 30120
   tgcctccccc tccgcctccc agctatatcg agacgcacgg cagcaggagt ctgagtatgg 30180
   aggggttggc aataacattc tcaatctgcc tcgtgctatt ggcctgggct ttcaagtcga 30240
   taattcggtc tacaatttct ggcaggagcg aggactctcc agcgtagacc ccgacttccg 30300
   ctgggacgat gacttctcga agcagatgct tgatggggtc cctgagcgtc attggggata 30360
   cctgctgcaa tccaagtcga agcaggaagc ggaactccgc cgtgcccgtc tgttggacac 30420
   gatggagaag gaagtcgaac tctccaagat gggtgtggcc ggtttcggtg gtcgcctggt 30480
   gggcaacctg gtggatctac ctacgctcat ctcgttcgtc cctgggttcg gtggtgcggg 30540
   cctcctcacg accacttcac gcatcgccaa tgctgcccgc atggctgccc tcggtgctgc 30600
   tacgaacgta gccttcgatg ctgcaacgat gcagttccgc cccacggcca ccccggatga 30660
   cctctacatc tccgctgcga tgggcctggg tctcggtgct gctggtggcc tctcggtgaa 30720
   tcctgcccgc ctggccgcgc aacgtctcgc tgctgagaac cgccgcctcg gtgagttcgg 30780
   tctccgtgaa tccggcaagg cgcagatcaa ggagcttggc gacaacggct tcaacttcgg 30840
   tgctggccgt gaggagttcg cacggcgcat ccaaggcaag cccgatgagc cggtggagat 30900
   caagtaccca ggcggtgcaa tcgtgctgcc gcggggcgat ggtgagcctc cgaagatttt 30960
   ccaccctggt gatccccctg aggttcgcaa gccagggaac atcaacgagc cgcttcctcc 31020
   cgaagctcct ccagctactc ctccggccac cggcccggtt gctcccaagg ctcctccagc 31080
   agaggcacct aagggcaagg gctggacctc cgagtgggac actccgcggt acgcctcagg 31140
   cggtggcaac gagcaactcc tcgtgctgcc tccggcaaag cgtgtgagtc agttggctga 31200
   gtatgtccgc cagttctcga agaacgggga catcgtgagg gtgatggacc gggtgctgaa 31260
   gggcatcgac ctccgcaagt tggagttcaa ggtcatcgag aagggtcagc gtttcggcca 31320
   gcgtgacatg gacaacgaaa tcctcggcgc gaagggcgct gtaggtactc cgcgaggttc 31380
   cattggtgac aacatcatga tgttcctgcg gggccactcg tgggagatgc ctggtgtcaa 31440
   cccgatgcac acggtgggtc tcaacgagga gacgttcgtt cacgaactcg ttcacgttgc 31500
   caccgtctac aagctccgcg gtgttgagcc tggcatgggt gtacgcatca cggaccctgt 31560
   tgtgcgcagg gctgctgatg acctggcgaa cctccacggg gacatcctcg accacgccag 31620
   gcaaaccttc ggggccaact ggaaaggtga actccaggga cgcctcggtg ccaacctgga 31680
   gaacgagaag gaactcatcg cctatggtct gacgaaccgg aacttccagg agtggctcaa 31740
   gacggtgccc gttgagggtg gccctgagaa gaacctgtgg gaccgcttcg tgcattccct 31800
   gcgcaagctc ctgggcatcg gcccgaagga acacaacgcc ttcacccggc tgatcgaact 31860
   gtccgcccct ctcacgaaga agggcgactt cgttgagcgc atcaagacga acccagagtt 31920
   ggaagcaacg ggtgggtttg ttgacgctga caccgtgaag gccgcgaacg aagctgacct 31980
   ggctccggtc tatggctggg gtctcggcct ggagaacagg ctgggtggtg ctaaggctcc 32040
   ccccgctgtt cgtcagttgg cctcgaagct gttcggcacc accatcggct acaaggacaa 32100
   cgcggtggtg aagctcaacg cttgggacga caccacgaag tgggctgact cctgggccgt 32160
   ggagatgcgc aagggcacct atccgcagtt cgaggagtgg ctcaagggct ctcagtacaa 32220
   gtggcacgag aagggcaagg cgttcgatga cttcggcgca caggtgtcca actacatccg 32280
   cggcttcgag ggtgattacc caccgcaggt ggtcaaggct ggcgagcaca tgcgcaagac 32340
   cctggccaac gtggtggact acatcaacag cccactgaag gacgaaggcc gagccaagat 32400
   tggtctcacc gagacggaca tccgagaccc ggagaccggc aaggtggagc gggtagggac 32460
   gctggagaag aacccgaact acctcccgcg caagcacgac atcaacaagt ggaactcgat 32520
   ggtctccaac ttcggcaggg atgccgtgga agggtggtgg gcacgggcct accaggctgg 32580
   ccgtgaggga atctctgacg aggccgctgc gaagtgggcc aagtggtatg tccgcacggt 32640
   ggaggaggct cacgccaacc gcactcagga catgctcgat gacctcctga agggcaccga 32700
   tagggacgcc ctgaagaact ccctgatgct caacggaggc tactccgaag cggaggctct 32760
   gcggatcatg gacgacatga ttcctggtag ggccaccgat gcaggccgca cgatggccag 32820
   cctgaagcac cgcaacacca tccgggaaac gcacaccgag cggtggacca cgaaggacgg 32880
   gacgaagatg gaggtgagtc tgaacgactt catccactcg aacgccttcg acgtggttga 32940
   gccgtacctc cgcaggaccg cgggcagtgt ggcgctggcc aagcatctcg acatctacaa 33000
   gatgggggac attgaccgcg ttatcgctga ggccaccggc aacaagcttg ggcaggagtt 33060
   caagtccacc cccgatattc agaagctccg caaggacctg aagttcgcct tcgagcgagt 33120
   ccaagggctt cccctggagg agttctccac gctgaacaag agcctggaga tgtggcgcaa 33180
   cttcaacgtt atccgcctga tgggtggagc agtctggaac caggccaccg aactcagcca 33240
   gatcatcggc acgatggggt ggaagactac gcttgcggct ctccctgagc ttcgagcact 33300
   gcgccgtgac atcgccaccg gcaaggcccc gcatgacatc ctggaccacc tggagaacac 33360
   cattggtggc gtagggtccg agtacgtggc ccgcctggag ttcaaggctg gtgacgattg 33420
   ggtccgcaac aagggggaca ccaggttcaa ccgctggctg gactctgctg acaccggcac 33480
   caggaagctg gcgaaaggtg tgctggatta caccggcatg actccgctga tgattcagca 33540
   gaagcgtgtc cacgcgattg cgttggtgaa ccacttcgtc aacgtggcga acggcaaggc 33600
   tgctgggttc ctcacgaagg atcgcctggc ctggatgggt atgagcgcgg atgacttcgg 33660
   caaggtcctg tctggcatca agcagttcac caagcccgct gatggtgagt tctcgaagac 33720
   cttcaagatg gacttcgcgg gctggcagaa ggcggacccg gagagctact cgaagttcat 33780
   gacggccatc caccgtgaat cccgcagggt catccaggag aacgacctgg gctccatgat 33840
   cccgctcatg ggcaccacgc tgggcaagac ggtcttccag ttcatgaact tctcgatgca 33900
   cggctggaac aagtcgctga tgttcgccat gaaccaccgc gactggtcca cactgtccac 33960
   cgtacttcac ggctcactct tcgcgtccat cgcctacatg gggcggacgc tgctgggtgc 34020
   cggtggcatg gaagcggaca agcgccagca gtatctcgac aagcggatgt ccgttggcca 34080
   gatcgttacc aacagcttcg ggcgcatctc tcaggcgtcc gtgctgccca acatgttcga 34140
   caccatctca ccgtatccgc tgttcagcgg aatgcggacc acgagtgacc tctccagtct 34200
   ggcatcgaac ccgacctacc aggccatcaa cggactcatc tcgatgaaga agctgattcg 34260
   gaatggtgtg tcggatgagt accaaaccac ggagaaggac atccgcacct ggggcaggct 34320
   actgcctctc aacaacgtct tcccggtgac cacgttcctg aaccacctgg cgaacgatta 34380
   tccgcacggc gaaaagcaac aataaacggg tagccctcgg cacgaccggg ggcaacctct 34440
   tttggagaat agatagtgcc ttacagttac gttcttctct cggggaacgg ctctgcgacc 34500
   aacttcggct tcagcttcgg ttatctcagc aagttccaca tcggagtgaa ggtgaacggt 34560
   gtagtcacca ccttcacctg ggtgacggac ttcaccattg gcatcacacc ggccccggcc 34620
   aacggtgcag tcatcgaggt tcgacggacg actccgttga atcaacccgc cgtggactgg 34680
   tcagatggct ccacgctcac cgaagcggac atggacctca acactcggtt ctctctgtac 34740
   actgctcagg aggccgctga tggtgttgca gcatccatca ctcagaactc cctggggcag 34800
   tgggacggcc agaaccgcag ggccgtcaac ttcgcagacc cggttgatcc acaagtactt 34860
   cgaggacgtg tacacttcga ggacgtgtac acacctcagt tggacgcgaa ggtcaccgaa 34920
   gccaccaacc aggccaacaa cgcggcctcc agcgccgcca ctgcgcaggg ctatgctctc 34980
   gctgcggaca actccgcgga cctcgctgcg gccctcctgg cgaccttcaa aggccagtac 35040
   ctcggtgccc ttgcatctaa ccccacgctg gacggtaacg gccagccggt gactgctggt 35100
   gacctctact tcagcaccac cgataacctg atgaaggtgt acaccgggtc cgcgtggatc 35160
   aacgctgggt caaccgtcca gtccaccatc aaacgtcctg tcacacccgt cgtggcaacc 35220
   gcaggccaga ccgtgttccc ggtgtctggt gggtacgacg ccccatacat tctcgtgttt 35280
   gtgaatgggg ttgaggtggc ttctccagat gtggacgtga ctaacggcag caccatcgta 35340
   ttctccagcg gcctgactgc tggagataaa gtggattacg cagcgtttgg tgcgttccag 35400
   gtggccaacc cggttatcga tgggaccagc gccgcagact tcatcaagac acgcaatgcc 35460
   cgtgtagtta cctctattgc cgacctgaag gccctcaata agaacaccta caacttcgtt 35520
   ctcgtcactg gcttctatgc ttcaggggat ggtggcggcg gtttcttcct tcaggttccc 35580
   acgatgccca ccaacggtat cgttcaggtc gggaatgacg gaggcatctg gcagttggtg 35640
   gttgatcggg attatgtttc cgcgaaacaa ctcggtgcga gactggacgg ttcaacggat 35700
   gactcctctc tcctgaacaa cgccaagtcc actctcgatg ctcttggtaa gaggctgtat 35760
   atcccgtctg gggtttgcag aatctcaaca gcaatcactc caccaaaggc tggtgtgttt 35820
   ggggatagtc ctcaagcgtc catcatccag tgtaacaact gctctgcatt cctattccca 35880
   gcaaattttg ggctctctcg tccggcttgt gtcattgaga agttggggat tcagtcctac 35940
   agcaacacct gcgatgggct atacgctttc cgtgcccctg gggtggcatc tggagcatcg 36000
   cccgtctaca acagcggcct aactgttagg gatgttgaga ttggtacggg cggacgattc 36060
   ggtggcggtt tctcactgaa ggacttcttc cgagtgaacg tagagaacat tggcatgact 36120
   gatgtgagtt ccgccgtatt gctcaccggg tcagttgtgc aggcagtatt ccgaaatgtc 36180
   accgcaaacg gtgataacgc accaactgtt cttaaccggt atggtttcca aacagccgca 36240
   gcctcctatt ccagcggtac gctaggtcct gaacacatta gtacgtggga ttgcagcttc 36300
   attcgctata cacgcggtgt tcaacacgat gctgggctca tggtctcgtt caacaatacg 36360
   gacctggaaa ctttcacaca cggcttctat ctctcgcagc cctgcactgt gcgtggtggt 36420
   attagcgccc cggctccggc agcttcaggg actgctgcgt ggattgggct tttcaaagct 36480
   atttctgatt ttgacgtagc caacggcact ctgatcgatg accttgagat caacacgcta 36540
   aacaccccag gaactccagc ctcttcgtat ggggttctca ttggcaacaa tgtgaataag 36600
   tgcattggta ctacaatccg tagtcccagg attcgaggta acactagttc aatggtcggt 36660
   gggattgtcg ctaatctagc tggaggtgac atcgttatcg aggatgccat catcaacggc 36720
   agtgtggtta ctggaactac ggtgtctgtg aacaatgctt cctatgcaag ggttgtgggc 36780
   aatcgaagcg ccaccggtgg gactgtaaat ggttccctgt caatcacaga taacggtgtt 36840
   ggttccattg gtgatgttcg tggaaatgag tttgccacca ttaccaacac cctcaatgcc 36900
   tattccggta catggacgcc tggaacaatt cctaacggaa caccagcagc aacaacggtg 36960
   gccgtccctg gcgcagtggt tggtgacaaa gtagtggtcg gcctttccag cctgaccgga 37020
   tcggccaact gcatcatttc cggctatgtg tcttccaccg gaaatgtggc tgtcctgttg 37080
   tataacgtct ctggtgcatc acagacgatt ccctccggga ctctccaggt aacagtcctc 37140
   aagtcgtaat caagatgtcc ctctaggttt cccctggagg gacttcctct ttcaaggaaa 37200
   ggtatgagca atgcgctcaa cgtaagtaag ctggccacac tcacggcaac agaaatcaaa 37260
   gcataaccaa acaagaagta atcatgccga acatcgacaa agacgtacag aaggatgctc 37320
   tgaaggaggc cctcacggag tggctggaca agcagttcgc cacattcggg aagtgggcct 37380
   tgcggtccat cctggccgct gccttctcag tcctcatgta cctgtacctg acttctcaag 37440
   gctggcaccg ctgatatgac cgaaaagacc accgcttccg aaaaggagct tggcgaagtc 37500
   cacaacgaga tggccgcatg gtgcctggac atcctcaagg gaatcccggt caccgacaaa 37560
   gacggtaacc tcgtgattga ggatgggaga gttgctcgtc tccctccggc tcctgcctac 37620
   ctcaacgtca ttcgccagtt cctcaaggac aacgacatcc aggctgaacc cgccaagggc 37680
   tcctcgatgg gtgacctctc ggacctcccg gtgttcgagg atgacaacgt tgtgcctctc 37740
   aagtctcaat cgaaataaac gcgattagag gccctcagag cgattttaag cctccaaggt 37800
   agggtagcct atccgggcac ctgatcgcgt cctgtggggc catctcgcaa gccaagaatg 37860
   aaaataacaa ctgccgaggt ttcggcaaaa cgctgcccga agtgcggcga agaaaaacac 37920
   ctctccgagt tacacgcgaa tcacaccaag agggacggcc acaacaccat ctgcaagctc 37980
   tgcatgaagc aggtggcacg agactggcgc aacacacctc cgggccgctc caagcagatg 38040
   tggacgacct caaagaaacg tgcggaggag aggggctggg agttcaacct aacccccgag 38100
   tggattcagg aacgcctcga agctggcgtg tgtgaggcca ccgggattcc cttggagatg 38160
   tccgcggagg agttcaaagg ctacggccac ttccgtccat ggaccccctc actcgaccga 38220
   gacgatccaa cgaaagggta cacaaccgac aacgtgaagg ttgtgtgctg gatgtacaac 38280
   caggccaaag gcgtaagcat gcacgaagcc gtcctaagaa tggcccgtgc cctcgtagcg 38340
   aatgacaact aaacaacacc cagcacagaa agactttcgc gtctttatgt tcatggtgtg 38400
   gcgccacctc aatctccccg aacccacacc agtccaatat gacatcgccc actacttgca 38460
   acacggacca cgccgttcag tcatcgaagc gttccgtggt gtaggtaagt cctggatcac 38520
   ctccgcctta gtttgctggg ttctgtggaa cgacccacag aagaaaatcc tggtcatctc 38580
   cgcctcgaag gaacgagcag atgccttctc taccttcgtg aagcggctca tcaacgagct 38640
   tcccgttctc cagcacttga agcctaaggc ggaccagcga gactcgatga tttccttcga 38700
   tgttggtccc gcaactcctg accactcccc ctcggtcaag tccgttggta tcaacgggca 38760
   gatcactggt tctcgtgccg acatcatcat cgctgatgac gttgaggttc ccaataactc 38820
   cgccacgcag atgatgcgcg acaagctctc tgaggcggtg aaggaaatgg atgcggtcat 38880
   caaaccgctc cagacctccc gcatcatcta tctgggcacg cctcagacgg agatgtcgct 38940
   gtacaacgct ctccctgagc gtggatacga agcccgcatc tggccagcgc tgtaccccga 39000
   gcttcacctc gtggccaact acaagggccg cctggctcca ttcatcacgc gggctctgga 39060
   ggccgataag agtctcgtag gtgctcctac ggaccccagg cggttcaacg agactgacct 39120
   gttggagcgt aaggcgtcct atggacgtgc tggcttcgct ctccagttca tgctcgacac 39180
   gagcctcagc gatggtgacc gctacccgct gaagatcgcg gacctcatcg tccagaacct 39240
   caaccccacg atggcccatg tgaagatcgc ctgggctgct gcacctgaag tttgcatcaa     39300
   cgatctcccc gcggtggccc tcacgggtga ccgctactac cggcccatgt ggacggacca     39360
   gcagatgtcc gagtacacgg gctgtgtcat ggccatcgac ccctcgggcc gtggtgctga     39420
   cgagaccggc tacgccatca tcaagattct cgcaggcaac ctcttcctgg tggccgcggg     39480
   tggactctcc ggtggctact cagatgaaac tctggagacc ctggcgagac tcgctaagac     39540
   ccaccaggtg aaccacgtca tcatcgaggc caacttcggt gatggcatgt acaccaagct     39600
   catcactcca ttcttcggga aggtgggaca caaggtcctg gtggaggagg tgaagcactc     39660
   cacgcagaag gaagcccgta tcatcgacac ccttgagcct gtgctctcga ctcatcgtct     39720
   catcgttgac cagaaggtca tcgagaacga cttcaggacg gcagagcagg acatcaagta     39780
   cagcctgttc taccagatga cccggatcac ccgagacaag ggtgccctgg ctcatgatga     39840
   ccgtctcgat gcactggcca tcgctgttgc ctactggacg gagcatatgt ccagggacaa     39900
   cgataaggcc gctgctgcga tcaaggacaa ggcgctgaag gatgaactga agaagttcgt     39960
   tcacggtgtc cttgggagca aacccaagcg aacctcgtgg atgtcctcga actcaggctc     40020
   caggtgacat tcggtgccac aataggagaa ccctacgtgg gttcttcggg ggcttcatcc     40080
   gtagctgata tggatgccac acaccgtgtg gactcgggaa acctcagtgt gtggtgatgt     40140
   agtcgctgca ttctaggaca cccgttagtc tccctattcc tcatctctat ggggggtagg     40200
   ggggctaact taggtgttcc tagtgttgat gatatagcca ctgagatgtc aacctcagtg     40260
   tcccttaagt tgtctcttag ggttgcatta aggagacatc atcaccatca tctcccataa     40320
   ggtcatcctc cccatgttca ctctactagt cctcctctca ggtgtccccg tggtgttcct     40380
   tctgggtctc gttctgtatg gcctgttgga caactgatgg tgtccctgaa gtgccccctt     40440
   agggggaaaa cttccgacgc aaaaatttga aagccccact cgaaattcga cgcgggcaga     40500
   ttccccccgt gcccccccgc ggcccggccc tcgtggcccc tgccgaccca cctccgggca     40560
   ccctccaggc tgtacgctcc gctgactcc                                         40589